# EUROPEAN PATENT SPECIFICATION

(11) **EP 3 445 763 B1**
(45) Date of publication and mention of the grant of the patent: **10.11.2021**
(21) Application number: 17786237.2
(22) Date of filing: 18.04.2017
(51) Int. Cl.: C07D 471/04, A61K 31/437, A61P 35/00

(54) **NOVEL CRYSTALLINE SALT FORMS OF 3-(1,2,4-TRIAZOLO[4,3-A]PYRIDINE-3-YLETHYNYL)-4-METHYL-N-(4-((4-METHYLPIPERAZIN-1-YL)METHYL)-3-TRIFLUOROMETHYLPHENYL)BENZAMIDE FOR MEDICAL APPLICATION**
NEUARTIGE KRISTALLINE SALZFORMEN VON 3-(1,2,4-TRIAZOLO[4,3-A]PYRIDIN-3-YLETHYNYL)-4-METHYL-N-(4-((4-METHYLPIPERAZIN-1-YL)METHYL)-3-TRIFLUORMETHYLPHENYL)BENZAMID ZUR MEDIZINISCHEN ANWENDUNG
NOUVELLES FORMES DE SEL CRISTALLIN DU 3-(1,2,4-TRIAZOLO[4,3-A]PYRIDIN-3-YLÉTHYNYL)-4-MÉTHYL-N-(4-((4-MÉTHYLPIPÉRAZIN-1-YL)MÉTHYL)-3-TRIFLUOROMÉTHYLPHÉNYL)BENZAMIDE POUR UNE APPLICATION MÉDICALE

(30) Priority: 18.04.2016 RU 2016114904
(43) Date of publication of application: 27.02.2019
(73) Proprietor: Limited Liability Company «Fusion Pharma», Moscow 121205 (RU)
(72) Inventor: CHILOV, Germes Grigorievich, Krasnodar 350037 (RU); TITOV, Ilya Yurievich, Moscow 115432 (RU)
(74) Representative: Peterreins Schley
(86) International application number: PCT/RU2017/050025
(87) International publication number: WO 2017/184032

(56) References cited:
- EP-A1- 2 927 232
- WO-A1-2013/170774
- WO-A2-2015/108490
- RU-C2- 2 477 723
- MIAN A. A . ET AL.: 'PF-114, a potent and selective inhibitor of native and mutated BCR/ABL is active against Philadelphia chromosome-positive (Ph+) leukemias harboring the T315I mutation' LEUKEMIA vol. 29, 2015, pages 1104 - 1114, XP055435467

## Description

### The field of technology

This invention is referred to the chemistry of organic compounds, pharmacology, and medicine, namely, is referred to the compound salt form, as well as to its crystalline (polymorph) forms possessing the improved physicochemical properties and the high efficacy and safety as compared with the free base of this compound.

### The level of technology

For the purpose of drug production, it is important to have the pharmaceutical substance in the form which is convenient for its treatment and handling. This is important not only from the point of view of creating the commercially viable production process, but also from the point of view of the subsequent production of the pharmaceuticals containing this active substance. In addition, there are very essential factors such as chemical stability of active ingredients, stability of their solid forms, and their stability during storage. Pharmaceutical substances and medicinal compositions containing these substances should possess the ability to be stored efficiently during acceptable periods of time with no considerable changing the physicochemical characteristics of their active substances, such as chemical composition, density, hygroscopicity, and solubility. In this respect, the use of amorphous forms of substances as drugs seems to be undesirable. For example, these forms possess unstable physicochemical properties, such as solubility, hygroscopicity, friability, caking, etc. Thus, it is essential to have a drug as its crystalline and stabile form(s) to produce commercially viable and pharmaceutically acceptable medicinal compositions.

Solid substances including pharmaceutically active compounds frequently have more than one crystalline form; this phenomenon is known as polymorphism. Polymorphism takes place when a compound is crystallized as multiple different solid phases which differ from each other in crystalline package. Usually polymorphous modifications (polymorphs) have different physical characteristics including solubility and physical and/or chemical stability. Different solid salt forms of one and the same medicinal substance and, furthermore, different polymorphs of one and the same solid salt form can differ in the rate of releasing drug substance, as well as in the stability of the solid state of salt form and in their suitability for production of pharmaceutical medicines.

Selection of the suitable salt form for production of the corresponding substance possessing pharmacological effect is an important event of the preclinical phase of drug development. Changing the salt form of active drug substance is the commonly used way to modify its chemical and biological characteristics which does not lead to its structure modification. The choice of specific salt form can deeply influence the physicochemical properties of this drug (for example, the rate of dissolution, solubility, stability, and hygroscopicity). Replacing one salt form of the drug substance by the other salt form can change the drug therapeutical efficacy and/or safety which are especially important for large-scale production of the optimal drug composition. Nevertheless, there is no reliable way to accurately predict the effect of changing the salt form of the drug active substance on its safety and/or its biological activity. Moreover, even the study of physicochemical properties of different salt forms of the active drug substance cannot allow one to unambiguously identify the salt forms possessing the desired pharmacokinetic properties, efficacy, and safety. Briefly, there is no reliable way to predict the influence of specific salt types on behavior of the original compound in pharmaceutical medicines (Bergeetal., PharmaceuticalSalts// JournalPharm. Sci., 1977, Vol.66, No.1; Verbeeck et al. Generic substitution: The use of medicinal products containing different salts and implications for safety and efficacy // EP Journal Pharm. Sci, 28, 2006, 1-6.).

Pharmacokinetic parameters are the most important characteristics defining suitability of the solid salt form (or the specific polymorphous modification) for the use as a pharmaceutical medicine. The average daily and maximal drug concentrations in blood of humans and animals can substantially depend on the composition of the salt form and its polymorphic modification. As a rule, the drug substance salt forms which possess the higher solubility in water allow one to achieve the higher values of the maximal drug concentrations in blood and tissues of humans and animals. It is worth mentioning that the increase of the maximal drug concentrations in blood of animals correlates, as a rule, with the increase of toxic effects caused by the drug. For this reason, changing the substance salt form can lead to the change of the drug safety profile.

Solid salt forms are usually more preferable for peroral medications since these salt forms particularly tend to display the desirable physical characteristics and, in the case of basic drug substances, the salt forms obtained by acid accession are frequently the preferable salt forms. As mentioned above, different acids highly vary by their ability to add the desirable properties to the corresponding salt forms (such as stability during storage, easy process of production and purification, pharmacokinetic parameters, etc.) and such properties cannot be predicted with satisfactory accuracy. For example, some salts are solid substances at ambient temperature, whereas the other salts are liquids, viscous oils, or resins. Moreover, some salt forms are resistant to influence of heat and light under extreme conditions while the other salt forms are easily decomposed under much softer conditions. Thus, development of the suitable salt form resulted from acid accession to the basic drug substance and used in the pharmaceutical composition is an extremely important process which is far from being always predictable.

Protein kinases represent an important protein family that participates in regulation of key cellular processes. The impaired activity of protein kinases can lead to different diseases. A prospective approach to therapy of the diseases associated with the impaired activity of protein kinases is the use of low-molecular chemical compounds to inhibit their activity. Examples of such inhibitors approved for the use in clinical practice are as follows: Imatinib, Nilotinib, Dasatinib, Sunitinib, Sorafenib, Lapatinib, Gefitinib, Erlotinib, and Crizotinib. A large amount of drug candidates, the protein kinases inhibitors, are currently at the stage of clinical trials or at the stage of preclinical development.

BCR-ABL is a fusion protein, a product of hybrid gene BCR-ABL1, formed as a result of reciprocal translocation between chromosomes 9 and 22 (Philadelphia chromosome). BCR-ABL is the constitutively active tyrosine kinase that is responsible for oncogenic cell transformation (oncoprotein). The permanent activity of this tyrosine kinase makes the cell capable of dividing without influence of growth factors and causes its excessive proliferation. BCR-ABL is a key pathogenetic factor causing development of most cases of chronic myeloid leukemia and 20-50% cases of adult acute B-cell lymphoblastic leukemia. Thus, the inhibition of kinase activity of the BCR-ABL hybrid protein is a prospective strategy of fighting different oncological diseases and, in particular, chronic myeloid leukemia.

Earlier, in patent RU247772, we described derivatives of 1,2,4-triazolo[4,3-a]pyridine and, in particular, 3-(1,2,4-triazolo[4,3-a]pyridine-3-ylethynyl)-4-methyl-N-(4-((4-methylpiperazin-1-yl)methyl)-3-trifluoromethyl-phenyl)benzamide which are the efficient and selective inhibitors of Abl kinase and its mutant forms, as well as of the other therapeutically significant kinases.

The article of IAN A. A .et al in LEUKEMIA, vol. 29, 2015, pages 1104-1114, relates to "PF-114, a potent and selective inhibitor of native and mutated BCR/ABL is active against Philadelphia chromosome-positive (Ph+) leukemias harboring the T315l mutation.

EP 2 927 232 A1 discloses heteroaryl alkynyl moiety or pharmaceutically acceptable salts as protein tyrosine kinase inhibitors.

WO 2015/108490 A2 discloses heteroaryl alkyne compounds that act as kinase inhibitors.

WO 2013/170774 A1 discloses aromatic ring disubstituted acetylene derivatives for use in tumor treatment.

In the course of *in vitro* and *in vivo* studies, the potential possibility has been shown for application of these compounds to treat oncological diseases, in particular, acute lymphoblastic leukemia, chronic myeloid leukemia, hepatocellular carcinoma, non-small cell lung cancer, and gastrointestinal stromal tumors in humans and animals.

### The invention disclosure

The purpose of this invention is to develop and create a novel salt form of the kinase inhibitor, in particular, a novel salt form of the Abl kinase inhibitor, containing the pharmacologically acceptable counterion, possessing the crystallinity, the high solubility in water, and the constant composition, allowing easy scaling of production and purification processes, and being prospective in terms of clinical application to treat the diseases associated with the impaired activity of different kinases.

The technical result of this invention is the development and production of a novel salt form of the kinase inhibitor, in particular, a novel salt form of the Abl kinase inhibitor, including its novel polymorphic modifications (crystalline forms) possessing the high solubility in water, the high inhibition activity in regard to Abl kinase (and the clinically significant mutant forms of this enzyme), the high average daily concentration, and the high value of the AUC_{∞} parameter (the area under the curve of concentration versus time) in blood of humans and animals, as well as the favorable profile of safety and efficacy to treat the diseases associated with the impaired activity of protein kinases, in particular, acute lymphoblastic leukemia, chronic myeloid leukemia, hepatocellular carcinoma, non-small cell lung cancer, and gastrointestinal stromal tumors.

The technical result of this invention is also the development and production of a novel salt form of the kinase inhibitor which is characterized by easy scaling of production and purification processes, the use of low-toxic solvents, and the high purity of the final product with the minimal amount of product purification stages.

The indicated technical result is achieved by obtaining the salt of methanesulfonic acid and the base 3-(1,2,4-triazolo[4,3-a]pyridine-3-ylethynyl)-4-methyl-N-(4-((4-methylpiperazin-1-yl)methyl)-3-trifluoromethylphenyl)benzamide 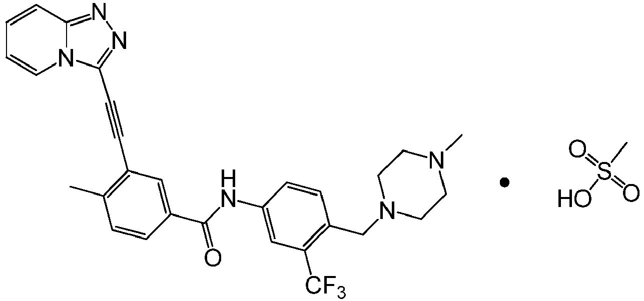 or its hydrate, solvate, as well as polymorphous modifications possessing the ability to inhibit the enzymatic activity of protein kinases, in particular, the Abl kinase.

One of the preferable variants of the invention implementation is the polymorphous modification of the salt of methanesulfonic acid and the base 3-(1,2,4-triazolo[4,3-a]pyridine-3-ylethynyl)-4-methyl-N-(4-((4-methylpiperazin-1-yl)methyl)-3-trifluoromethylphenyl)benzamide which is the crystalline phase with the parameters of the unit cell obtained by the method of powder X-ray diffraction at 25±5°C with the use of CuKα1 irradiation at wavelength of 1.5406 Å as follows: a = 51.46±0.05 Å; b = 7.81±0.05 Å; c = 7.63±0.05 Å; β = 108.9±0.1°; V = 2898.9±0.5 Å³; the space group *P*2₁/*n* and characteristic peaks in the Debye X-ray powder pattern with diffraction angle values (2θ) 3.6; 7.2; 11.4; 11.8; 12.5; 13.4; 14.5; 16.2; 16.5; 16.9; 17.2; 17.4; 17.8; 18.1; 18.4; 18.7; 20.8; 21.4; 22.7; 22.8; 23.0; 23.2; 23.4; 24.1; 24.5; 25.4; 25.9; 26.0; 26.2; 26.7; 27.1; 28.4; 33.0; 33.3; and 36.7.

Another preferable variant of the invention implementation is the polymorphous modification of the salt of methanesulfonic acid and the base 3-(1,2,4-triazolo[4,3-a]pyridine-3-ylethynyl)-4-methyl-N-(4-((4-methylpiperazin-1-yl)methyl)-3-trifluoromethylphenyl)benzamide which is the crystalline phase with the parameters of the unit cell obtained by the method of powder X-ray diffraction at 25±5°C with the use of CuKα1 irradiation at wavelength of 1.5406 Å as follows: a = 13.77±0.05 Å; b = 8.09±0.05 Å; and c = 30.83±0.05 Å; β = 117.8±0.1; V = 3036.36 ±0.5 Å³; the space group P2₁/c and characteristic peaks in the Debye X-ray powder pattern with diffraction angle values (2θ) 7.1; 7.3; 11.6; 11.8; 12.7; 12.9; 13.1; 14.2; 14.6; 16.9; 17.2; 17.4; 17.6; 18.1; 18.3; 19.4; 19.7; 20.8; 21.2; 21.6; 22.0; 22.5; 22.6; 23.2; 23.4; 23.8; 24.9; 25.1; 25.6; 25.9; 26.1; 26.6; 28.3; 28.8; 29.6; and 30.1.

The indicated technical result is also achieved by application of the salt or its hydrate, solvate, as well as polymorphous modifications according to the invention to obtain the pharmaceutical composition to prevent and/or treat the disorder associated with the kinase activity in humans or animals. In some variants of the invention implementation, the kinase is selected from a group comprising receptor tyrosine kinases, non-receptor tyrosine kinases, and serine/threonine protein kinases, in particular, ABL1, ABL2/ARG, BLK, DDR1, DDR2, EPHA2, EPHA8, EPHB2, FGR, FLT4/VEGFR3, FMS, FRK/PTK5, FYN, HCK, KDR/VEGFR2, LCK, LYN, LYN B, P38a/MAPK14, PDGFRa, PDGFRb, RAF1, RET, RIPK3, ZAK/MLTK(Mian et al., PF-114, a potent and selective inhibitor of native and mutated BCR/ABL is active against Philadelphia chromosome-positive (Ph+) leukemias harboring the T315l mutation // Leukemia., 2015, Vol.29, No.5)

This invention is also referred to the way of modulation of kinase catalytic activity including the way of achieving the contact between abovementioned kinase and the compound according to the invention. This way is designed to modulate the activity of kinases selected from a group comprising receptor tyrosine kinases, non-receptor tyrosine kinases, and serine/threonine protein kinases, in particular, Abl kinase, c-Src, Yes, Lyn, Lck, EGFR1 (Flt-1), VEGFR2, VEGFR3, PDGFR kinases.

This invention also includes the way of prevention or/and treatment of a disorder associated with the kinase activity in the body including the introduction of the pharmaceutical composition according to the invention into the abovementioned body. This disorder associated with the kinase activity represents an oncological, chronic, inflammatory, and/or the other disease, in particular, acute lymphoblastic leukemia, chronic myeloid leukemia, hepatocellular carcinoma, non-small cell lung cancer, and gastrointestinal stromal tumors. In particular cases of the invention implementation, the body represents a human being or an animal. In some variants of the invention implementation, an animal represents a cat, a dog, or a horse.

The indicated technical result is also achieved by the method of obtaining the crystalline compounds according to this invention including the following stages:
a. The introduction of the solution of methanesulfonic acid or its hydrate (in the organic solvent) into the suspension or solution of the base 3-(1,2,4-triazolo[4,3-a]pyridine-3-ylethynyl)-4-methyl-N-(4-((4-methylpiperazin-1-yl)methyl)-3-trifluoromethylphenyl)benzamide in the organic solvent or in the mixture of solvents. The introduction of the solution of methanesulfonic acid or its hydrate can be done both at room temperature and at heating or cooling of each component; one can also use the opposite order of mixing the reagents.
b. The crystallization of the obtained salt from the solution.
c. The separation of the salt crystals from the solvent.

In some variants of the invention implementation, the solvent used at stage (a) as a medium for suspending 3-(1,2,4-triazolo[4,3-a]pyridine-3-ylethynyl)-4-methyl-N-(4-((4-methylpiperazin-1-yl)methyl)-3-trifluoromethylphenyl)benzamide is represented by acetone.

In particular cases of the invention implementation, the solvent used at stage (a) to prepare the solution of methanesulfonic acid or its hydrate is represented by ethanol.

In some variants of the invention implementation, an additional recrystallization of the salt is conducted after stage (c).

In some other particular cases of the invention implementation, the additional stage of initiation of crystal formation is performed in the cases when the salt is obtained from solutions. The initiation of crystal formation can be achieved by introducing small amounts of the same salt into the solution or by the other ways.

In particular cases, one can additionally apply the stage of purification of the base 3-(1,2,4-triazolo[4,3-a]pyridine-3-ylethynyl)-4-methyl-N-(4-((4-methylpiperazin-1-yl)methyl)-3-trifluoromethylphenyl)benzamide by the way of its transforming into the salt of sulfuric, hydrochloric, benzenesulfonic, 4-methyl-benzenesulfonic, 2-methyl-benzenesulfonic, methanesulfonic, citric, phosphoric, trifluoroacetic, 4-nitro-benzenesulfonic, tetrafluoroboric, hexafluorophosphoric, or the other acid with the subsequent use of this salt to obtain the base 3-(1,2,4-triazolo[4,3-a]pyridine-3-ylethynyl)-4-methyl-N-(4-((4-methylpiperazin-1-yl)methyl)-3-trifluoromethylphenyl)benzamide that is used to obtain the salt of methanesulfonic acid.

The free base 3-(1,2,4-triazolo[4,3-a]pyridine-3-ylethynyl)-4-methyl-N-(4-((4-methylpiperazin-1-yl)methyl)-3-trifluoromethylphenyl)benzamide is known and described in patent RU247772.

### Definitions (terms)

The term «C», when used with reference to the temperature, means the centigrade scale or the Celsius temperature scale.

The term «**IC₅₀**» means the concentration of the tested compound that is sufficient to achieve the semi-maximal inhibition of kinase activity.

The term **«modulation»** used in the present document is referred to the change of kinase catalytic activity. In particular, modulation is referred to the activation or inhibition of kinase catalytic activity.

The term **«polymorphous modification»** is referred to the solid phase of the substance which possesses several different forms due to the different disposition and/or conformation of molecules in the crystalline lattice. Polymorphous modifications usually have different chemical and physical properties. In addition, the term **«polymorphous modification»** is also referred to solvates (i.e., crystalline forms containing the solvent or water), as well as to the different non-solvated crystalline forms of the compound.

The term **«solvate»** is used to describe the molecular complex containing the compound according to the invention and one or more molecules of the pharmaceutically acceptable solvent, for example, ethanol. The term **«hydrate»** is used when the mentioned solvent is water.

The term **«aberrant activity»** of kinase used in the present document means the kinase activity which substantially differs from the basic level of the kinase activity in cells in the absence of pathology. The aberrant activity can be caused by the change of the level of kinase expression, by the impairment of processes leading to kinase activation, by the regulation disorder of degradation pathways, and by the other factors.

The term **«auxiliary substance»** means any pharmaceutically acceptable substance of inorganic or organic origin included in the composition of drug product or used in the drug production process to achieve the required physicochemical properties of the drug product.

The term **«AUC»** means the pharmacokinetics parameter characterizing the total concentration of the drug in blood plasma during all time of observation. From the mathematic point of view AUC is defined as the integral from 0 to **∞** in a plot of concentration of the drug in blood plasma against time (the pharmacokinetics curve) and is equal to the area restricted by the pharmacokinetics curve and the coordinate axes.

The possibility of objective achievement of the technical result by the implementation of invention is confirmed by reliable data shown in examples containing the experimental results obtained in the course of the studies conducted according to the methodology accepted in this field. The essence of the invention is illustrated by figures.

One should understand that these examples and all examples included in the patent application are not limiting and shown only to illustrate the present invention.

### The way of therapeutical application of compounds

The subject of this invention also includes the introduction of the therapeutically efficient amount of the compound according to the invention into the patient's body in need of the correspondent treatment. The therapeutically efficient amount means such an amount of the compound which causes, with the highest probability, the desirable reaction of the patient's body on treatment (prevention). The precise required amount of the compound can vary from the subject to subject depending on the age, the body weight and the common patient's condition, severity of the disease, the procedure of drug administration, the combination of treatment with the other drugs, etc.

The compound according to the invention or the pharmaceutical composition containing the compound can be introduced into the patient's body in any amount and by any way efficient for treatment or prevention of the disease.

After mixing the drug with the specific suitable pharmaceutically accessible carrier in desirable dosage, the pharmaceutical compositions according to the invention can be introduced into the human body or into animals perorally, parenterally, locally, etc.

The introduction of the compound can be performed once or several times for a day, a week (or for any other time interval), or from time to time. In addition, the compound can be introduced into the patient's body every day for a certain time period (for example, for 2-10 days) followed by a time period with no compound administration (for example, for 1-30 days).

In the case, when the compound according to the invention is used as a part of the regime of combination therapy, the dose of each component of the combination therapy is introduced into the patient's body for the time period required for treatment. The compounds comprising the combination therapy can be introduced into the patient's body once (as a dosage of all components) or several times (as individual dosages of the components).

### Pharmaceutical compositions

The invention is also referred to the pharmaceutical compositions which contain the compounds according to the invention (the prodrug form or the other pharmaceutically acceptable derivative) and one or several pharmaceutically acceptable carriers, adjuvants, solvents, and/or excipients, such as those which can be introduced into the patient's body along with the compound of this invention, which cannot destroy the pharmacological activity of this compound, and which are nontoxic at the dosages sufficient to deliver the therapeutic amount of the compound.

The pharmaceutical compositions claimed in this invention contain the compounds of this invention together with the pharmaceutically acceptable carriers which can include any solvents, diluters, dispersions, suspensions, surfactants, isotonic agents, thickeners, emulsifiers, preservatives, binders, lubricants, etc., which are suitable for the certain dosage form. The materials which can serve as the pharmaceutically acceptable carriers include but are not limited by mono- and oligosaccharides as well as their derivatives; gelatin; talc; excipients, such as cocoa butter and wax for suppositories; vegetable oils, such as peanut butter, cottonseed oil, safflower oil, sesame oil, olive oil, corn oil, and soybean oil; glycols, such as propylene glycol; complex esters, such as ethyl oleate and ethyl laurate; agar; buffer substances, such as magnesium hydroxide and aluminum hydroxide; alginic acid; non-pyrogenic water; isotonic solution; Ringer solution; ethyl alcohol, and phosphate buffer solutions. The pharmaceutical compositions can also include the other nontoxic compatible lubricants, such as sodium lauryl sulfate and magnesium stearate, as well as coloring agents, separative liquids, film-forming agents, sweeteners, flavors, fragrances, preservatives, and antioxidants.

The subject of this invention also includes drug forms, i.e., the pharmaceutical compositions which are optimized for a certain way of introduction into the body in the therapeutically efficient dosage, for example, the introduction by oral, local, pulmonary (for example, as inhalation sprays), intravenous, intranasal, subcutaneous, intramuscular, and infusion ways according to the recommended dosage.

The drug forms of this invention can contain the pharmaceutical compositions obtained by the methods using liposomes, the methods of microencapsulation, the methods for obtaining the drug nanoforms, or by the other methods known in pharmaceutics.

To obtain the composition, for example, in the form of a tablet, the active substance is mixed with one or several pharmaceutical excipients, such as gelatin, starch, lactose, magnesium stearate, talc, silica, acacia gum, mannitol, microcrystalline cellulose, hypromellose, or analogous compounds.

The tablets can be covered with sucrose, cellulose derivatives, or the other substances suitable for making the cover. The tablets can be obtained by different ways, such as the direct compression, the dry or wet granulation, or the hot fusion in hot state.

The pharmaceutical composition in the form of a gelatin capsule can be obtained by mixing the active substance with a solvent and filling the soft or solid capsules with the obtained mixture.

For introduction by parenteral way, one can use aqueous suspensions, isotonic saline solutions, or sterile solutions for injections which contain the pharmacologically compatible agents, for example, propylene glycol or butylene glycol.

### Examples of pharmaceutical compositions

The substances described in this invention can be used to prevent and/or treat diseases in humans or animals as the following formulations ("Substance" means the active ingredient):

| Tablet I | mg/tablet |
|---|---|
| Substance | 50 |
| Lactose Ph. Eur | 223.75 |
| Croscarmellose sodium | 6.0 |
| Corn starch (5% w/v paste) | 15 |
| Polyvinylpyrrolidone | 2.25 |
| Magnesium stearate | 3.0 |

| Tablet II | mg/tablet |
|---|---|
| Substance | 200 |
| Lactose Ph. Eur | 182.75 |
| Croscarmellose sodium | 12.0 |
| Corn starch (5% w/v paste) | 2.25 |
| Magnesium stearate | 3.0 |

| Capsule | mg/capsule |
|---|---|
| Substance | 10 |
| Lactose Ph. Eur | 488.5 |
| Magnesia | 1.5 |

| Composition for injections I | (50 mg/mL) |
|---|---|
| Substance | 5.0% w/v |
| 1M solution of sodium hydroxide | 15.0% w/v |
| 1M solution of hydrochloric acid to pH | 7.6 |
| Polyethylene glycol 400 | 4.5% w/v |
| Water for injections up to 100% | |

| Aerosol I | mg/mL |
|---|---|
| Substance | 10 |
| Sorbitan trioleate | 13.5 |
| Trichlorofluoromethane | 910.0 |
| Dichlorodifluoromethane | 490.0 |

| Ointment | mL |
|---|---|
| Substance | 40 mg |
| Ethanol | 300 µL |
| Water | 300 µL |
| 1-Dodecylazacycloheptanon | 50 µL |
| Polyethylene glycol | up to 1 mL |

These compositions can be prepared in accordance with the standard pharmaceutical procedures. Tablets (I)-(II) can be covered with the intestine-soluble coat with the use, for example, cellulose acetate phthalate. The aerosol composition (I) can be used in combination with the standard dispensers. Sorbitan monooleate, sorbitan polyoleate, polysorbate 80, polyglycerol oleate, or oleic acid can be used as suspending agents instead of Sorbitan trioleate and soybean lecithin.

### The description of figures

**Figure 1****.** Crystallization of salts of 3-(1,2,4-triazolo[4,3-a]pyridine-3-ylethynyl)-4-methyl-N-(4-((4-methylpiperazin-1-yl)methyl)-3-trifluoromethylphenyl)benzamide in the scale of 100 mg.
**Figure 2****.** Crystallization of salts of 3-(1,2,4-triazolo[4,3-a]pyridine-3-ylethynyl)-4-methyl-N-(4-((4-methylpiperazin-1-yl)methyl)-3-trifluoromethylphenyl)benzamide in the scale of 100 mg after adding methyltretbuthyl ester.
**Figure 3****.** The X-ray diffraction powder patterns of free base 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-194-1 (polymorphous modification I);
   b) sample ― HAL-G-194-2 (polymorphous modification II).
**Figure 4****.** The photograph of the sample crystals of free base 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-194-1, polymorphous modification I) obtained by the method of polarization microscopy.
**Figure 5****.** The spectrum of ¹H nuclear magnetic resonance of the sample of free base 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-194-1 (polymorphous modification I);
   b) sample ― HAL-G-194-2 (polymorphous modification II).
**Figure 6****.** The DSC curve (differential scanning calorimetry) of the sample of free base 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-194-1, polymorphous modification I).
**Figure 7****.** The TGA curve (thermogravimetric analysis) of the sample of free base 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-194-1, polymorphous modification I).
**Figure 8****.** The hygroscopicity of the sample of free base 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-194-1, polymorphous modification I) according to the data of gravimetric moisture absorption.
**Figure 9****.** The X-ray diffraction powder patterns of the salt of hydrochloric acid and free base 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-194-1 (free base, polymorphous modification I);
   b) sample ― HAL-G-196-2 (polymorphous modification I);.
   c) sample ― HAL-G-196-3 (polymorphous modification II).
**Figure 10****.** The spectrum of ¹H nuclear magnetic resonance of the sample of the salt of hydrochloric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-2 (polymorphous modification I);
   b) sample ― HAL-G-196-3 (polymorphous modification II).
**Figure 11****.** The TGA curve (thermogravimetric analysis) of the sample of the salt of hydrochloric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-2, polymorphous modification I).
**Figure 12****.** The DSC curve (differential scanning calorimetry) of the sample of the salt of hydrochloric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-2 (polymorphous modification I);
   b) sample ― HAL-G-194-3 (polymorphous modification II).
**Figure 13****.** The hygroscopicity of the sample of the salt of hydrochloric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-2, polymorphous modification I) according to the data of gravimetric moisture absorption.
**Figure 14****.** The X-ray diffraction powder patterns of the salt of sulfuric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ―HAL-G-196-6).
**Figure 15****.** The spectrum of ¹H nuclear magnetic resonance of the sample of the salt of sulfuric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-6).
**Figure 16****.** The DSC curve (differential scanning calorimetry) of the sample of the salt of sulfuric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-6).
**Figure 17****.** The X-ray diffraction powder patterns of the salt of hydrobromic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-7 (polymorphous modification I);
   b) sample ― HAL-G-196-8 (polymorphous modification II).
**Figure 18****.** The spectrum of ¹H nuclear magnetic resonance of the sample of the salt of hydrobromic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-7 (polymorphous modification I);
   b) sample ― HAL-G-196-8 (polymorphous modification II).
**Figure 19****.** The DSC curve (differential scanning calorimetry) of the sample of the salt of hydrobromic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-7 (polymorphous modification I);
   b) sample ― HAL-G-196-8 (polymorphous modification II).
**Figure 20****.** The TGA curve (thermogravimetric analysis) of the sample of the salt of hydrobromic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-7, polymorphous modification I).
**Figure 21****.** The X-ray diffraction powder patterns of the salt of phosphoric acid and free base 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-13;
   b) sample ― HAL-G-198-3 (after desolvation of HAL-G-196-13);
**Figure 22****.** The spectrum of ¹H nuclear magnetic resonance of the sample of the salt of phosphoric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-13).
**Figure 23****.** The DSC curve (differential scanning calorimetry) of the sample of the salt of phosphoric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-13).
**Figure 24****.** The TGA curve (thermogravimetric analysis) of the sample of the salt of phosphoric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-13).
**Figure 25****.** The X-ray diffraction powder patterns of the salt of tartaric acid and free base 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-16;
   b) sample ― HAL-G-198-1 (after desolvation of HAL-G-196-16).
**Figure 26****.** The spectrum of ¹H nuclear magnetic resonance of the sample of the salt of tartaric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-16).
**Figure 27****.** The DSC curve (differential scanning calorimetry) of the sample of the salt of tartaric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-16).
**Figure 28****.** The TGA curve (thermogravimetric analysis) of the sample of the salt of tartaric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-16).
**Figure 29****.** The X-ray diffraction powder patterns of the salt of methanesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-19;
   b) sample ― HAL-G-196-20;
   c) sample ― HAL-G-196-21.
**Figure 30****.** The photograph of the sample crystals of the salt of methanesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-21) obtained by the method of polarization microscopy.
**Figure 31****.** The spectrum of ¹H nuclear magnetic resonance of the sample of the salt of methanesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-19).
**Figure 32****.** The DSC curve (differential scanning calorimetry) of the sample of the salt of methanesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-21).
**Figure 33****.** The X-ray diffraction powder patterns of the salt of 4-methylbenzenesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-23;
   b) sample ― HAL-G-196-24.
**Figure 34****.** The photograph of the sample crystals of the salt of 4-methylbenzenesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-24) obtained by the method of polarization microscopy.
**Figure 35****.** The spectrum of ¹H nuclear magnetic resonance of the sample of the salt of 4-methylbenzenesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-24).
**Figure 36****.** The DSC curve (differential scanning calorimetry) of the sample of the salt of 4-methylbenzenesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-24).
**Figure 37****.** The hygroscopicity of the sample of the salt of 4-methylbenzenesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-24) according to the data of gravimetric moisture absorption.
**Figure 38****.** The X-ray diffraction powder patterns of the salt of malic acid and free base 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-25;
   b) sample ― HAL-G-198-26.
**Figure 39****.** The spectrum of ¹H nuclear magnetic resonance of the sample of the salt of malic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-25).
**Figure 40****.** The DSC curve (differential scanning calorimetry) of the sample of the salt of malic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-25).
**Figure 41****.** The TGA curve (thermogravimetric analysis) of the sample of the salt of malic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-25).
**Figure 42****.** The X-ray diffraction powder patterns of the salt of fumaric acid and free base 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-28;
   b) sample ― HAL-G-198-29.
**Figure 43****.** The spectrum of ¹H nuclear magnetic resonance of the sample of the salt of fumaric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-29).
**Figure 44****.** The DSC curve (differential scanning calorimetry) of the samples of the salt of fumaric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-28;
   b) sample ― HAL-G-198-29.
**Figure 45****.** The TGA curve (thermogravimetric analysis) of the samples of the salt of fumaric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Sample ― HAL-G-196-28;
   b) sample ― HAL-G-198-29.
**Figure 46****.** The photograph of the sample crystals of the salt of fumaric acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (sample ― HAL-G-196-29) obtained by the method of polarization microscopy.
**Figure 47****.** The spectra of ¹H and ¹³C nuclear magnetic resonance of the sample of the salt of methanesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (polymorphous modification I):
   a) ¹H-NMR spectrum (BrukerDRX500, 13400, 500.13 MHz, DMSO-d6);
   b) ¹³C-NMR spectrum (BrukerDRX500, 125.76 MHz, DMSO-d6).
**Figure 48****.** The spectra of ¹H and ¹³C nuclear magnetic resonance of the sample of the salt of methanesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (polymorphous modification II):
   a)¹H-NMR spectrum (BrukerDRX500, 13, 500.13 MHz, DMSO-d6);
   b) ¹³C-NMR spectrum (BrukerDRX500, 13, 125.76 MHz, DMSO-d6).
**Figure 49****.** The X-ray diffraction powder patterns of the salt of methanesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Polymorphous modification I;
   b) polymorphous modification II.
**Figure 50****.** General view of the unit cell independent part of the salt of methanesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
   a) Polymorphous modification I;
   b) polymorphous modification II.
**Figure 51****.** The kinetics of dissolution of free base 3-(1,2,4-triazolo[4,3-a]pyridine-3-ylethynyl)-4-methyl-N-(4-((4-methylpiperazin-1-yl)methyl)-3-trifluoromethylphenyl)benzamide and polymorphous modifications of the salt of methanesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide.
**Figure 52****.** The mean values of the concentration of 3-(1,2,4-triazolo[4,3-a]pyridine-3-ylethynyl)-4-methyl-N-(4-((4-methylpiperazin-1-yl)methyl)-3-trifluoromethylphenyl)benzamide in blood plasma of C57BL/6 mice after a single peroral introduction. The mean values were determined for each time point on the basis of the individual data obtained for three animals.
   a) Peroral introduction of free base in dosage of 50 mg/kg;
   b) peroral introduction of the salt of methanesulfonic acid and 3-(1,2,4-triazolo[4,3-a]pyridine-3-ylethynyl)-4-methyl-N-(4-((4-methylpiperazin-1-yl)methyl)-3-trifluoromethylphenyl)benzamide (polymorphous modification I) in dosage of 59 mg/kg (50 mg/kg in the calculation of the free base).

### 3-(1,2,4-triazole[4,3-a]pyridine-3-ylethinyl)-4-methyl-N-(4-((4-methylpiperazine-1-yl)methyl)-3-trifluoromethylphenyl)benzamide salt form optimization

To obtain the salt form with best physical properties, various salt forms of 3-(1,2,4-triazole[4,3-a]pyridine-3-ylethinyl)-4-methyl-N-(4-((4-methylpiperazine-1-yl)methyl)-3-trifluoromethylphenyl)benzamide were synthesized. The key objective of salt form optimization was to obtain the salt form of 3-(1,2,4-triazole[4,3-a]pyridine-3-ylethinyl)-4-methyl-N-(4-((4-methylpiperazine-1-yl)methyl)-3-trifluoromethylphenyl)benzamide, containing pharmacologically acceptable counter-ion (preferably anion) with the following characteristics: crystallinity, high solubility in water (more than 10 g/l) and fixed composition. Besides, the process of the salt form production should be easily scalable and carried out in low-toxic organic solvents.

Various 3-(1,2,4-triazole[4,3-a]pyridine-3-ylethinyl)-4-methyl-N-(4-((4-methylpiperazine-1-yl)methyl)-3-trifluoromethylphenyl)benzamide salt forms were obtained in polar non-toxic (Classes 2 and 3) organic solvents. Counter-ions were selected based pharmacological acceptability and acid potency (pKa no higher than 5.0). The requirement to acid potency is based on that protic atom of nitrogen 3-(1,2,4-triazole[4,3-a]pyridine-3-ylethinyl)-4-methyl-N-(4-((4-methylpiperazine-1-yl)methyl)-3-trifluoromethylphenyl)benzamide is base with pKa ≈ 6.4.

At the first stage the initial base was tested for solubility in selected organic solvents. The maximum selected solvent volume used in this test was 1.25 ml per 1 mg of base considering the further scaling process. Results of the test for solubility of the initial base in selected organic solvents are given in Table 1. Low toxic (Class 3), low-boiling (T_{boiling} < 100 °C) high-polar solvents with the solubility potential of no less than 10 mg/ml were selected for further studies.

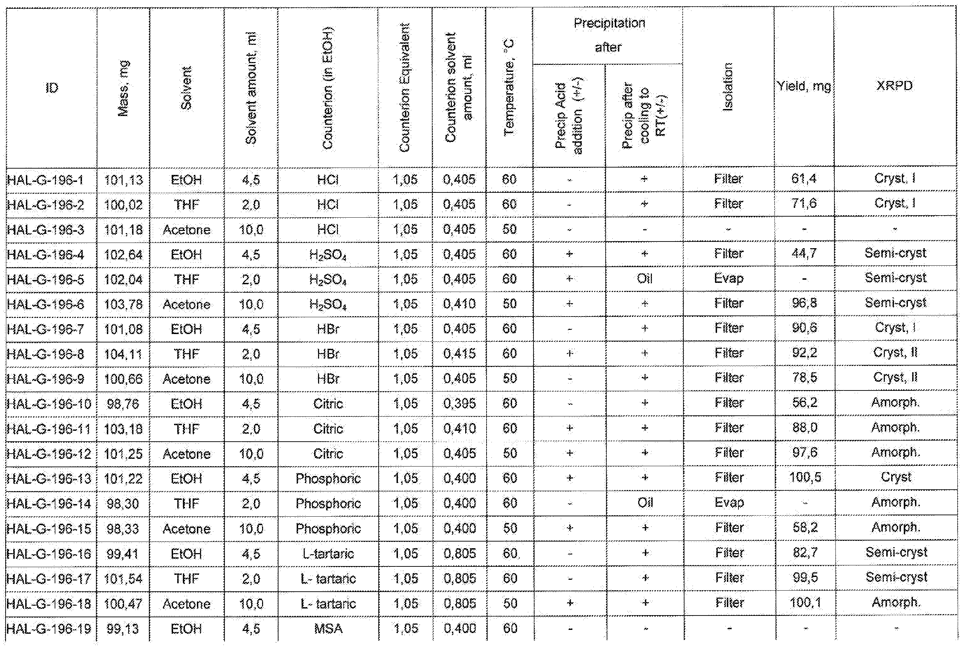

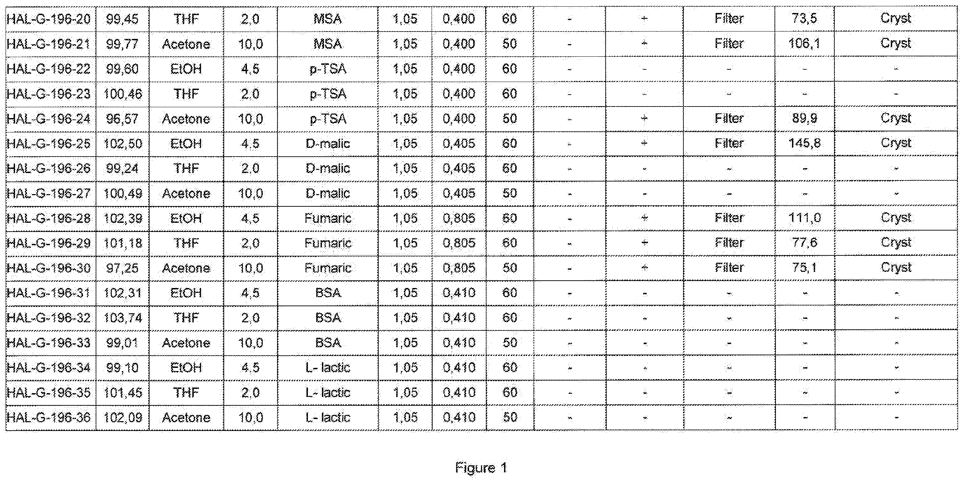

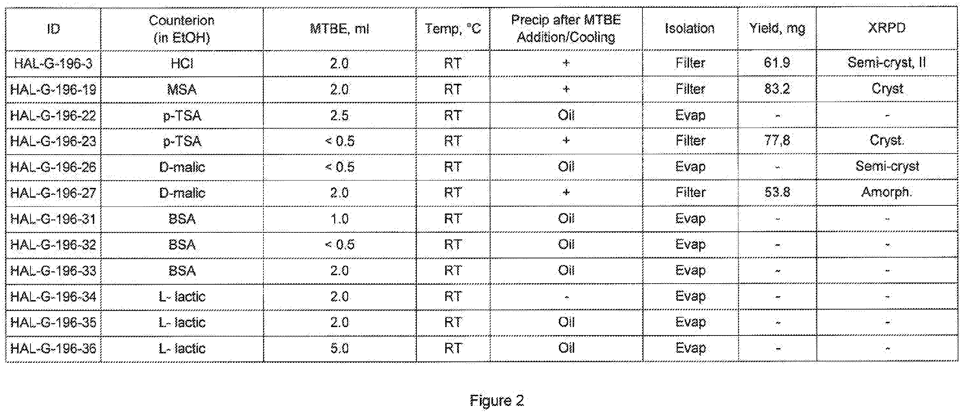

At the second stage it was attempted to obtain salts from 100 mg of base of 3-(1,2,4-triazole[4,3-a]pyridine-3-ylethinyl)-4-methyl-N-(4-((4-methylpiperazine-1-yl)methyl)-3-trifluoromethylphenyl)benzamide. For this study stage such solvent/acid pairs were selected, in which either the test sample was fully dissolved in the solvent and a precipitate was formed after adding the acid, or the system was homogenized after adding the acid and a precipitate was formed after cooling the system to the room temperature. In most cases 3-(1,2,4-triazole[4,3-a]pyridine-3-ylethinyl)-4-methyl-N-(4-((4-methylpiperazine-1-yl)methyl)-3-trifluoromethylphenyl)benzamide salting took place either immediately after adding the acid or after cooling the solution (see Fig. 1). In cases when no precipitate was formed after cooling the solution, to initiate crystallization process methyl tert-butyl ether was added to corresponding salts solutions (see Fig. 2).

**Table 1 ― Solubility of 3-(1,2,4-triazole[4,3-a]pyridine-3-ylethinyl)-4-methyl-N-(4-((4-methylpiperazine-1-yl)methyl)-3-trifluoromethylphenyl)benzamide in various solvents**

| Sample | Quantity, mg | Solvent | Solvent volume, ml | Temperature, °C | Solubility (+/-) | Solvent toxicity class |
|---|---|---|---|---|---|---|
| HAL-G-194-2 | 4.6 | Water | 4.0* | 50 | ― | ― |
| HAL-G-194-3 | 4.0 | Methanol | 0.1 | 50 | + | II |
| HAL-G-194-4 | 4.7 | Ethanol | 0.3 | 50 | + | III |
| HAL-G-194-5 | 3.6 | Isopropanol | 0.4 | 50 | + | III |
| HAL-G-194-6 | 3.6 | n-Butanol | 0.2 | 50 | + | III |
| HAL-G-194-7 | 3.5 | Acetonitrile | 4.0* | 50 | - | II |
| HAL-G-194-8 | 3.6 | Tetrahydrofuran | 0.1 | 50 | + | III |
| HAL-G-194-9 | 3.7 | 2-methyltetrahydrofuran | 0.6 | 50 | + | - |
| HAL-G-194-10 | 3.9 | Ethyl acetate | 1.3 | 50 | + | III |
| HAL-G-194-11 | 4.0 | Isopropyl acetate | 4.0 | 50 | - | III |
| HAL-G-194-12 | 3.4 | Acetone | 0.3 | 50 | + | III |
| HAL-G-194-13 | 4.8 | Methyl ethyl ketone | 0.4 | 50 | + | III |
| HAL-G-194-14 | 4.1 | Methyl isobutyl ketone | 0.6 | 50 | + | III |
| HAL-G-194-15 | 3.7 | Dichloromethane | 0.4 | 50 | + | II |
| HAL-G-194-16 | 3.4 | Toluene | 4.0* | 50 | - | II |
| HAL-G-194-17 | 4.7 | Dimethylformamide | 0.1 | RT | + | II |
| HAL-G-194-18 | 3.7 | N-methylpyrrolidone | 0.1 | RT | + | II |
| HAL-G-194-19 | 3.8 | Dimethyl sulfoxide | 0.1 | RT | + | III |
| HAL-G-194-20 | 4.7 | Methyl tert-butyl ether | 4.0* | 50 | - | III |
| HAL-G-194-21 | 3.3 | Heptane | 4.0* | 50 | - | III |
| HAL-G-194-22 | 4.3 | Cyclohexane | 4.0* | 50 | - | II |
| RT ― room temperature, * no solution happened. | | | | | | |

Crystallinity of all samples obtained was studied using the method of X-ray powder diffraction (to study the crystallinity structure). Diffraction patterns were obtained at a temperature of 25 °C (±5 °C) and relative air humidity of ≈70 % using CubiX-Pro XRD X-ray powder diffractometer (anode voltage 45 kV, current 40 mA), with X'Celerator detector. Survey step 0.02° 2θ, angle range 3-45° 2θ. Diffraction patterns obtained were studied in detail using X'Pert HighScore Plus software package.

Studies of samples crystallinity using the method of X-ray powder diffraction showed that studied samples HAL-G-194-1, HAL-G-196-1, HAL-G-196-2, HAL-G-196-4, HAL-G-196-5, HAL-G-196-6, HAL-G-196-7, HAL-G-196-8, HAL-G-196-9, HAL-G-196-13, HAL-G-196-16, HAL-G-196-17, HAL-G-196-25, HAL-G-196-28, HAL-G-196-29, HAL-G-196-30, HAL-G-196-3, HAL-G-196-19, HAL-G-196-20, HAL-G-196-21, HAL-G-196-23, HAL-G-196-24, HAL-G-196-26, HAL-G-196-35 represented individual crystalline phases or phase mixtures (see fig. 1 and 2). Solubility of such samples was studied using the method of high-performance liquid chromatography (HPLC) (chromatograms were obtained using Agilent 1100 Series apparatus with Phenomenex Luna column, 5 µM, 4.6 × 250 mm. Mobile phase (10 mM KH₂PO₄ pH = 3): acetonitrile volume ratio of 60:40. Flow rate is 1.0 ml/min. Detection was carried out at 254 nm. Runtime is 16 minutes). Samples were also studied using the following methods: polarization microscopy (Leica DMRB Polarized Microscope, resolution of 1600 × 1200) ― to confirm crystallinity, ion chromatography - to confirm anion and cation stoichiometric proportion, differential scanning calorimetry (DSC) and thermogravimetry (TG) - to confirm composition and study of temperature stability of samples; 1H NMR (500 MHz Bruker AVANCE 500,13 MHz, solvent DMSO-d6) ― to confirm structure, assess purity and content of organic solvents; gravimetric moisture absorption - to assess hygroscopicity. DSC was carried out using Mettler 822e DSC apparatus. The measuring system was calibrated according to ISO 11357-1 standard based on standard substances phase changes (C₆H₁₂; Hg; benzoic acid; Ga; KNO₃; In; Sn; Bi; CsCI; purity grade 99.99%). Temperature calibration regular error (determined based on In) is 0.1°. Samples were tested in standard aluminum cells in the artificial air flow at a temperature range of 30-300 °C with heating speed of 10°/min. TG measurements were taken using Mettler 851e SDTA/TGA TG analyzer. The apparatus was calibrated using standard substances melting points (Ag; Al; Bi; In; Sn; purity grade of 99.99%). Weighing error is NMT 0.1% (determined using CaC₂O₄·2H₂O standard). The test was carried out in a standard open aluminum container in the artificial air flow at a temperature range of 30-150 °C with heating speed of 10°/min.

To avoid dehydration, the material was not exposed to mechanical treatment before taking measurements.

### Study of physical and chemical properties of free base of 3-(1,2,4-triazole[4,3-a]pyridine-3-ylethinyl)-4-methyl-N-(4-((4-methylpiperazine-1-yl)methyl)-3-trifluoromethylphenyl)benzamide (polymorph modification I)

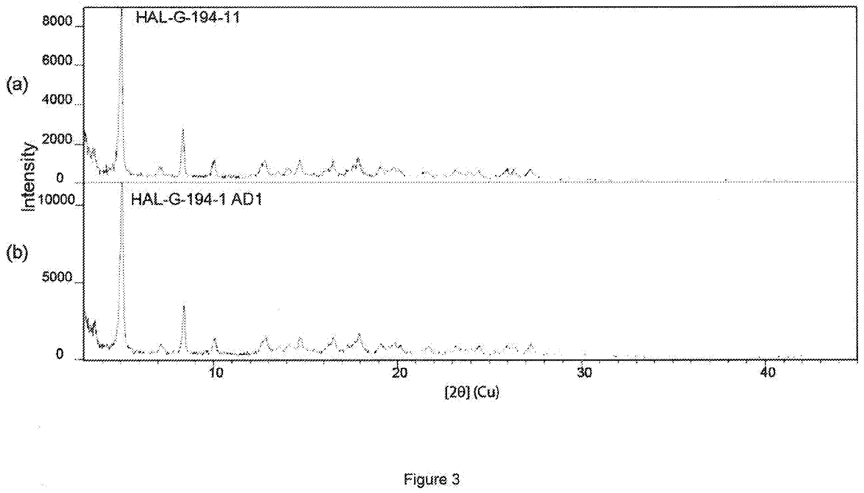

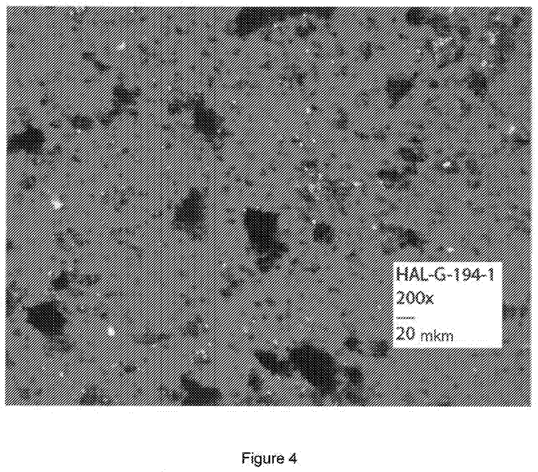

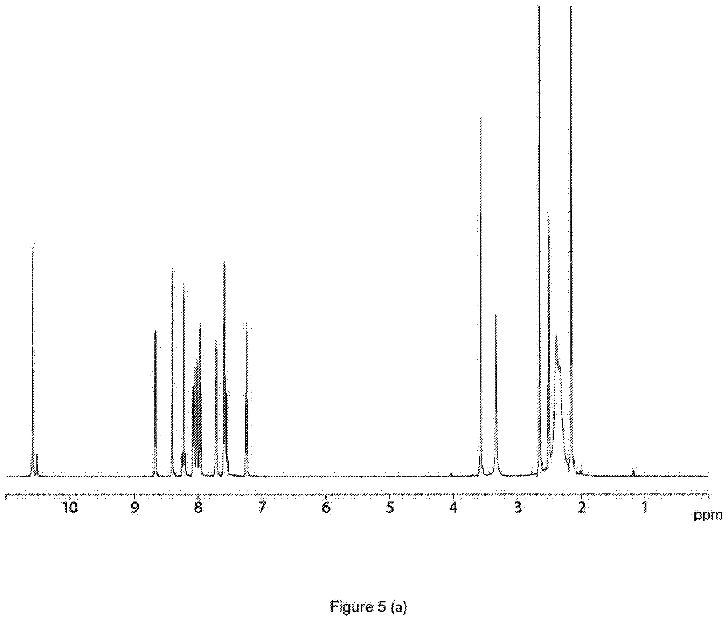

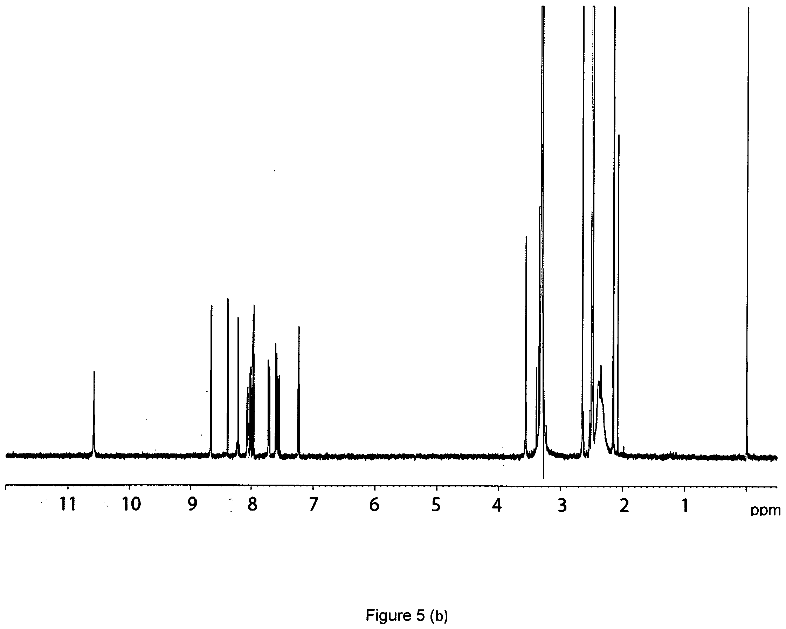

Based on the X-ray powder diffraction data (see Fig. 3), HAL-G-194-1 free base sample is an individual crystalline phase, which was also confirmed with polarization microscopy (see Fig. 4). The structure of the compound was confirmed using 1H NMR spectroscopy method (see Fig. 5). The apparent solubility of a free base in deionized water was less than 1 mg/ml (see Table 2).

**Table 2 ― Apparent solubility of a free base and various salt forms of 3-(1,2,4-triazole[4,3-a]pyridine-3-ylethinyl)-4-methyl-N-(4-((4-methylpiperazine-1-yl)methyl)-3-trifluoromethylphenyl)benzamide in water**

| Sample | Acid | Crystallinity (form) | Weight, mg | Temperature, °C | Water volume, ml | Apparent solubility, mg/ml |
|---|---|---|---|---|---|---|
| HAL-G-194-1 | (free base) | Form I | 4.6 | RT | 4.0^{#} | <1 |
| HAL-G-196-2 | HCI | Form I | 4.4 | | 0.15 | >29 |
| HAL-G-196-3 | HCI | Form II | 4.3 | | 1.25* | ~3 |
| HAL-G-196-6 | H2SO4 | Semi-crystalline | 4.5 | | 4.0^{#} | <1 |
| HAL-G-196-7 | HBr | Form I | 4.6 | | 1.0* | ~5 |
| HAL-G-196-8 | HBr | Form II | 4.8 | | 4.0^{#} | <1 |
| HAL-G-196-20 | Methanesulfonic acid | Crystalline | 4.6 | | 0.1 | >46 |
| HAL-G-196-24 | 4-methylbenzene sulfonic acid | Crystalline | 4.9 | | 4.0* | ~1 |
| HAL-G-196-29 | Fumaric acid | Crystalline | 4.1 | | 4.0^{#} | <1 |
| RT ― room temperature, | | | | | | |
| * ― muddy solution, | | | | | | |
| # ― no solution happened. | | | | | | |

| | | | | | | |
|---|---|---|---|---|---|---|
| Equilibrium solubility of a free base in deionized water was approximately 2.3 * 10⁻⁴ mg/ml based on HPLC analysis (see Table 3). | | | | | | |

**Table 3 ― Equilibrium solubility of a free base and various salt forms of 3-(1,2,4-triazole[4,3-a]pyridine-3-ylethinyl)-4-methyl-N-(4-((4-methylpiperazine-1-yl)methyl)-3-trifluoromethylphenyl)benzamide in water**

| Salt form | Sample code | Weight, mg | Water quantity, ml | Crystallinity | | pH | Solubility, mg/ml |
|---|---|---|---|---|---|---|---|
| | | | | initial | in 24 h | | |
| Free base | HAL-G-194-1 | 18.113 | 0.6 | Cryst. | Cryst. | 5.3 | 2.3 × 10⁻⁴ |
| Hydrochloride | HAL-G-196-3 | 21.939 | 0.6 | Form II | Amorphous | 4.0 | 37.1 |
| Mesylate | HAL-G-196-20 | 19.912 | 0.2 | Cryst. | Dissolution | 3.6 | > 100 |
| Tosylate | HAL-G-196-24 | 15.631 | 0.6 | Cryst. | Cryst. | 3.5 | 2.2 |
| Fumarate | HAL-G-196-29 | 18.960 | 0.6 | Cryst. | Cryst. | 4.2 | 7.4 × 10⁻³ |

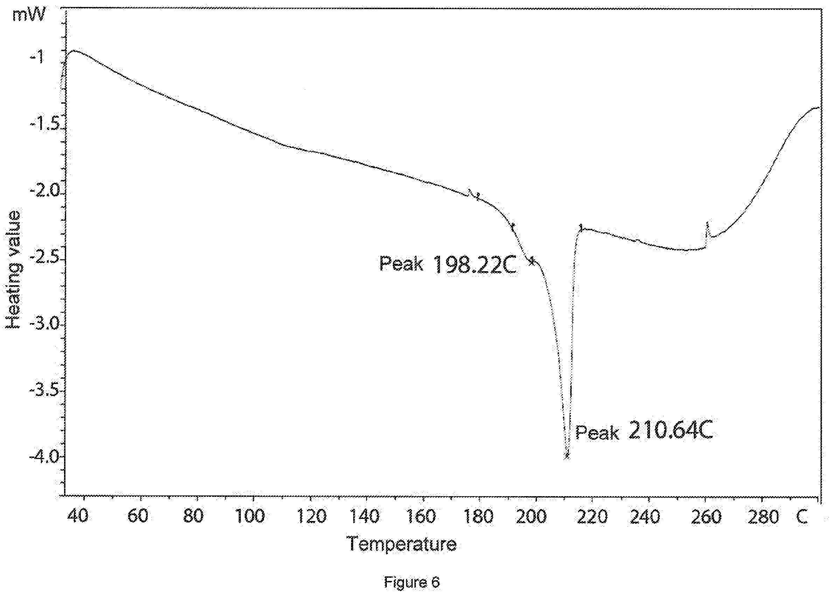

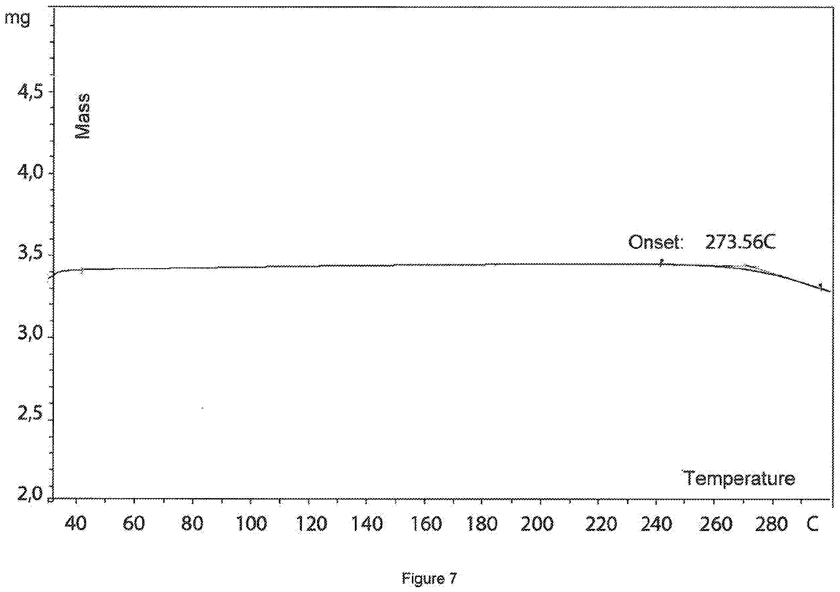

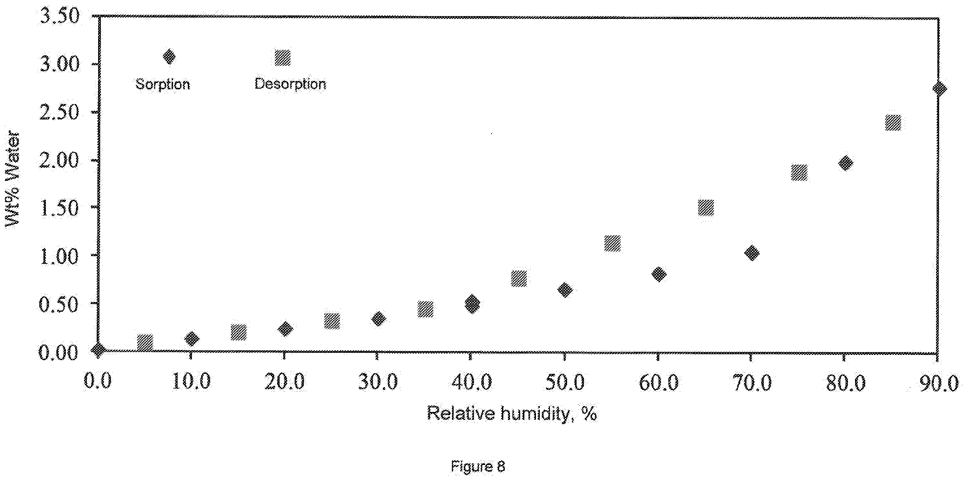

Results of samples tests using DSC and TG methods are given in Figures 6 and 7. DSC analysis of free base sample showed that the sample underwent no modifications when heated up to 198 °C, free base melts at 211 °C (see Fig. 6). During TG analysis, no weight loss of the sample was identified (see Fig. 7). Study of hygroscopicity of free base of 3-(1,2,4-triazole[4,3-a]pyridine-3-ylethinyl)-4-methyl-N-(4-((4-methylpiperazine-1-yl)methyl)-3-trifluoromethylphenyl)benzamide showed that at a relative air humidity of 90% the sample absorbed less than three mass per cent of water (see Fig. 8). The content of impurities remains constant when the sample is kept during seven days at a temperature of 60 °C (see Table 4).

**Table 4 ― Stability of free base and various salt forms of 3-(1,2,4-triazole[4,3-a]pyridine-3-ylethinyl)-4-methyl-N-(4-((4-methylpiperazine-1-yl)methyl)-3-trifluoromethylphenyl)benzamide (7 days at T = 60 °C)**

| Salt form | Sample code | Weight, mg | Relative peak area, initial, % | Relative peak area, in 7 days, % | Crystallinity | |
|---|---|---|---|---|---|---|
| | | | | | initial | in 7 days |
| Free base | HAL-G-194-1 | 12.7 | 99.7 | 99.7 | Form I | Form I |
| Hydrochloride | HAL-G-196-20 | 10.0 | 99.7 | 99.7 | Cryst. | Cryst. |
| Mesylate | HAL-G-196-24 | 8.8 | 99.8 | 99.9 | Cryst. | Cryst. |
| Tosylate | HAL-G-196-3 | 9.5 | 99.9 | 99.9 | Form II | Form II |
| Fumarate | HAL-G-196-29 | 12.2 | 99.8 | 99.9 | Cryst. | Cryst. |

Study of stability of free base polymorph modification of 3-(1,2,4-triazole[4,3-a]pyridine-3-ylethinyl)-4-methyl-N-(4-((4-methylpiperazine-1-yl)methyl)-3-trifluoromethylphenyl)benzamide with suspending in a solvent (acetone) during 6 days showed that crystalline structure of HAL-G-194-1 sample changes (see Fig. 3, Table 5). The structure and purity of the sample received were confirmed using 1H NMR spectroscopy (See Fig. 5).

**Table 5 ― Stability of free base polymorph modifications and various salt forms of 3-(1,2,4-triazole[4,3-a]pyridine-3-ylethinyl)-4-methyl-N-(4-((4-methylpiperazine-1-yl)methyl)-3-trifluoromethylphenyl)benzamide (suspending in acetone for 6 days)**

| Salt form | Sample code | Weight, mg | Quantity of acetone, ml | Temperature | Crystallinity | |
|---|---|---|---|---|---|---|
| | | | | | initial | in 6 days |
| Free base | HAL-G-194-1 | 36.5 | 0.3 | Room | Form I | Form II |
| Hydrochloride | HAL-G-200-9 | 22.5 | | | Form II | Form II |
| Mesylate | HAL-G-200-10 | 20.8 | | | Cryst. | Cryst. |
| Tosylate | HAL-G-196-21 | 19.4 | | | Cryst. | Cryst. |
| Fumarate | HAL-G-196-30 | 19.0 | | | Cryst. | Cryst. |

### Study of physical and chemical properties of chloride and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorph modification I)

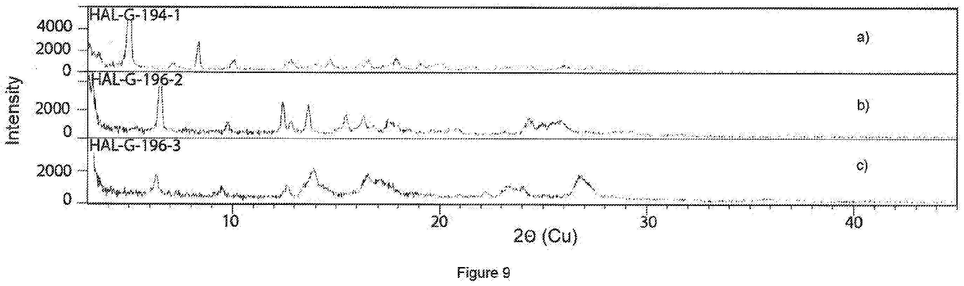

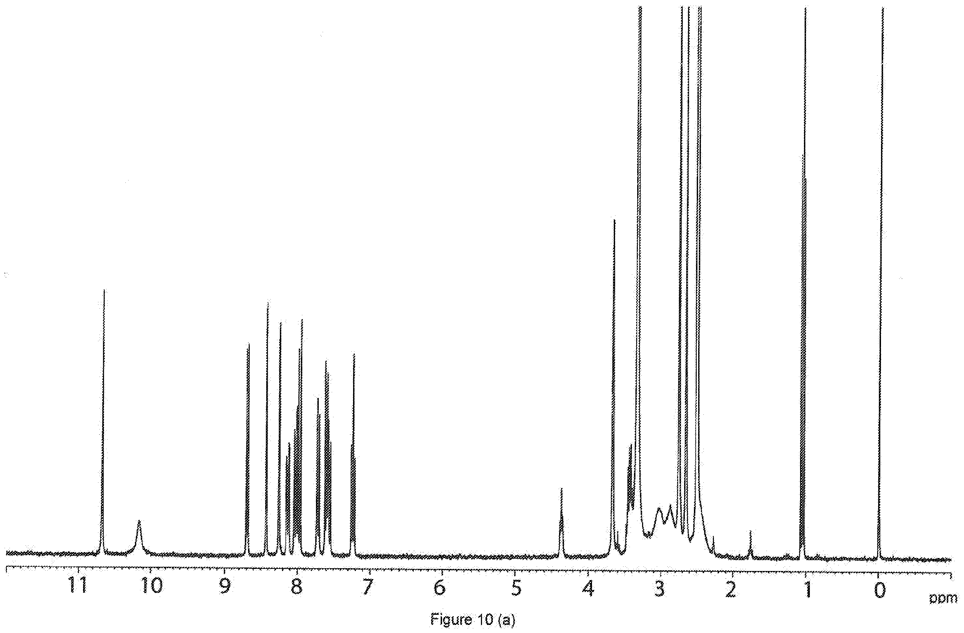

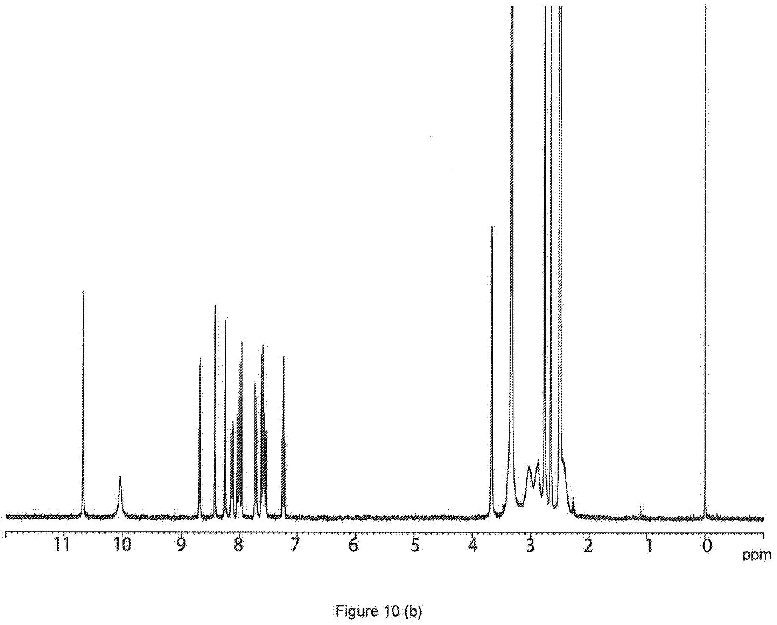

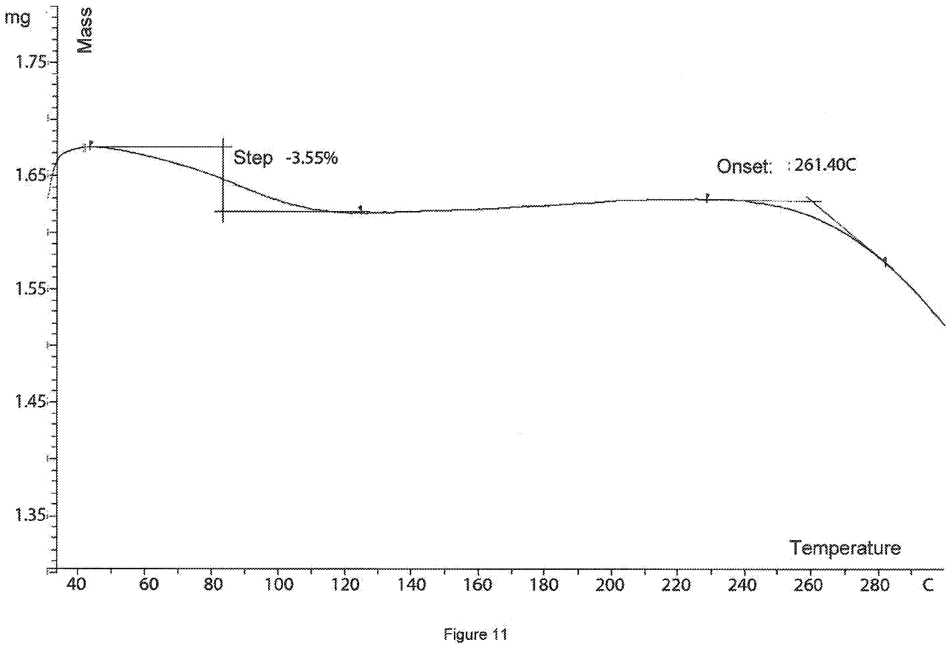

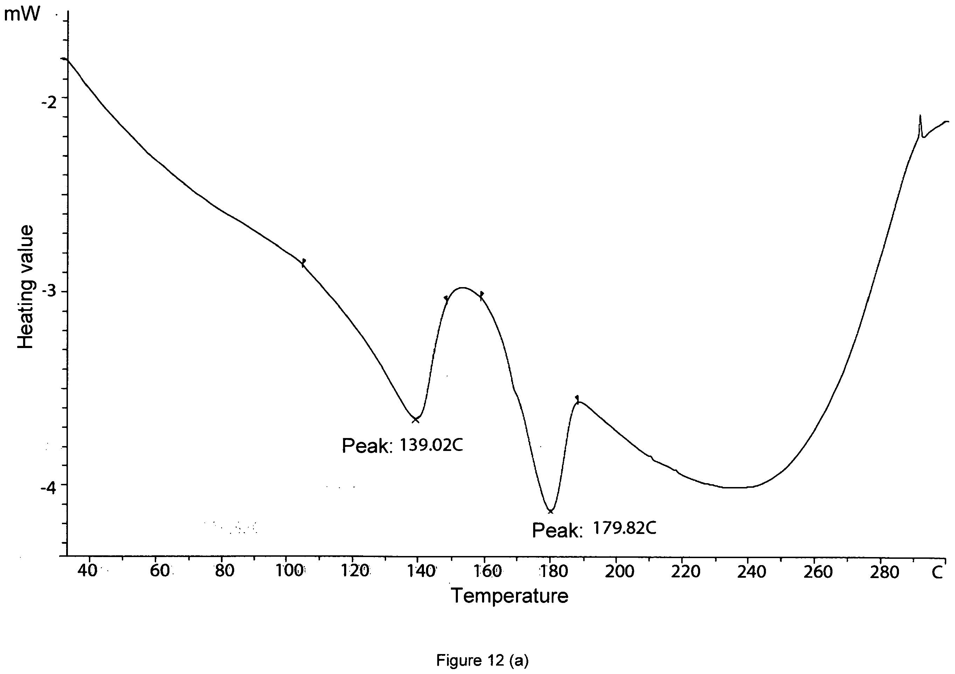

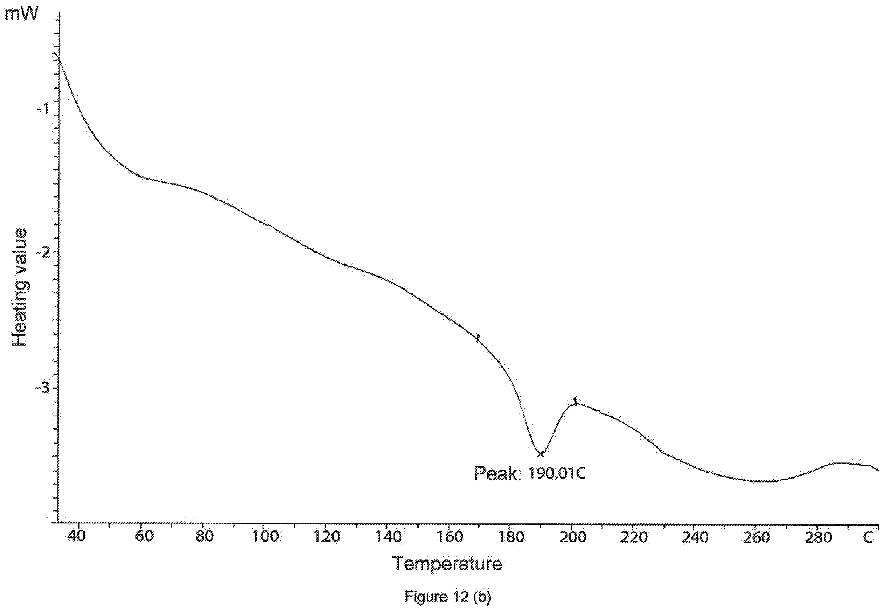

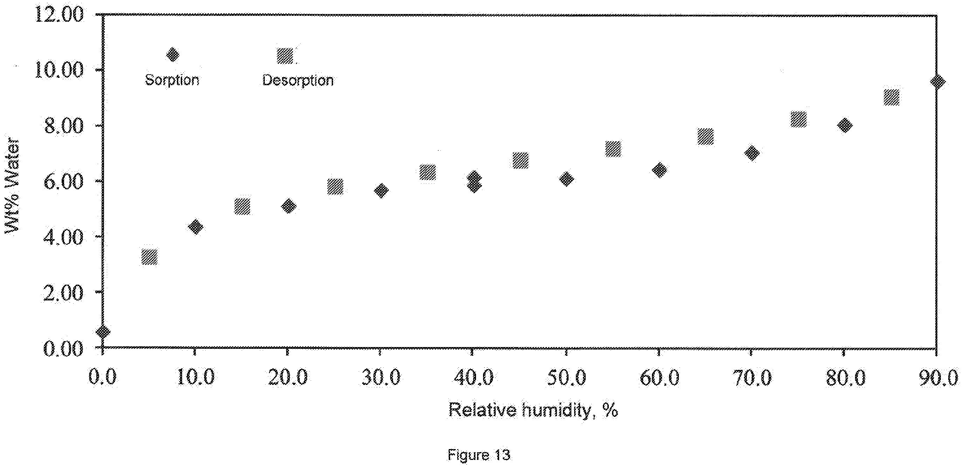

Sample of HAL-G-196-2 chloride and 3-(1,2,4-triazole[4,3-a]pyridine-3-ylethinyl)-4-methyl-N-(4-((4-methylpiperazine-1-yl)methyl)-3-trifluoromethylphenyl)benzamide obtained in the tetrahydrofuran (THF) based on X-ray powder diffraction (see Fig. 9), is an individual crystalline phase. The same crystalline phase was revealed in the sample of chloride and free base obtained in ethanol media (HAL-G-196-1 sample). The compound structure was confirmed with 1H NMR spectroscopy (see Fig. 10). It should be noted that 1H NMR spectrum of HAL-G-196-1 and HAL-G-196-2 samples contains signals of residual solvents. Anion and cation stoichiometric proportion determined using ion chromatography confirms formation of monochloride. DSC analysis (see Fig. 11) resulted in identification of two endothermic transitions ― first one (T = 139 °C) corresponds to solvent loss, second one (T = 180 °C) ― sample melting. During TG analysis, the sample weight loss by 3.6% was observed, which was probably caused by loss of residual solvent quantities (see Fig. 12). Study of sample hygroscopicity showed that HAL-G-196-2 sample probably was a dehydrate, as it desorbed and sorbed water, volume of which corresponded to a dehydrate (see Fig. 13). Further sample studies were recognized as unfeasible.

### Study of physical and chemical properties of chloride and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorph modification II)

Sample of HAL-G-196-3 chloride and 3-(1,2,4-triazole[4,3-a]pyridine-3-ylethinyl)-4-methyl-N-(4-((4-methylpiperazine-1-yl)methyl)-3-trifluoromethylphenyl)benzamide obtained in the acetone media after adding methyl tert-butyl ether (MTBE) based on X-ray powder diffraction (see Fig. 9), is an individual crystalline phase, which is different from the crystalline phase of HAL-G-196-1 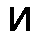 HAL-G-196-2. The compound structure was confirmed with 1H NMR spectroscopy (see Fig. 10). Anion and cation stoichiometric proportion determined using ion chromatography confirms formation of monochloride. DSC analysis (see Fig. 11) resulted in identification of one endothermic transition (T = 190 °C) corresponding to sample melting. During TG analysis, no sample weight loss was observed. The apparent solubility of HAL-G-196-3 sample in deionized water amounted to around 3 mg/ml (see Table 2). Equilibrium solubility of chloride and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorph modification II) in deionized water was around 37.1 mg/ml according to the data of HPLC analysis (see Table 3). The study of sample hygroscopicity showed that HAL-G-196-3 sample absorbs less than 8 mass per cent of water at a relative air humidity of 90% (see Fig. 13). The content of impurities remains constant when the sample is kept during seven days at a temperature of 60 °C (see Table 4). Study of stability of HAL-G-196-3 sample with suspending in a solvent (acetone) during 6 days showed that crystalline structure of HAL-G-196-3 sample remained unchanged (see Table 5). Therefore, chloride and 3-(1,2,4-triazole[4,3-a]pyridine-3-ylethinyl)-4-methyl-N-(4-((4-methylpiperazine-1-yl)methyl)-3-trifluoromethylphenyl)benzamide by its physical and chemical properties met requirements set in the course of salt form optimization.

### Study of physical and chemical properties of sulfate and 3-(1,2,4-triazole[4,3-a]pyridine-3-ylethinyl)-4-methyl-N-(4-((4-methylpiperazine-1-yl)methyl)-3-trifluoromethylphenyl)benzamide

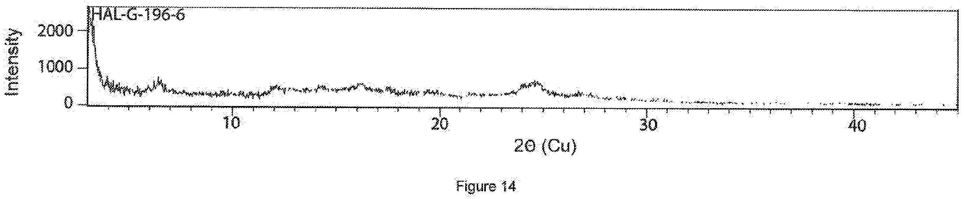

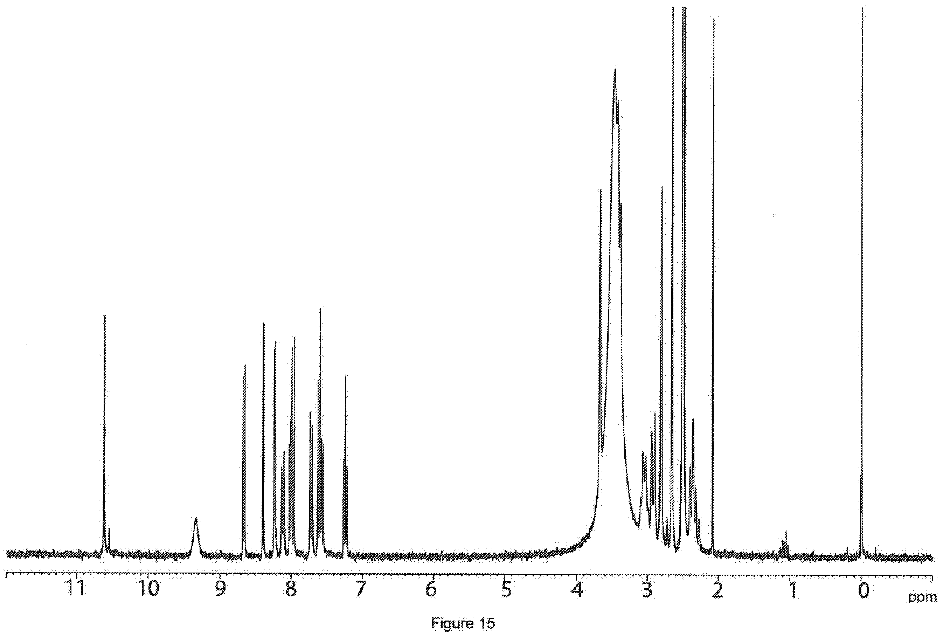

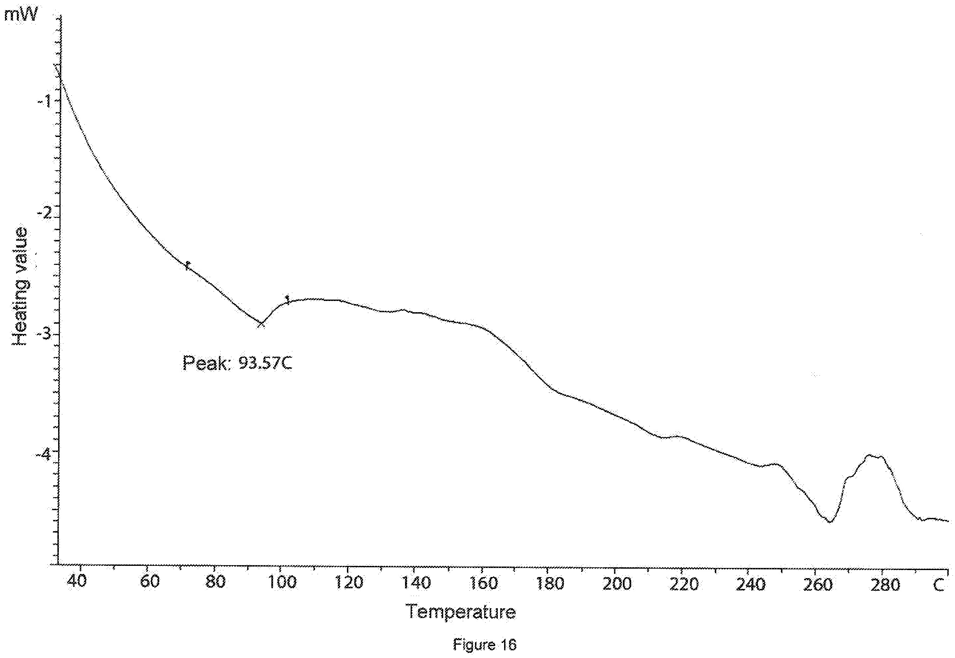

HAL-G-196-6 sample of sulfate and 3-(1,2,4-triazole[4,3-a]pyridine-3-ylethinyl)-4-methyl-N-(4-((4-methylpiperazine-1-yl)methyl)-3-trifluoromethylphenyl)benzamide obtained in the acetone media based on X-ray powder diffraction (see Fig. 14), contains a combination of crystalline phases, as the diffusion nature of peaks and inability to establish and describe diffraction patterns using phase reflections with one elementary cell demonstrated presence of several crystalline phases and, probably, significant share of amorphous phase in the test sample. The compound structure was confirmed with 1H NMR spectroscopy (see Fig. 15). It should be mentioned that 1H NMR spectrum of HAL-G-196-6 sample contains signals of the residual solvent. Anion and cation stoichiometric proportion determined using ion chromatography confirms formation of monosulfate. DSC analysis (see Fig. 16) resulted in identification of one endothermic transition, which probably corresponded to solvent loss. Absence of express endothermic transition corresponding to substance melting demonstrates that there is a great share of amorphous phase in the test sample. Apparent solubility of sulfate and 3-(1,2,4-triazole[4,3-a]pyridine-3-ylethinyl)-4-methyl-N-(4-((4-methylpiperazine-1-yl)methyl)-3-trifluoromethylphenyl)benzamide in deionized water was around 1 mg/ml (see Table 2). Further sample studies were recognized as unfeasible due to low solubility of this salt.

### Study of physical and chemical properties of bromide and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorph modification I)

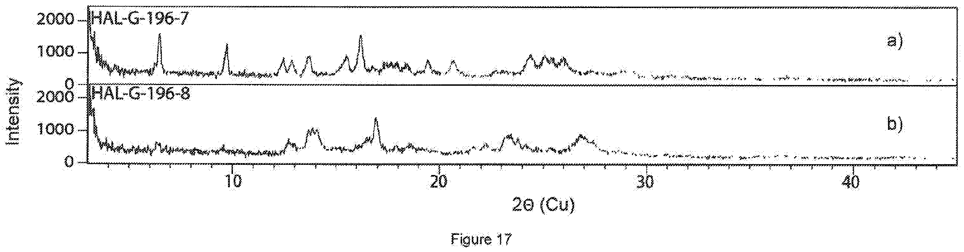

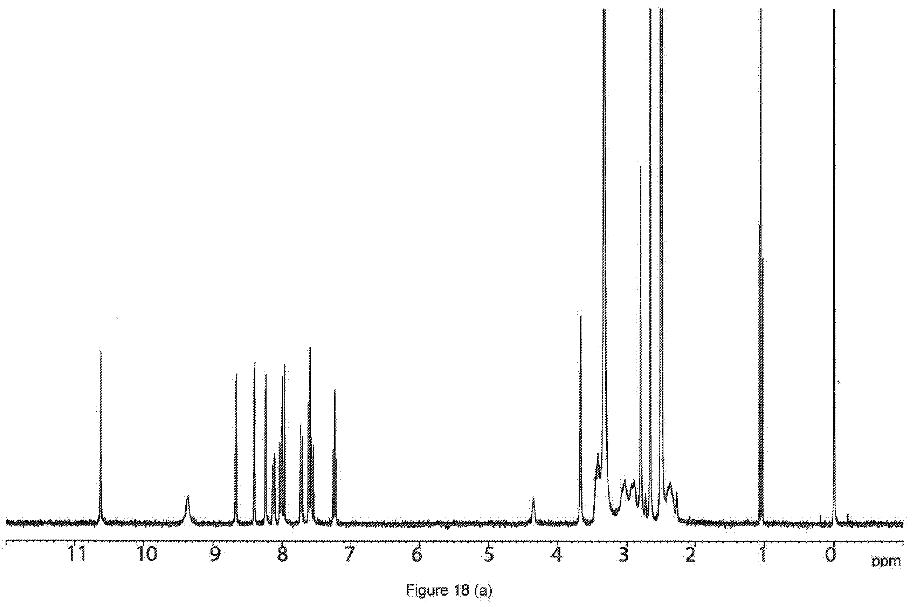

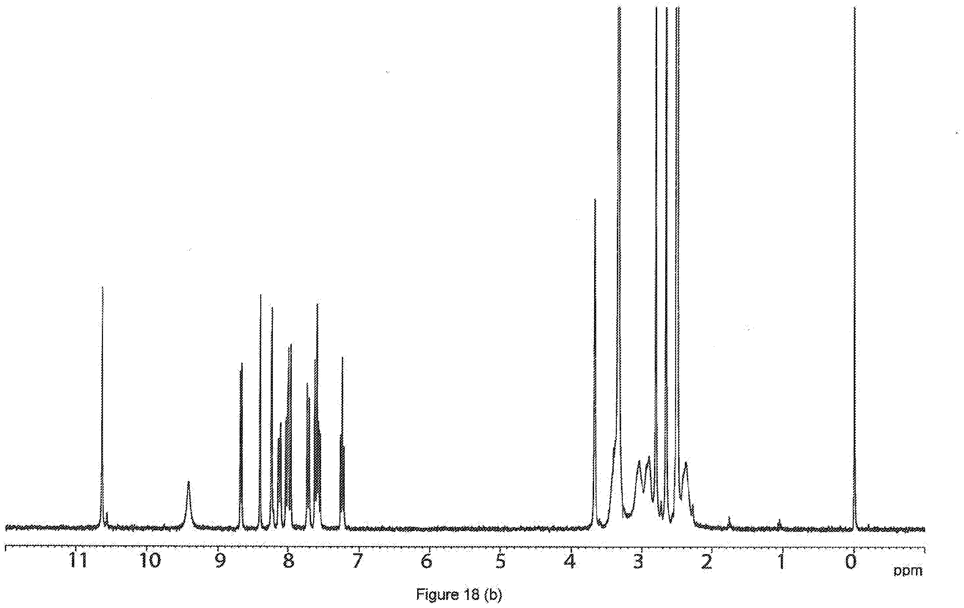

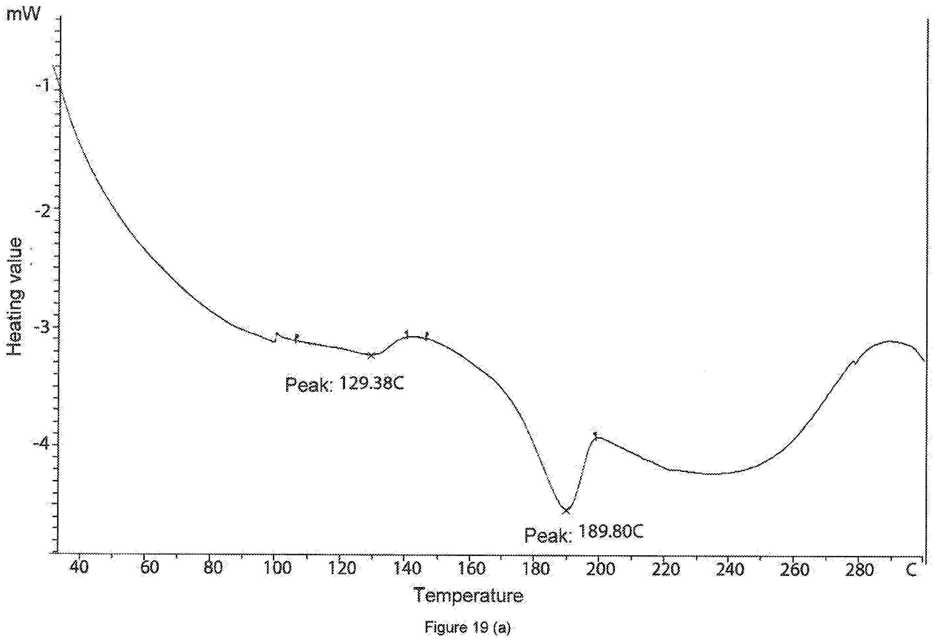

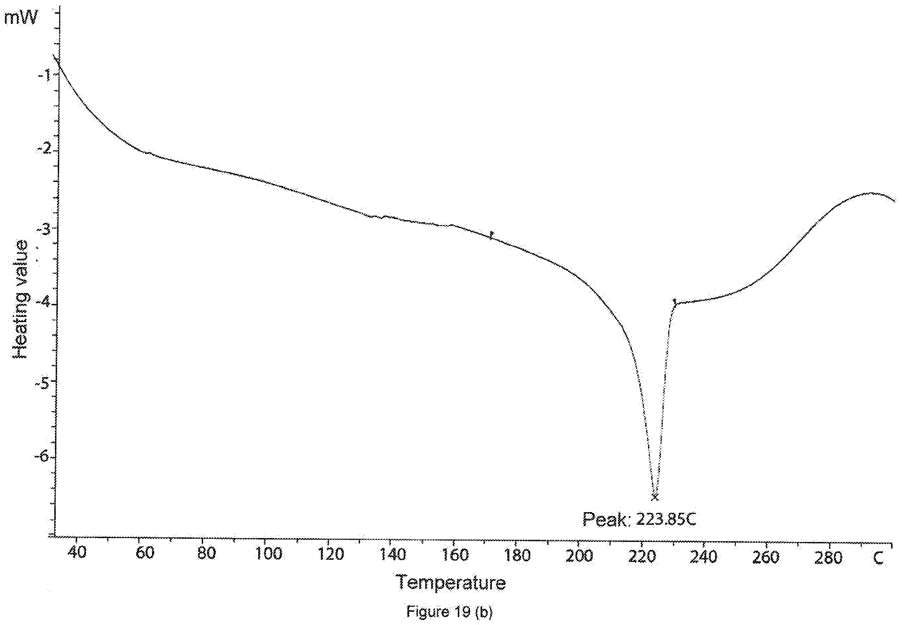

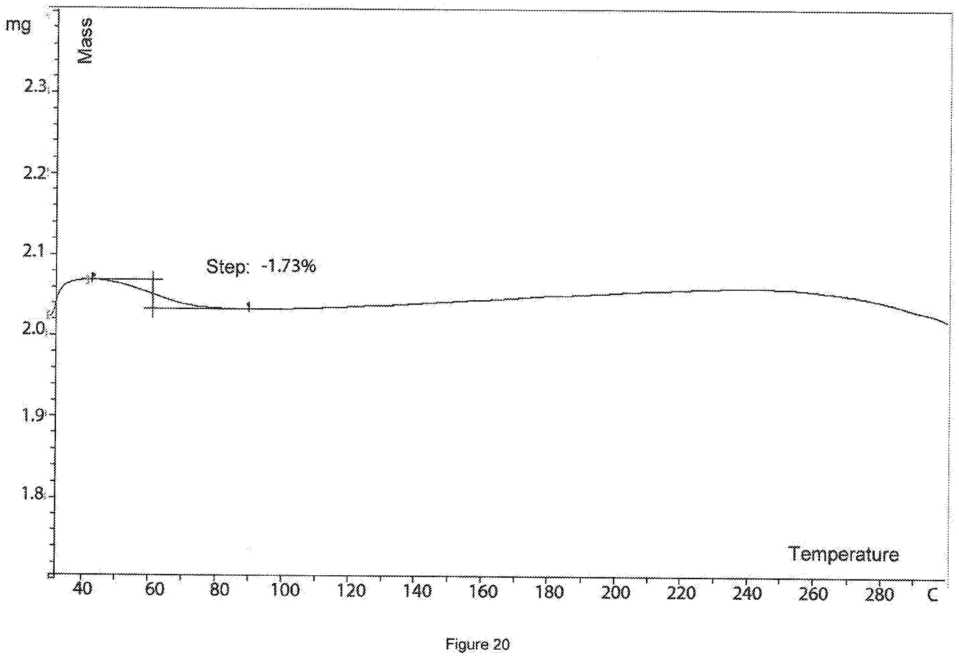

HAL-G-196-7 sample of bromide and 3-(1,2,4-triazole[4,3-a]pyridine-3-ylethinyl)-4-methyl-N-(4-((4-methylpiperazine-1-yl)methyl)-3-trifluoromethylphenyl)benzamide obtained via crystallization in the ethanol media based on X-ray powder diffraction (see Fig. 17) is an individual crystalline phase. The compound structure was confirmed with 1H NMR spectroscopy (see Fig. 18). 1H NMR spectrum of HAL-G-196-7 sample contains signals of the residual solvent. Anion and cation stoichiometric proportion determined using ion chromatography confirms formation of monobromide. DSC analysis (see Fig. 19) resulted in identification of two endothermic transitions ― first one (T = 129 °C) corresponds to solvent loss, second one (T = 190 °C) ― sample melting. During TG analysis, the sample weight loss by 1.7% was observed, which was probably caused by loss of residual solvent quantities (see Fig. 20). Apparent solubility of bromide and 3-(1,2,4-triazole[4,3-a]pyridine-3-ylethinyl)-4-methyl-N-(4-((4-methylpiperazine-1-yl)methyl)-3-trifluoromethylphenyl)benzamide in deionized water was around 5 mg/ml (see Table 2). Study of sample hygroscopicity showed that at a relative air humidity of 90% the sample absorbed more than ten mass per cent of water and is spread in the air. This is why further sample studies were recognized as unfeasible.

### Study of physical and chemical properties of bromide and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorph modification II)

HAL-G-196-8 sample of bromide and 3-(1,2,4-triazole[4,3-a]pyridine-3-ylethinyl)-4-methyl-N-(4-((4-methylpiperazine-1-yl)methyl)-3-trifluoromethylphenyl)benzamide obtained via crystallization in the tetrahydrofuran (THF) media based on X-ray powder diffraction (see Fig. 17) is an individual crystalline phase. The same crystalline phase was revealed in bromide and free base sample obtained via crystallization from acetone (HAL-G-196-9 sample). The compound structure was confirmed with 1H NMR spectroscopy (see Fig. 18). 1H NMR spectrum of HAL-G-196-8 and HAL-G-196-9 samples contains signals of residual solvents. Anion and cation stoichiometric proportion in HAL-G-196-8 and HAL-G-196-9 samples determined using ion chromatography confirms formation of monobromide. DSC analysis (see Fig. 19) resulted in identification of one endothermic transition (T = 224 °C), which corresponded to sample melting. TG analysis revealed no weight loss of the sample. Apparent solubility of bromide and 3-(1,2,4-triazole[4,3-a]pyridine-3-ylethinyl)-4-methyl-N-(4-((4-methylpiperazine-1-yl)methyl)-3-trifluoromethylphenyl)benzamide in deionized water was less than 1 mg/ml (see Table 2). Further sample studies were recognized as unfeasible due to low solubility of this salt.

### Study of physical and chemical properties of phosphate and 3-(1,2,4-triazole[4,3-a]pyridine-3-ylethinyl)-4-methyl-N-(4-((4-methylpiperazine-1-yl)methyl)-3-trifluoromethylphenyl)benzamide

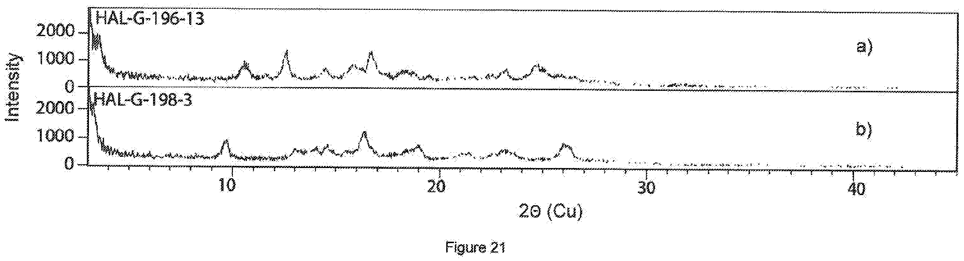

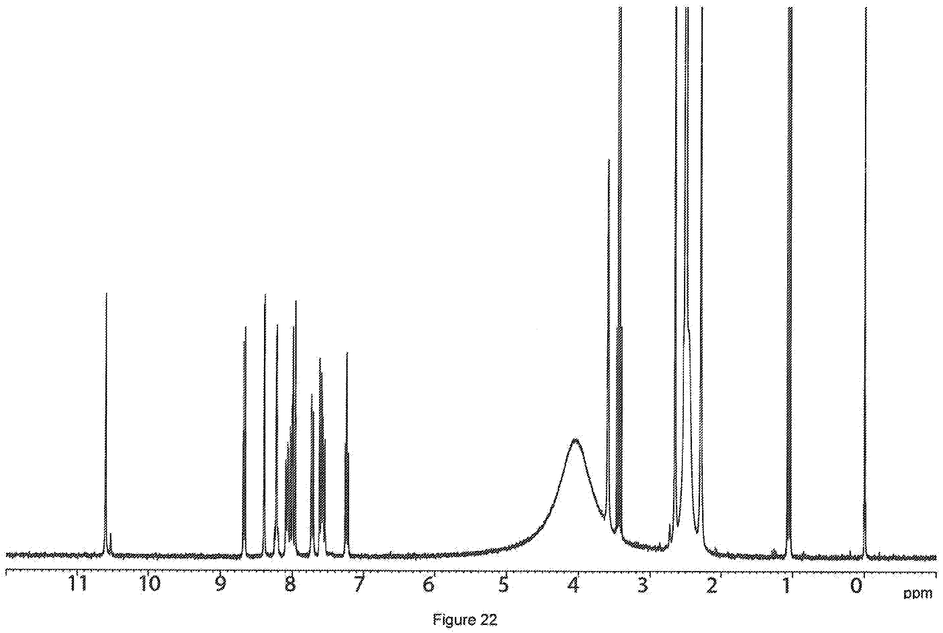

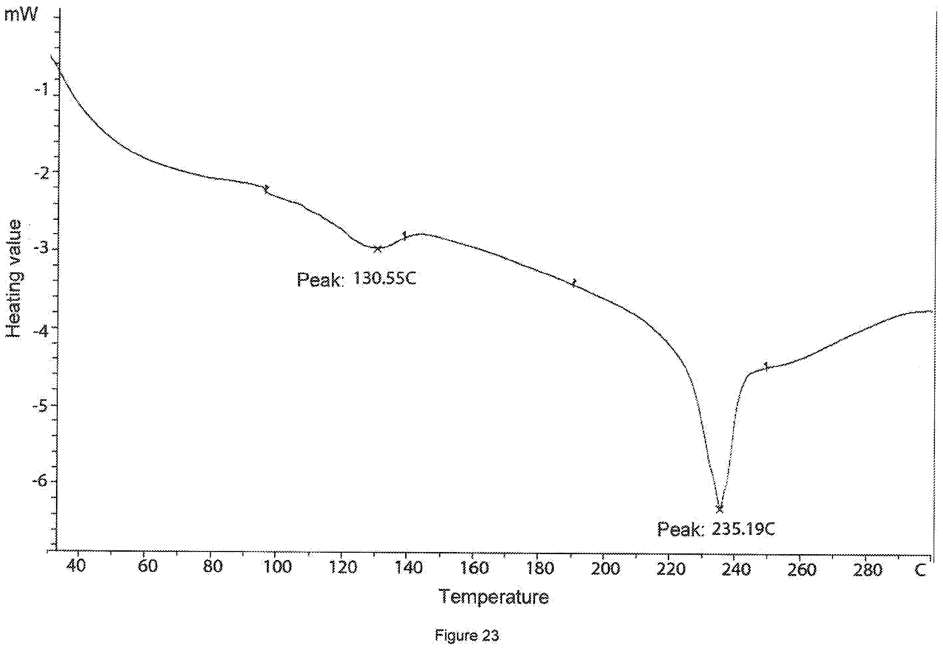

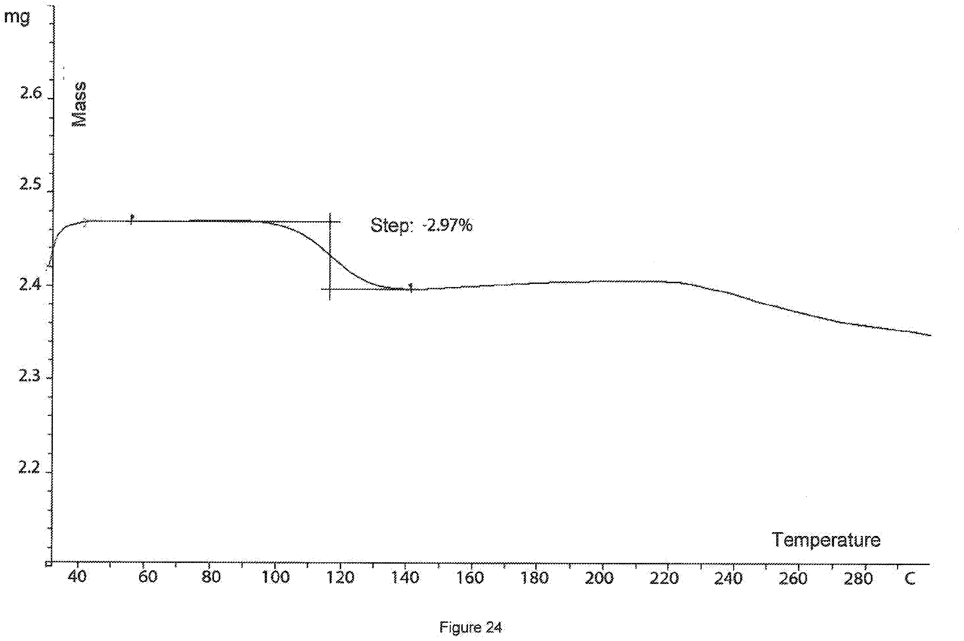

HAL-G-196-13 sample of phosphate and 3-(1,2,4-triazole[4,3-a]pyridine-3-ylethinyl)-4-methyl-N-(4-((4-methylpiperazine-1-yl)methyl)-3-trifluoromethylphenyl)benzamide obtained via crystallization in the ethanol media based on X-ray powder diffraction (see Fig. 21), is an individual crystalline phase. The compound structure was confirmed with 1H NMR spectroscopy (see Fig. 22). 1H NMR spectrum of HAL-G-196-13 sample contains signals of residual solvent. Anion and cation stoichiometric proportion determined using ion chromatography confirms formation of dihydrophosphate. DSC analysis (see Fig. 23) resulted in identification of two endothermic transitions ― first one (T = 131 °C) corresponds to solvent loss, second one (T = 235 °C) ― sample melting. During TG analysis, the sample weight loss by 3% was observed, which was probably caused by loss of residual solvent quantities (see Fig. 24). HAL-G-196-13 sample is probably a solvate containing ethanol in the crystalline structure, which was confirmed by X-ray powder diffraction data (see Fig. 21). Further sample studies were recognized as unfeasible due to the content of the solvent in the crystalline structure of this salt.

### Study of physical and chemical properties of tartrate and 3-(1,2,4-triazole[4,3-a]pyridine-3-ylethinyl)-4-methyl-N-(4-((4-methylpiperazine-1-yl)methyl)-3-trifluoromethylphenyl)benzamide

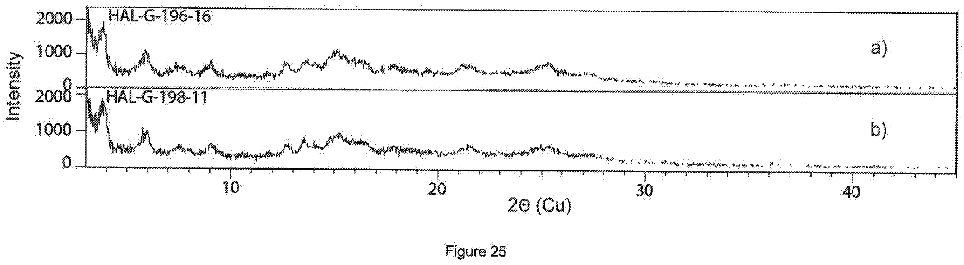

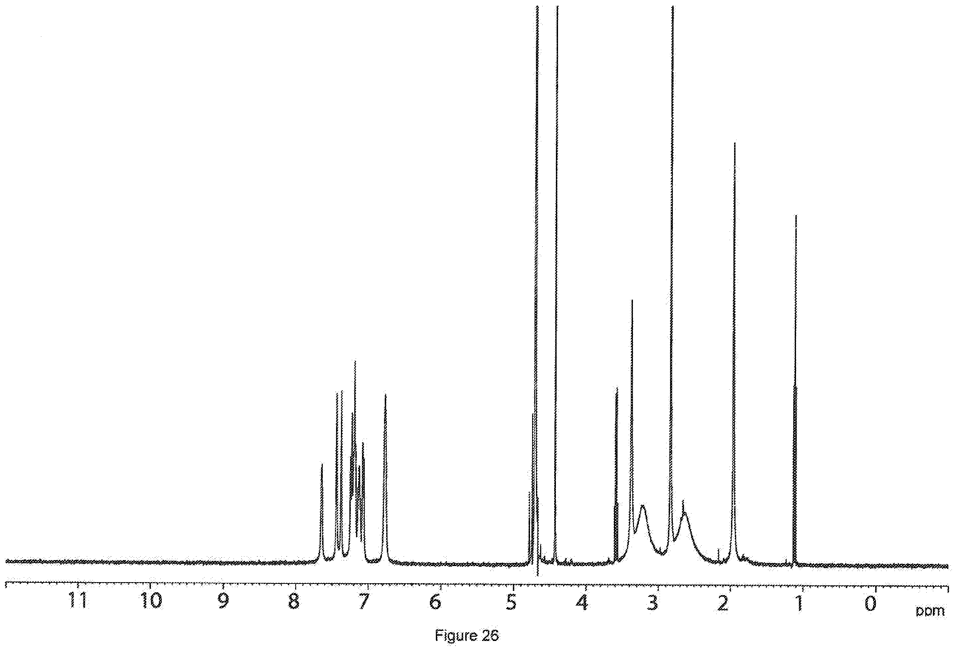

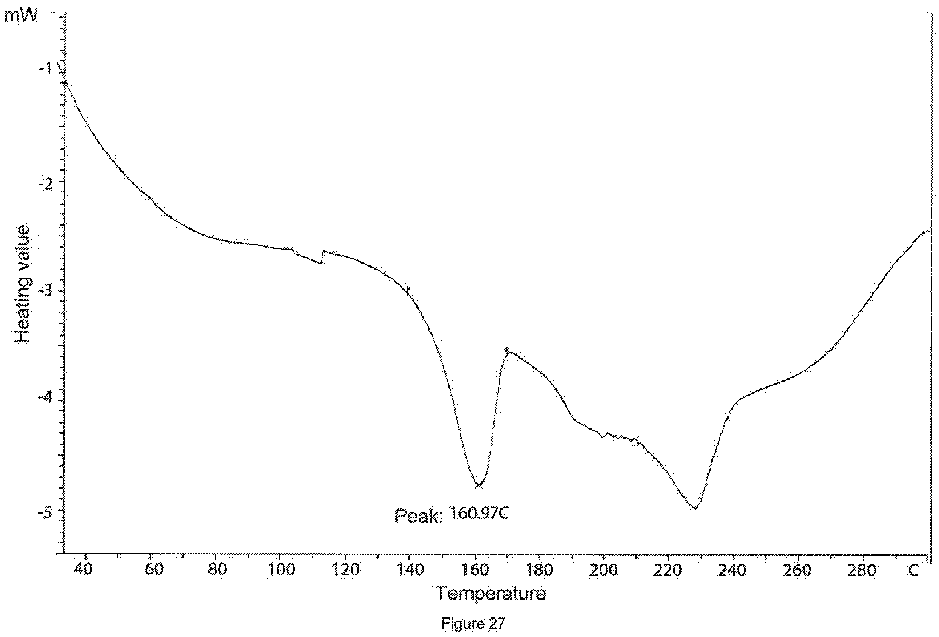

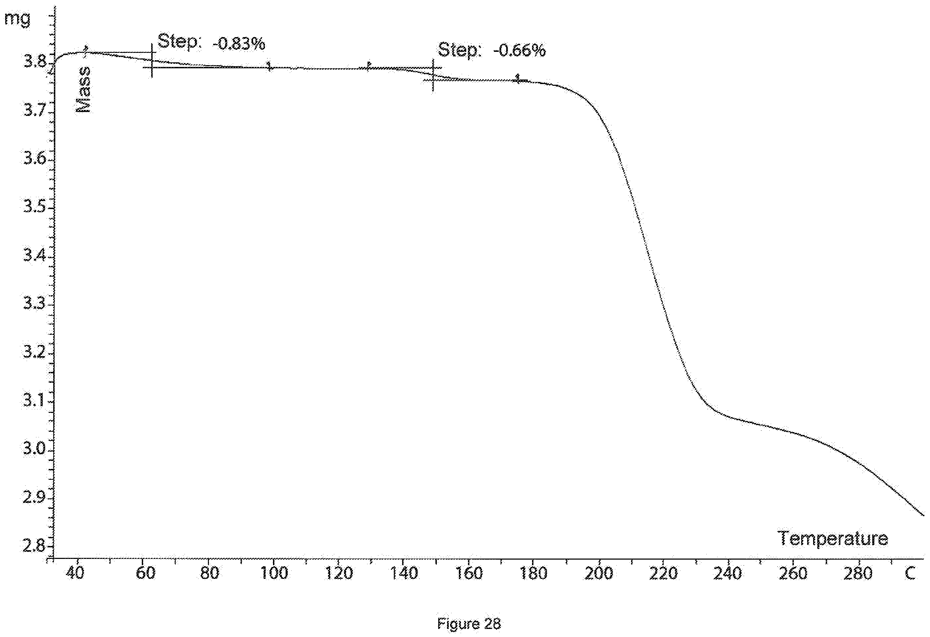

HAL-G-196-16 sample of tartrate and 3-(1,2,4-triazole[4,3-a]pyridine-3-ylethinyl)-4-methyl-N-(4-((4-methylpiperazine-1-yl)methyl)-3-trifluoromethylphenyl)benzamide obtained via crystallization in the ethanol media based on X-ray powder diffraction (see Fig. 25), is an individual crystalline phase. The same crystalline phase was revealed in the sample of tartrate and free base obtained via crystallization in THF media (HAL-G-196-17 sample). The compound structure was confirmed with 1H NMR spectroscopy (see Fig. 26). 1H NMR spectrum of HAL-G-196-8 and HAL-G-196-9 samples contain signals of residual solvents. Anion and cation stoichiometric proportion in HAL-G-196-16 and HAL-G-196-17 samples determined using ion chromatography confirms formation of monotartrate for each sample. DSC analysis (see Fig. 27) resulted in identification of one endothermic transition (T = 161 °C) corresponding to solvent loss and sample melting. During TG analysis of HAL-G-196-16 sample, weight loss by 0.8% at a temperature range of 30-100 °C and then additional weight loss by 0.7% at a temperature range of 130-170 °C were observed, which was probably due to partial degradation of the sample (see Fig. 28). HAL-G-196-16 sample is probably a solvate containing ethanol in the crystalline structure, which was confirmed by X-ray powder diffraction data (see Fig. 25). Further sample studies were recognized as unfeasible due to the content of the solvent in the crystalline structure of this salt.

### Study of physical and chemical properties methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide

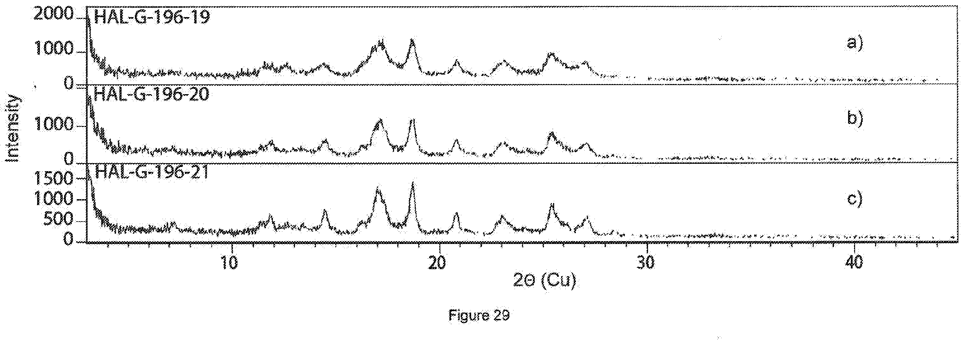

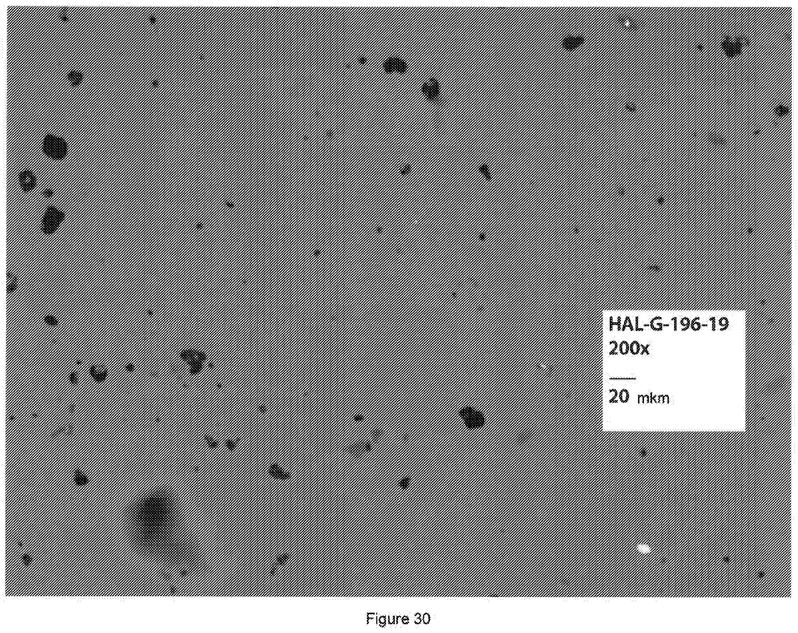

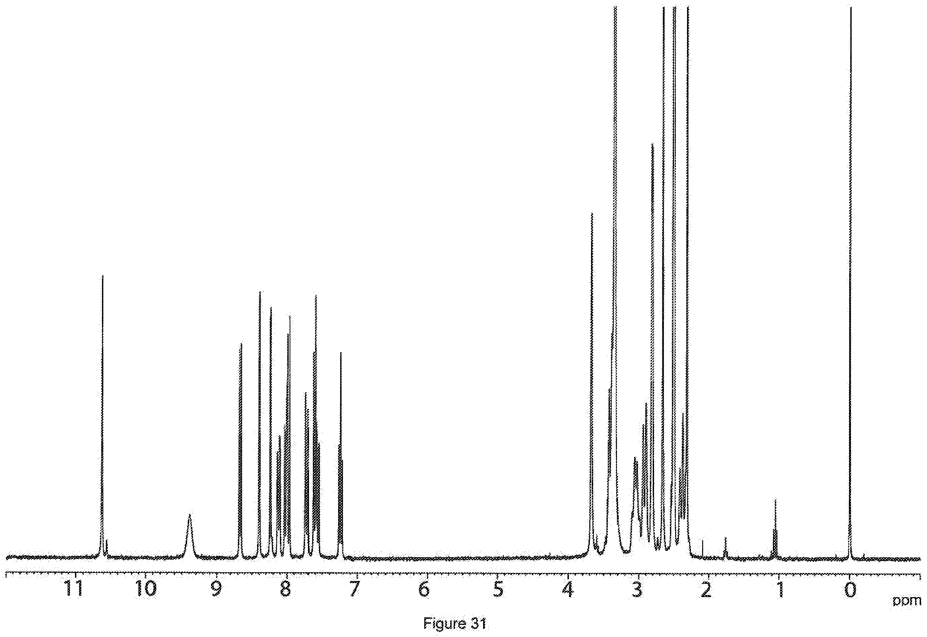

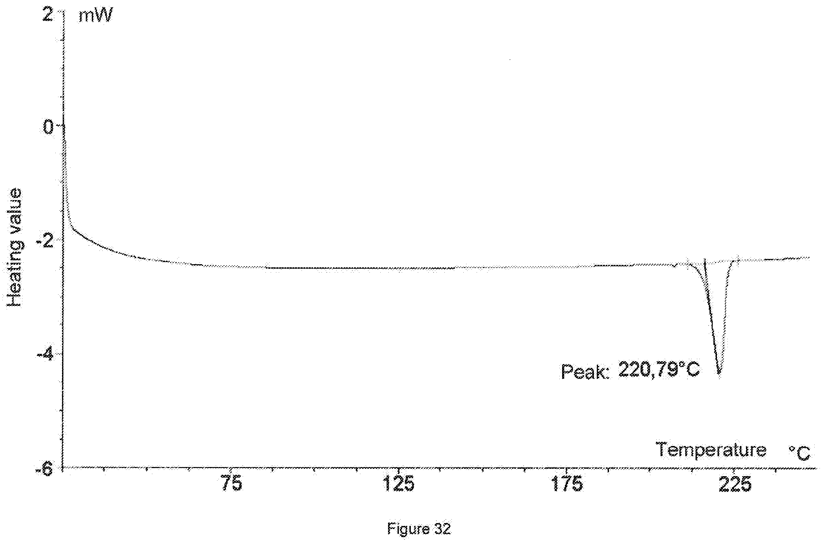

HAL-G-196-21 sample of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide obtained via crystallization in the acetone media based on X-ray powder diffraction (see Fig. 29) is an individual crystalline phase, which was also confirmed by polarization microscopy (see Fig. 30). The same crystalline phase was revealed in the sample of methanesulfonic acid salt and free base obtained via crystallization in THF media (HAL-G-196-20 sample) and ethanol media (HAL-G-196-19 sample). The compound structure was confirmed with 1H NMR spectroscopy (see Fig. 31). Anion and cation stoichiometric proportion in HAL-G-196-19, HAL-G-196-20 and HAL-G-196-21 samples determined using ion chromatography confirms formation of monomesylate for each sample. DSC analysis of these samples (see Fig. 32) resulted in identification of one endothermic transition (T = 220 °C) corresponding to sample melting. During TG analysis, no sample weight loss was observed. Study of hygroscopicity of sample of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide showed that at a relative air humidity of 90% the sample absorbed less than two mass per cent of water. The apparent solubility of free base in deionized water was more than 46 mg/ml (see Table 2). Equilibrium solubility of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide in deionized water was more than 100 mg/ml according to the data of HPLC analysis (see Table 3). The content of impurities remains constant when the sample is kept during seven days at a temperature of 60 °C (see Table 4). Study of stability of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide treated by a solvent (acetone) during 6 days showed that crystalline structure of the sample remained unchanged (see Table 5). Therefore, methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide by its physical and chemical properties met requirements set in the course of salt form optimization.

### Study of physical and chemical properties of 4-methylbenzene sulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide

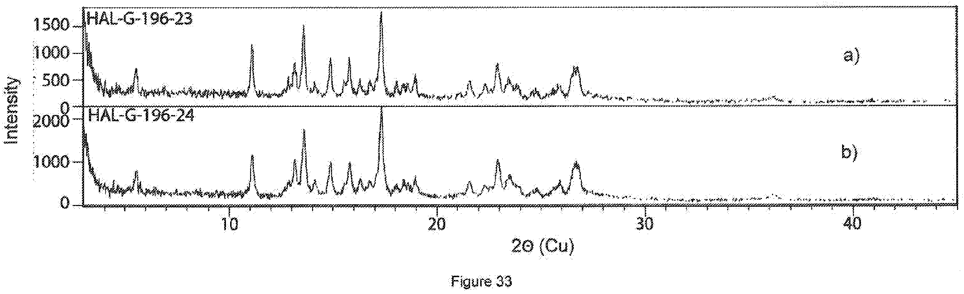

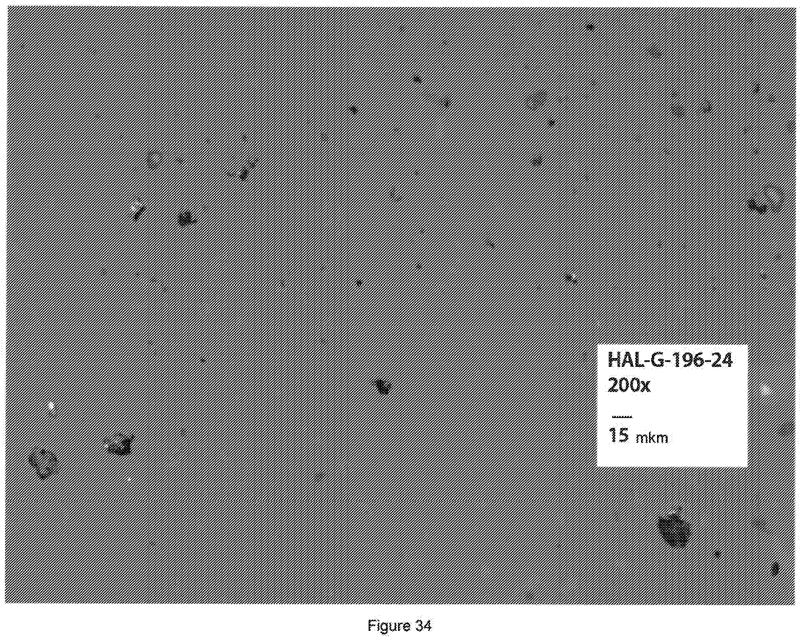

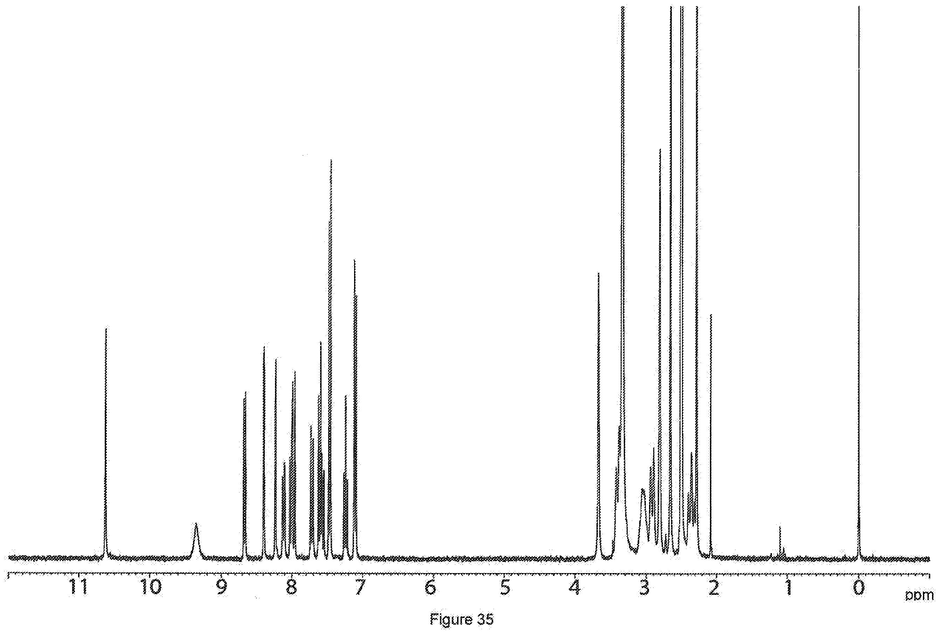

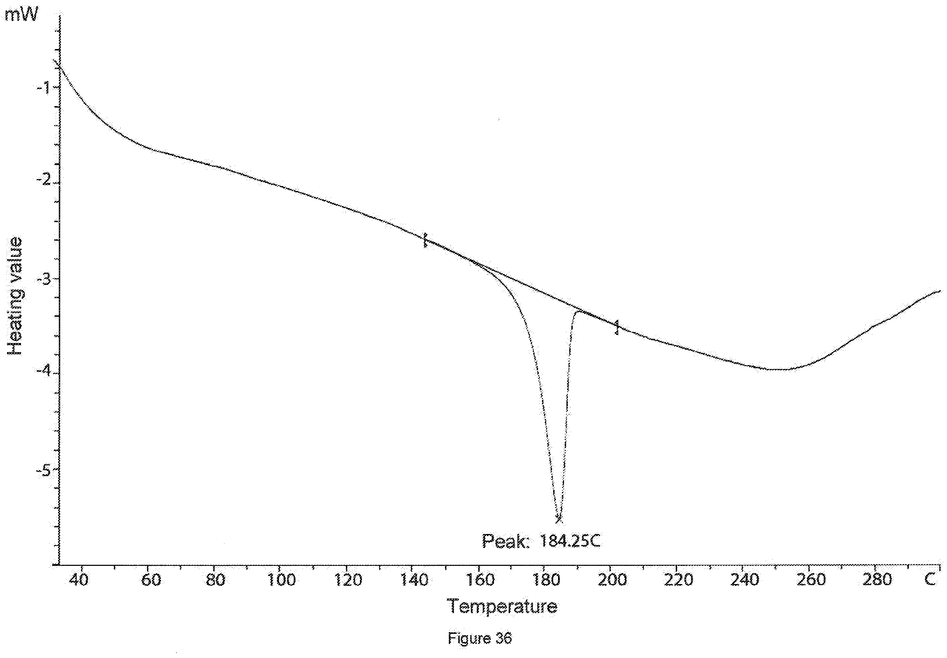

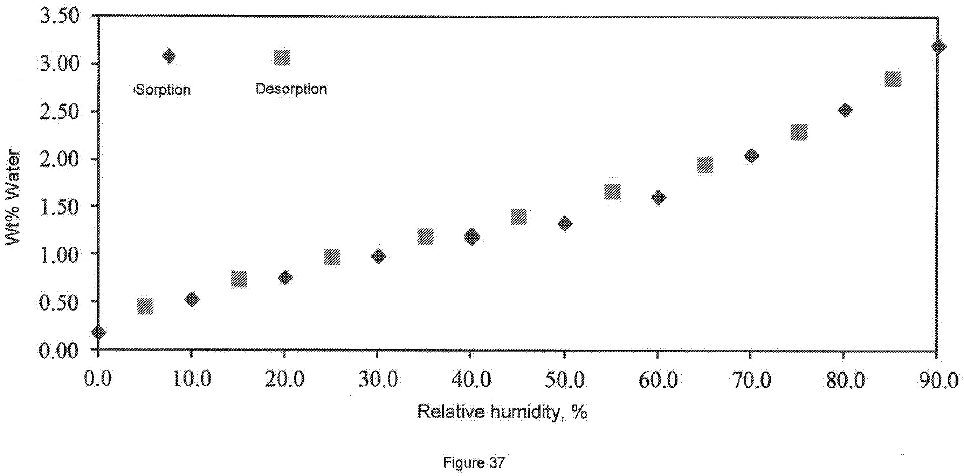

HAL-G-196-24 sample of 4-methylbenzene sulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide obtained via crystallization in the acetone media based on X-ray powder diffraction (see Fig. 33) is an individual crystalline phase, which was also confirmed by polarization microscopy (see Fig. 34). The same crystalline phase was revealed in samples of 4-methylbenzene sulfonic acid salt and free base obtained via crystallization in THF media (HAL-G-196-23 sample). The compound structure was confirmed with 1H NMR spectroscopy (see Fig. 35). Anion and cation stoichiometric proportion in HAL-G-196-23 and HAL-G-196-24 samples determined using ion chromatography confirms formation of monotosylate for each sample. DSC analysis of HAL-G-196-24 sample (see Fig. 36) resulted in identification of one endothermic transition (T = 184 °C) corresponding to sample melting. During TG analysis, no sample weight loss was observed. Study of sample hygroscopicity showed that at a relative air humidity of 90% HAL-G-196-24 sample absorbed less than four mass per cent of water (see Fig. 37). The apparent solubility of free base in deionized water was less than 1 mg/ml (see Table 2). Equilibrium solubility of 4-methylbenzene sulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide in deionized water was 2.2 mg/ml according to HPLC analysis data (see Table 3). The content of impurities remains constant when the sample is kept during seven days at a temperature of 60 °C (see Table 4). Study of stability of 4-methylbenzene sulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide treated by a solvent (acetone) during 6 days showed that crystalline structure of the sample remained unchanged (see Table 5). Therefore, 4-methylbenzene sulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide by its physical and chemical properties met requirements set in the course of salt form optimization.

### Study of physical and chemical properties of malate and 3-(1,2,4-triazole[4,3-a]pyridine-3-ylethinyl)-4-methyl-N-(4-((4-methylpiperazine-1-yl)methyl)-3-trifluoromethylphenyl)benzamide

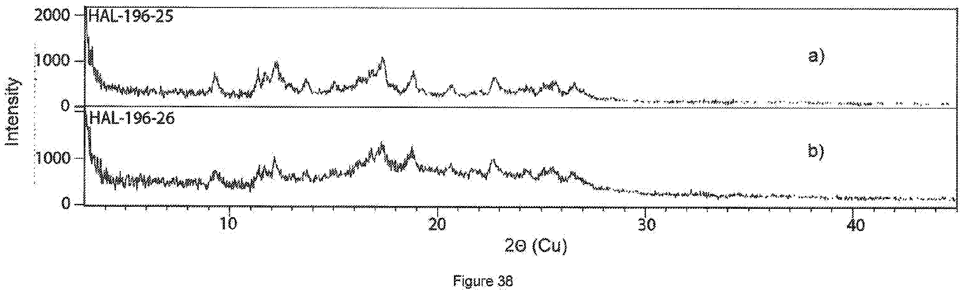

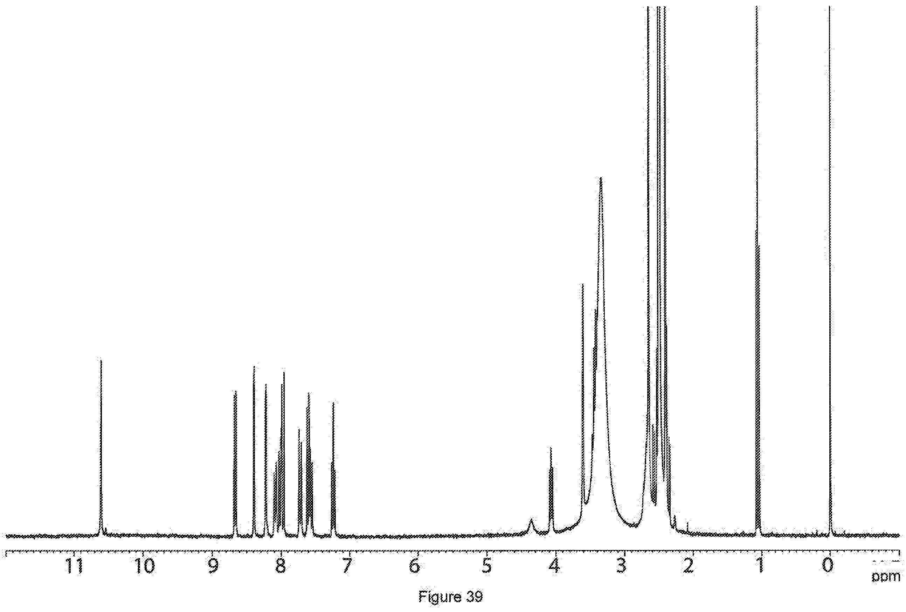

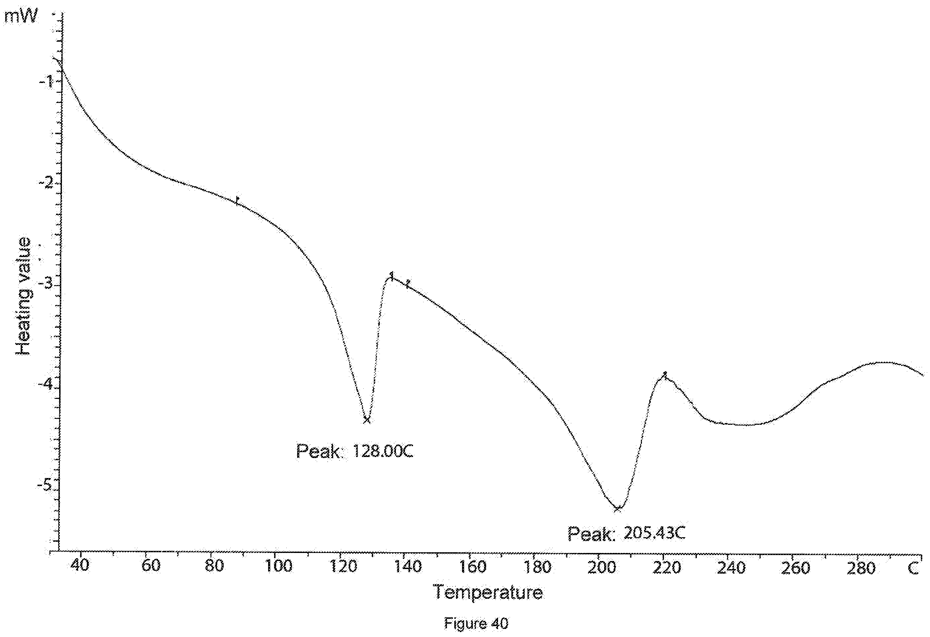

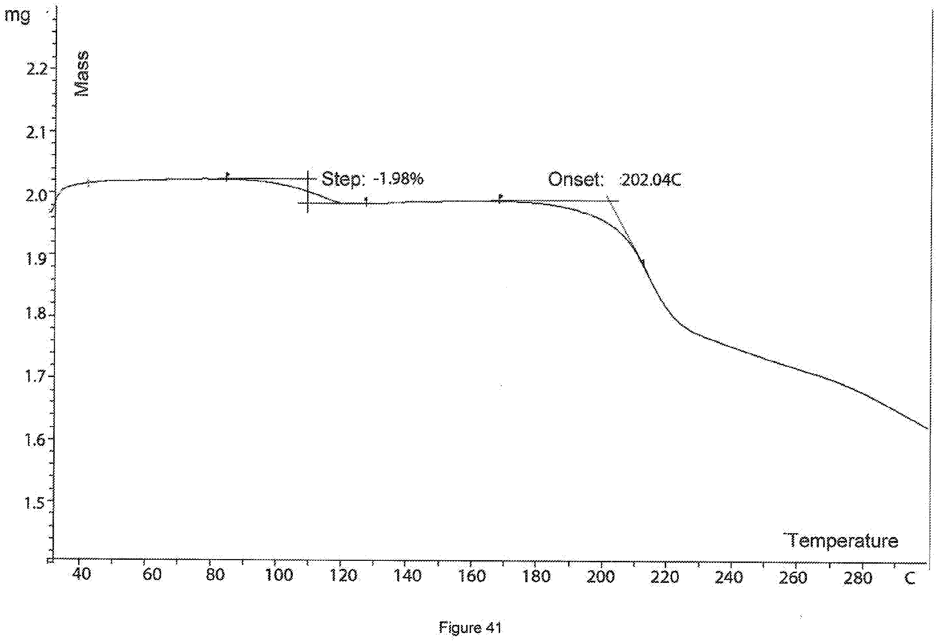

HAL-G-196-25 sample of malate and 3-(1,2,4-triazole[4,3-a]pyridine-3-ylethinyl)-4-methyl-N-(4-((4-methylpiperazine-1-yl)methyl)-3-trifluoromethylphenyl)benzamide obtained via crystallization in the ethanol media based on X-ray powder diffraction (see Fig. 38) is an individual crystalline phase. The same crystalline phase was revealed in samples of malate and free base obtained via crystallization in THF media (HAL-G-196-26 sample). The compound structure was confirmed with 1H NMR spectroscopy (see Fig. 39). Anion and cation stoichiometric proportion in HAL-G-196-19, HAL-G-196-25 and HAL-G-196-26 samples determined using ion chromatography confirms formation of monomalate for each sample. 1H NMR spectrum of HAL-G-196-25 sample contains signals of the residual solvent. DSC analysis (see Fig. 40) resulted in identification of two endothermic transitions ― first one (T = 128 °C) corresponds to solvent loss and sample melting, the second (T = 205 °C) — subsequent sample degradation. During TG analysis, sample weight loss by 2% at a temperature range of 80-130 °C was observed, which was probably due to the loss of residual solvents quantities during salt melting (see Fig. 41). Subsequent weight loss is probably associated with degradation of the sample melted. Based on the studies performed it was assumed that HAL-G-196-25 sample was a solvate. Further sample studies were recognized as unfeasible due to the content of solvent in the crystalline structure of this salt.

### Study of physical and chemical properties of fumarate and 3-(1,2,4-triazole[4,3-a]pyridine-3-ylethinyl)-4-methyl-N-(4-((4-methylpiperazine-1-yl)methyl-3-trifluoromethylphenyl)benzamide(monofumarate)

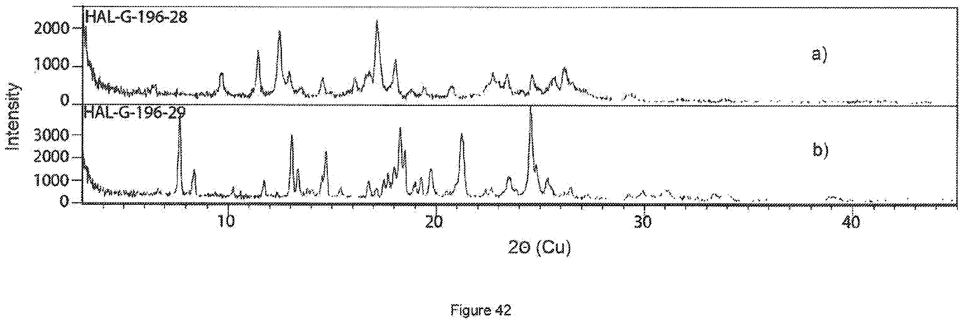

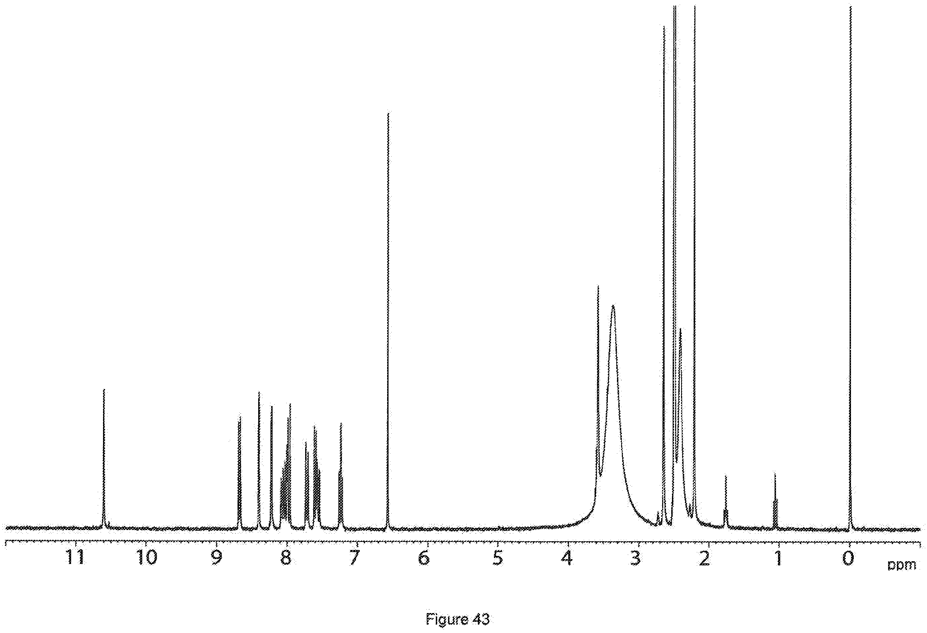

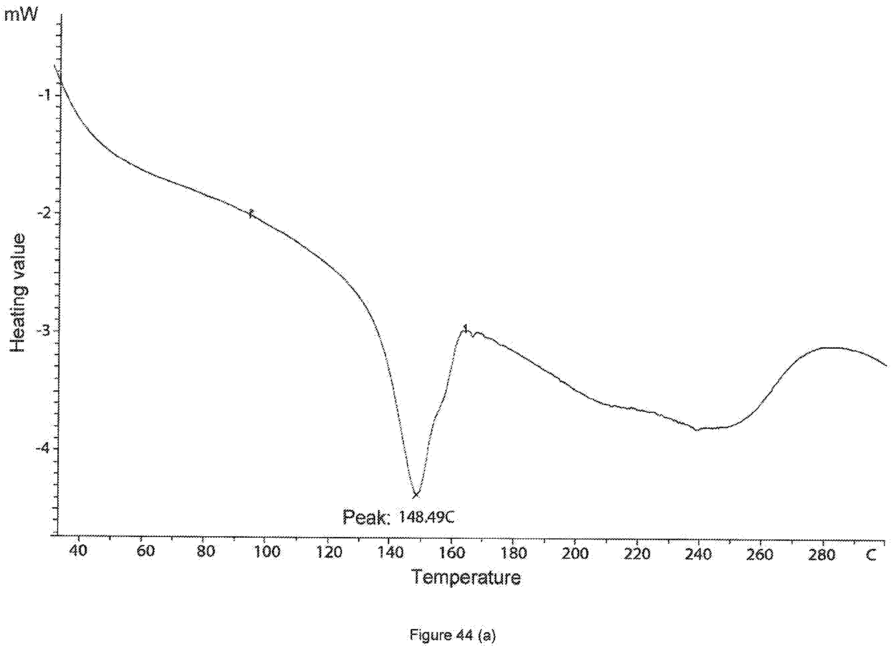

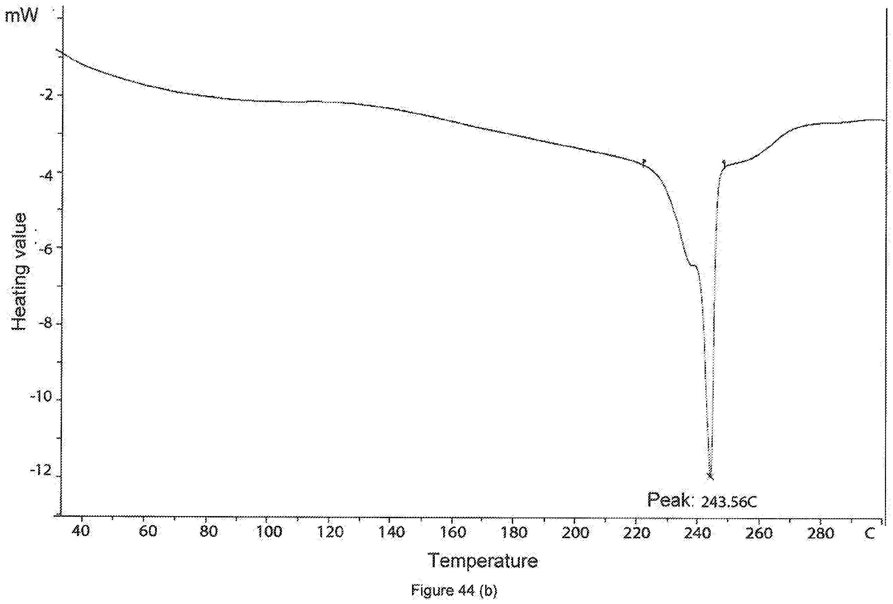

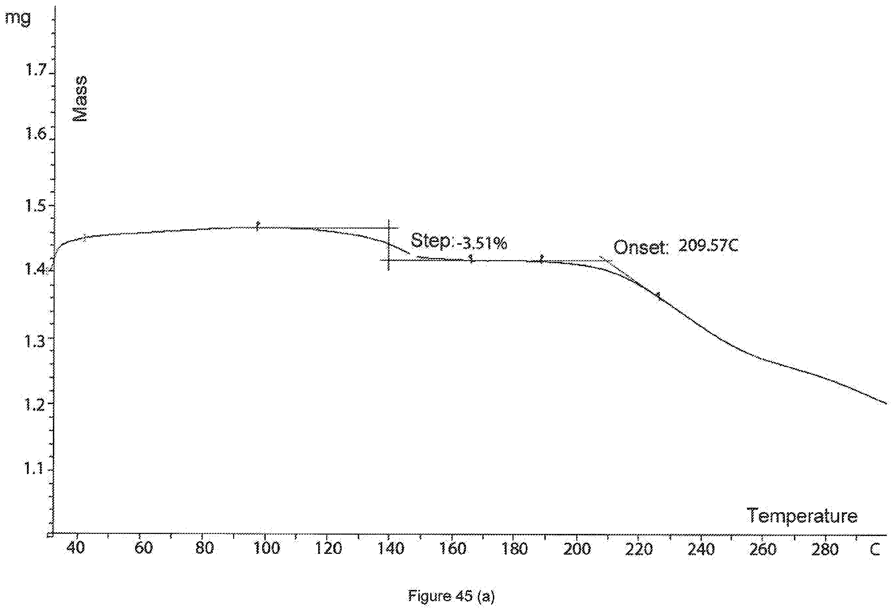

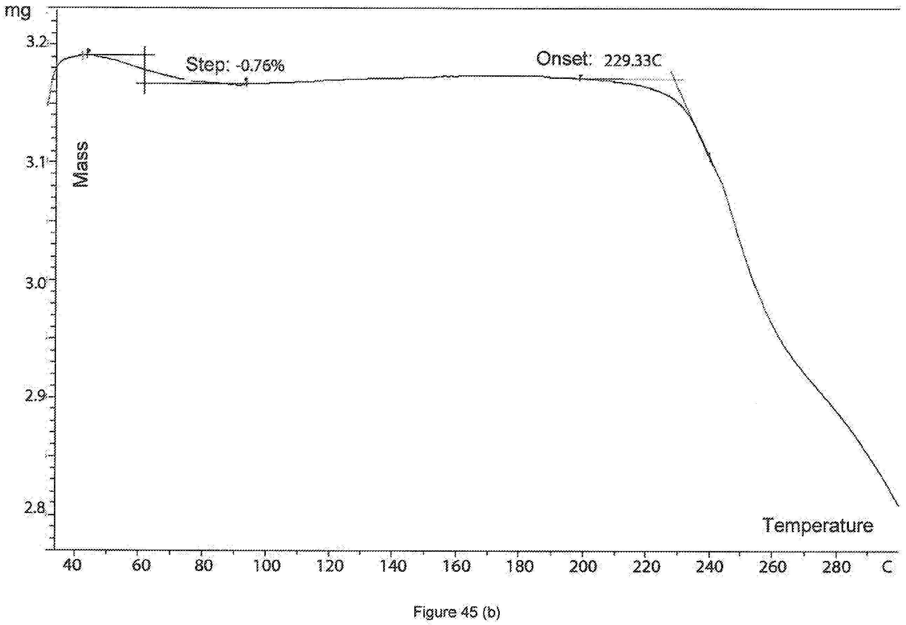

HAL-G-196-28 sample of fumarate and 3-(1,2,4-triazole[4,3-a]pyridine-3-ylethinyl)-4-methyl-N-(4-((4-methylpiperazine-1-yl)methyl)-3-trifluoromethylphenyl)benzamide obtained in the ethanol media based on X-ray powder diffraction (see Fig. 42) is an individual crystalline phase. The compound structure was confirmed with 1H NMR spectroscopy (see Fig. 43). Anion and cation stoichiometric proportion in HAL-G-196-28 sample determined using ion chromatography confirms formation of monofumarate. 1H NMR spectrum of HAL-G-196-28 sample contains residual solvent signals. DSC analysis (see Fig. 44) resulted in identification of one endothermic transition (T = 148 °C) corresponding to solvent loss and salt melting, which is probably accompanied with partial sample degradation. During TG analysis, sample weight loss by 3.5 % at a temperature range of 95-170 °C was observed, which was probably caused by the loss of residual solvent quantities during salt melting (see Fig. 45). Based on the studies performed it was assumed that HAL-G-196-28 sample was a solvate. Further sample studies were recognized as unfeasible due to the content of solvent in the crystalline structure of this salt.

### Study of physical and chemical properties of fumarate and 3-(1,2,4-triazole[4,3-a]pyridine-3-ylethinyl)-4-methyl-N-(4-((4-methylpiperazine-1-yl)methyl-3-trifluoromethylphenyl)benzamide (hemifumarate)

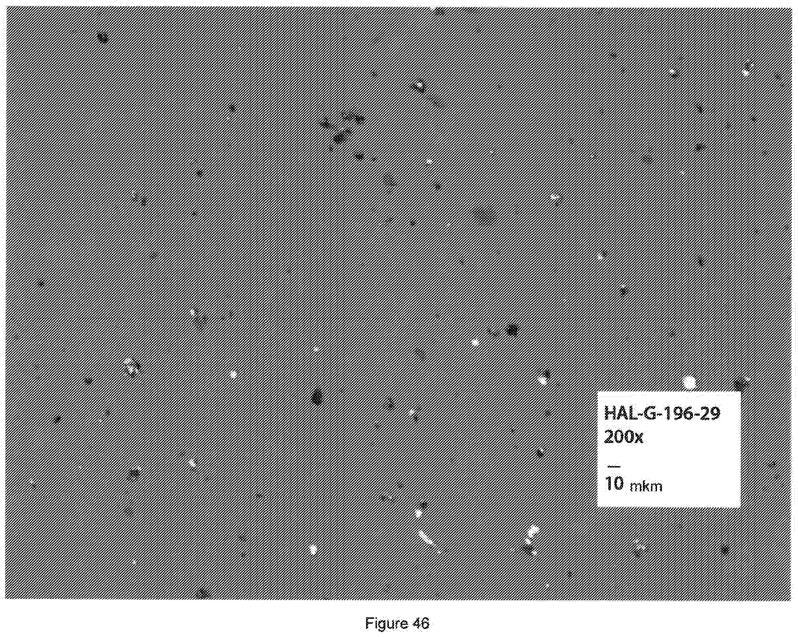

HAL-G-196-29 sample of fumarate and 3-(1,2,4-triazole[4,3-a]pyridine-3-ylethinyl)-4-methyl-N-(4-((4-methylpiperazine-1-yl)methyl)-3-trifluoromethylphenyl)benzamide obtained via crystallization in THF media based on X-ray powder diffraction (see Fig. 42) is an individual crystalline phase, which was also confirmed with polarization microscopy (see Figure 46). The same crystalline phase was revealed in samples of fumarate and free base obtained via crystallization in acetone media (HAL-G-196-30 sample). The compound structure was confirmed with 1H NMR spectroscopy (see Fig. 43). Anion and cation stoichiometric proportion in HAL-G-196-29 and HAL-G-196-30 samples determined using ion chromatography confirms formation of hemifumarate. DSC analysis of HAL-G-196-29 sample (see Fig. 44) resulted in identification of one endothermic transition (T = 244 °C) corresponding to solvent loss and sample melting. During TG analysis, sample weight loss by 1 % was observed, which was probably caused by the loss of residual solvent quantities (see Fig. 45). Study of sample hygroscopicity of fumarate and 3-(1,2,4-triazole[4,3-a]pyridine-3-ylethinyl)-4-methyl-N-(4-((4-methylpiperazine-1-yl)methyl)-3-trifluoromethylphenyl)benzamide showed that at a relative air humidity of 90% the sample absorbed less than four mass per cent of water. The apparent solubility of fumarate and 3-(1,2,4-triazole[4,3-a]pyridine-3-ylethinyl)-4-methyl-N-(4-((4-methylpiperazine-1-yl)methyl)-3-trifluoromethylphenyl)benzamide in deionized water was less than 1 mg/ml (see Table 2). Equilibrium solubility of fumarate and 3-(1,2,4-triazole[4,3-a]pyridine-3-ylethinyl)-4-methyl-N-(4-((4-methylpiperazine-1-yl)methyl)-3-trifluoromethylphenyl)benzamide in deionized water was 7.4 × 10⁻³ mg/ml according to HPLC analysis data (see Table 3). The content of impurities remains constant when the sample is kept during seven days at a temperature of 60 °C (see Table 4). Study of stability of fumarate and 3-(1,2,4-triazole[4,3-a]pyridine-3-ylethinyl)-4-methyl-N-(4-((4-methylpiperazine-1-yl)methyl)-3-trifluoromethylphenyl)benzamide treated by a solvent (acetone) during 6 days showed that crystalline structure of the sample remained unchanged (see Table 5). Therefore, fumarate and 3-(1,2,4-triazole[4,3-a]pyridine-3-ylethinyl)-4-methyl-N-(4-((4-methylpiperazine-1-yl)methyl)-3-trifluoromethylphenyl)benzamide by its physical and chemical properties met requirements set in the course of salt form optimization.

### Results of optimization of the salt form of 3-(1,2,4-triazole[4,3-a]pyridine-3-ylethinyl)-4-methyl-N-(4-((4-methylpiperazine-1-yl)methyl)-3-trifluoromethylphenyl)benzamide

To sum up, in the course of conducted studies on optimization of the salt form of 3-(1,2,4-triazole[4,3-a]pyridine-3-ylethinyl)-4-methyl-N-(4-((4-methylpiperazine-1-yl)methyl)-3-trifluoromethylphenyl)benzamide, more than 50 samples of various salt forms, containing 12 counter-ions and obtained with 4 different solvents, were studied. Conducted studies showed that salts of four acids (muriatic acid, methanesulfonic acid, 4-methylbenzene sulfonic acid and fumaric acid) have favorable physical and chemical properties, i.e. crystallinity, high solubility in water compared to a free base, as well as high purity upon preparation and temperature stability. Besides, such salts may be obtained in low-toxic organic solvents using the easily scalable method and contain a pharmacologically acceptable anion.

However, such salts significantly differ in terms of solubility in water: e.g., fumarate solubility (7.4 × 10⁻³ mg/ml) is comparable to free base solubility (2.3 × 10⁻⁴ mg/ml); solubility of 4-methylbenzene sulfonic acid (2.4 mg/ml), muriatic acid (37.1 mg/ml) and methanesulfonic acid (more than 100 mg/ml) is 10,000-fold more than solubility of 3-(1,2,4-triazole[4,3-a]pyridine-3-ylethinyl)-4-methyl-N-(4-((4-methylpiperazine-1-yl)methyl)-3-trifluoromethylphenyl)benzamide in the form of a free base. Here, only methanesulfonic acid salt may be obtained via crystallization from acetone or ethanol without addition of methyl tert-butyl ether or additional cooling of the solution below the room temperature.

Thus, the conducted study allows concluding that the preferable crystalline salt form of 3-(1,2,4-triazole[4,3-a]pyridine-3-ylethinyl)-4-methyl-N-(4-((4-methylpiperazine-1-yl)methyl)-3-trifluoromethylphenyl)benzamide, which may be obtained in low-toxic organic solvents using the easily scalable method and containing a pharmacologically acceptable anion having crystallinity and high solubility in water, is methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide.

### Obtaining and description of polymorphous modifications of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide

### Examples:

For the purpose of further study of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide, the method of its preparation has been developed. During method development it was revealed that methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide could exist in two polymorphous modifications. Differences in formation these phases we revealed can be summarized by saying that in some cases of 3-(1,2,4-triazole[4,3-a]pyridine-3-ylethinyl)-4-methyl-N-(4-((4-methylpiperazine-1-yl)methyl)-3-trifluoromethylphenyl)benzamide mesylate preparation in acetone, precipitate does not spontaneously form. This results in salt crystallization out of a more concentrated solution. However, it should be noted that in this case a combination of phases or any individual phase may also form. X-ray powder diffraction method was applied to both polymorphous modifications in order to study structure crystallinity. Diffraction patterns of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide samples were obtained at 25 °C (±5 °C) and relative air humidity of ≈70 % using X-ray powder diffractometer Bruker D8 Advance in Bragg-Brentano geometry (anode voltage of 40 kV, currency of 40 mA) equipped with nickel filter (radiation CuKα1, wave length = 1.5406 Å) and position-sensitive detector LynxEye, survey step of 0.02° 2θ, angle range of 4-65° 2θ. Diffraction patterns obtained were studied in detail using Bruker TOPAS5 software package.

### Synthesis of 3-(1,2,4-triazole[4,3-a]pyridine-3-ylethinyl)-4-methyl-N-(4-((4-methylpiperazine-1-yl)methyl)-3-trifluoromethylphenyl) benzamide

Add to the stirred solution of acetylene derivative (43.6 g, 0.105 mol) and 3-bromine-[1,2,4]triazole[4,3-a]pyridine (19.8 g, 0.1 mol) in 350 ml of anhydrous degased DMFA 14.6 ml (0.105 mol) of anhydrous degased triethylamine, tetrakis(triphenylphosphine)palladium (0) (11.5 g, 0.01 mol, 10% (mol)) and copper iodide (I) (1.9 g, 0.01 mol, 10 mol). Degas the reaction mixture again and stir in argon atmosphere at 80 °C during 12 hours. Remove part of the solvent in vacuum (at a temperature of 50-60 °C) to the reaction mixture volume of approximately 200 ml. Add 10% solution of sodium hydroxide (400 ml) to the reaction mixture and dilute with water to 3-4 liters. Filter the precipitate, wash with water (5 × 200 ml), dissolve in 2 I of 20% ethanol in dichloromethane, add sodium sulfate, silica gel and activated carbon. Stir the mixture obtained during 30 minutes and filter through the celite layer. Remove dichloromethane in vacuum, add water to the remaining solution, filter the precipitate, wash with ethyl acetate, dry, dissolve in ethanol and treat with modified silica gel during 12 hours at 60 °C (three times). Remove ethanol in vacuum and dry. Output: -50%.

### Synthesis methanesulfonic acid salt u 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification I)

Heat 3-(1,2,4-triazole[4,3-a]pyridine-3-ylethinyl)-4-methyl-N-(4-((4-methylpiperazine-1-yl)methyl)-3-trifluoromethylphenyl)benzamide suspension (53.2 g, 0.10 mol) in acetone (1,050 ml, in the amount of 20 ml per gram) to boiling and boil during 10 minutes vigorously stirring. Then continuing heating and stirring add in one portion freshly prepared (immediately before adding) solution of methanesulfonic acid (10.1 g, 0.105 mol) in 200 ml of ethanol (quantity of ethanol is calculated in a way that concentration of the solution obtained was 0.5 mol/l). Boil the reaction mixture during 15 minutes, then cool to 20 °C at the rate of approximately 10 °C/hour, then leave for 12 hours at a temperature of +10 °C for crystallization and formation of the precipitate. Filter the precipitate, wash through the filter with acetone (3 × 150 ml) and dry in a cabinet at a temperature of 60 °C until constant mass. Output: 85-90%.
NMR ¹H (500 MHz, DMSO-*d*₆) spectrum: 2.36-2.45 (m, 1H, H_{piperazine}), 2.41 (c, 3H, Me), 2.67 (c, 3H, Me), 2.86 (c, 3H, Me), 2.94 (d, J = 11.2 Hz, 1H, H_{piperazine}), 3.08 (t, J = 10.7 Hz, 1H, H_{piperazine}), 2.94 (d, J = 10.7 Hz, 1H, H_{piperazine}), 4.06 (c, 2H, CH_{2(benzyl)}), 7.24 (t, J = 6.8 Hz, 1H, H_{(arom.)}), 7.53-7.63 (m, 2H, H_{(arom.)}), 7.73 (d, J = 8.6 Hz, 1H, H_{(arom.)}), 7.96 (d, J = 9.2 Hz, 1H, H_{(arom.)}), 8.03 (dd, J₁ = 8.6 Hz, J₂ = 1.6 Hz, 1H, H_{(arom.)}), 8.12 (d, J = 8.6 Hz, 1H, H_{(arom.)}), 8.25 (c, 1H, H_{(arom.)}), 8.40 (c, 1H, H_{(arom.)}), 8.65 (d, J = 6.8 1H, H_{(arom.)}), 10.60 (c, 1H, NH_{amide}).
Mass-spectrum, m/z: 533.2263

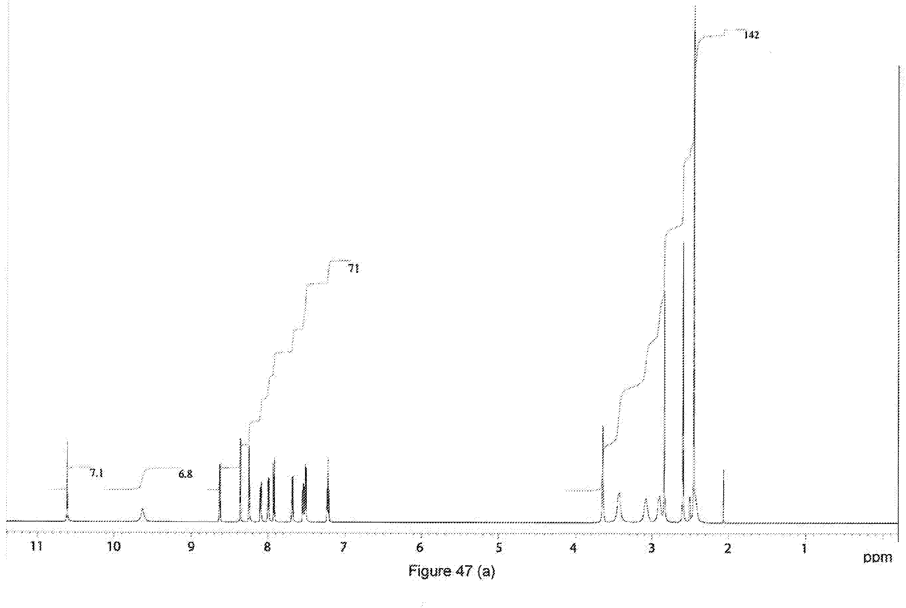

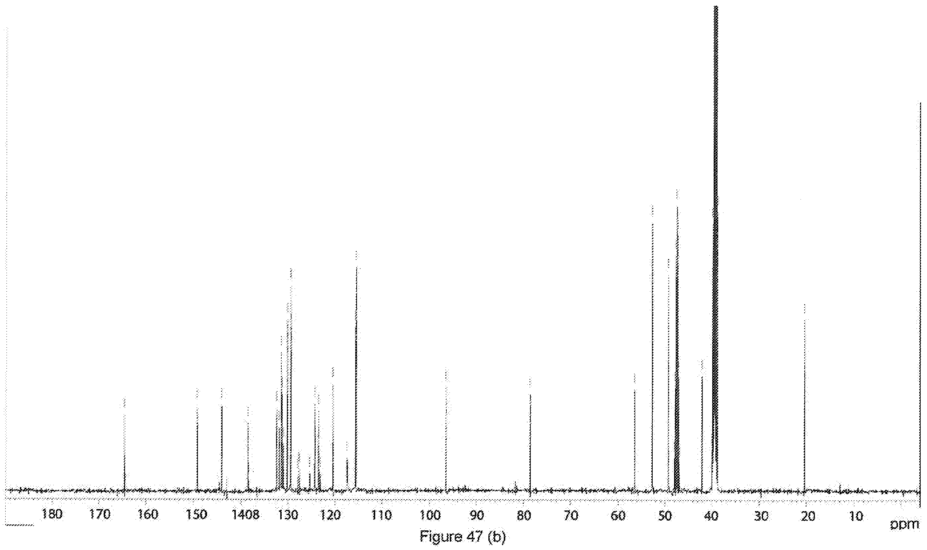

NMR ¹H and ¹³C spectra of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification I) base are given in Figure 47.

### Synthesis of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification II)

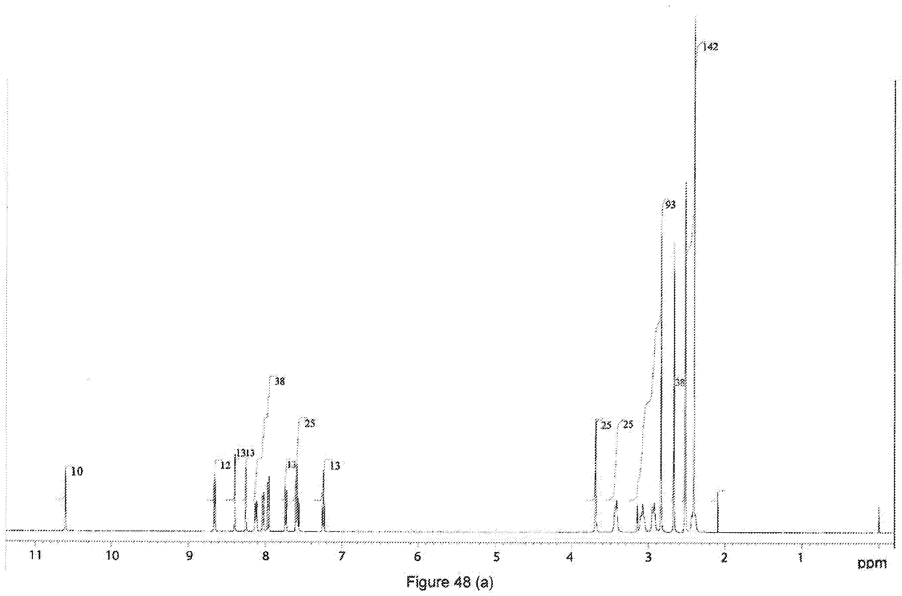

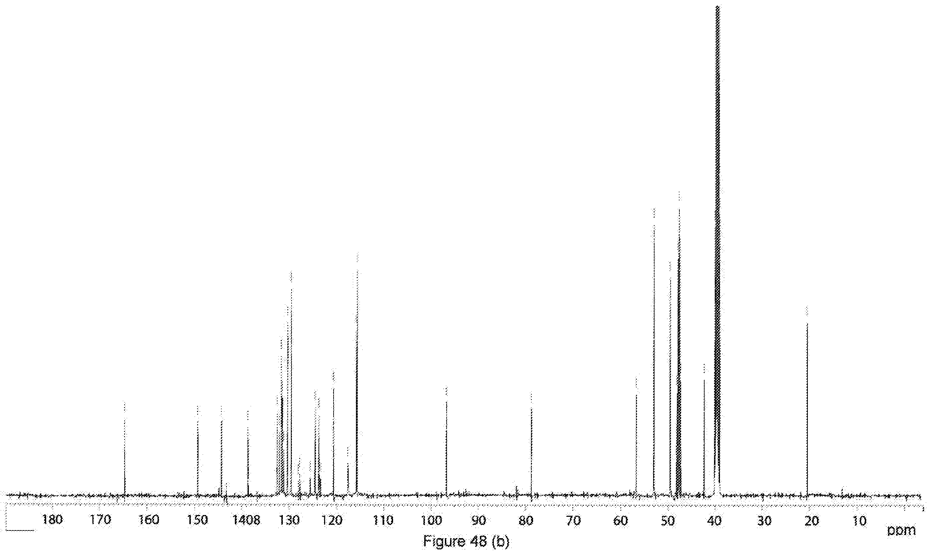

Heat 3-(1,2,4-triazole[4,3-a]pyridine-3-ylethinyl)-4-methyl-N-(4-((4-methylpiperazine-1-yl)methyl)-3-trifluoromethylphenyl)benzamide suspension (17.73 g, 0.033 mol) in 350 ml of acetone to boiling and boil during 10 minutes vigorously stirring. Then continuing heating and stirring add in one portion freshly prepared (immediately before adding) solution of methanesulfonic acid (3.36 g, 0.035 mol) in 70 ml of ethanol. Boil the reaction mixture during 15 minutes, then cool to 20 °C; no precipitate forms. Boil down the solution at low pressure to the half of the initial volume and leave for 24 hours at a temperature of 20-25 °C. Filter the precipitate, wash through the filter with acetone (3x150 ml) and dry in a cabinet at a temperature of 45 °C until constant mass. Output: 85-90%.
NMR ¹H (500 MHz, DMSO-*d*₆) spectrum: 2.35-2.43 (m, 1H, H_{piperazine}), 2.41 (c, 3H, Me), 2.66 (c, 3H, Me), 2.87 (c, 3H, Me), 2.95 (d, J = 11.3 Hz, 1H, H_{piperazine}), 3.10 (t, J = 10.5 Hz, 1H, H_{piperazine}), 2.94 (d, J = 10.5 Hz, 1H, H_{piperazine}), 4.05 (c, 2H, CH_{2(benzyl)}), 7.26 (t, J = 6.9 Hz, 1H, H_{(arom.)}), 7.52-7.61 (m, 2H, H_{(arom.)}), 7.73 (d, J = 8.6 Hz, 1H, H_{(arom.)}), 7.96 ( 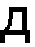 , J = 9.1 Hz, 1H, H_{(arom.)}), 8.03 (dd, J₁ = 8.6 Hz, J₂= 1.6 Hz, 1H, H_{(arom.)}), 8.12 (d, J = 8.6 Hz, 1H, H_{(arom.)}), 8.27 (c, 1H, H_{(arom.)}), 8.41 (c, 1H, H_{(arom.)}), 8.65 (d, J = 6.9 1H, H_{(arom.)}), 10.62 (c, 1H, NH_{amide}).
Mass-spectrum, m/z: 533.2268.
NMR ¹H and ¹³C spectra of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification I) base are given in Figure 48.

### Study of crystallinity of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification I)

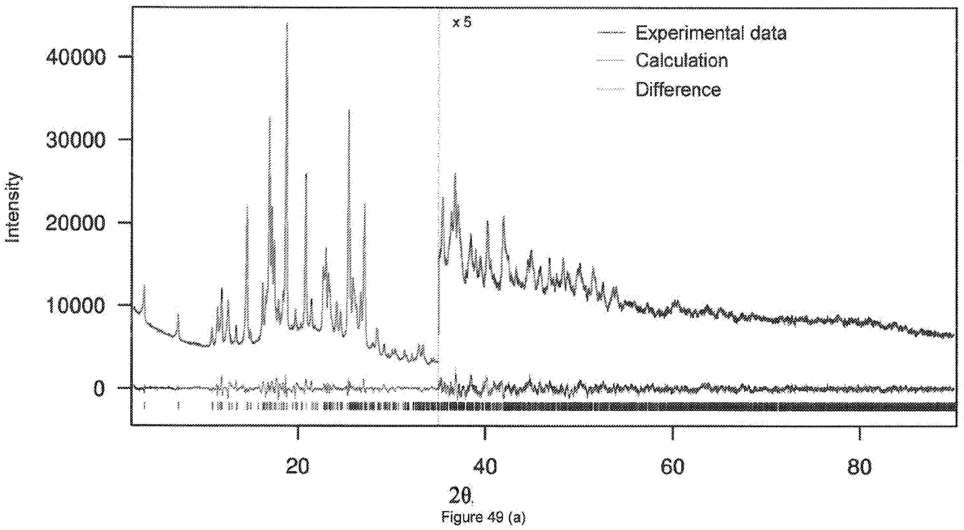

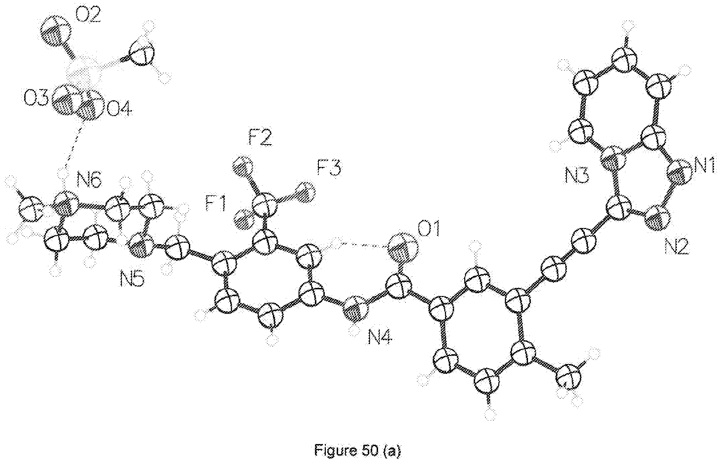

Methanesulfonic acid salt sample and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification I) are an individual crystalline phase with the following elementary cell parameters: a = 51.46 ± 0.05 Å; b = 7.81 ± 0.05 Å and c = 7.63 ± 0.05 Å, β = 108.9 ± 0.1°, V = 2898.9 ± 0.5 Å³. *P*2₁/*n* space group. Volume of the independent part corresponded to one formula unit (see Figure 49a). Positions and intensities of characteristic, visually distinguishable peaks on sample Debye powder diagram are given in Table 6. The general appearance of the independent part of an elementary cell of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide in polymorphous modification I, are given in Figure 50a.

**Table 6 ― Positions and intensities of characteristic, visually distinguishable peaks on Debye powder diagram of a sample of methanesulfonic acid salt hydrate and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification I). Intensities are peak heights (with a background adjustment). Positions correspond to maximums on the diffraction pattern, not to reflections positions.**

| Peak position (2Θ) | Relative intensity | Peak position (2Θ) | Relative intensity | Peak position (2Θ) | Relative intensity |
|---|---|---|---|---|---|
| 1 | 2 | 3 | 4 | 5 | 6 |
| 3.6 | 10.9 | 25.4 | 68.6 | 40.2 | 3.7 |
| 7.2 | 8.0 | 25.9 | 17.9 | 40.5 | 1.2 |
| 10.8 | 4.6 | 26.0 | 14.0 | 40.9 | 0.7 |
| 11.4 | 9.0 | 26.2 | 11.3 | 41.9 | 3.8 |
| 11.8 | 19.3 | 26.4 | 4.2 | 42.2 | 2.2 |
| 12.5 | 8.9 | 26.7 | 14.3 | 42.4 | 2.3 |
| 12.9 | 1.3 | 27.1 | 41.9 | 42.7 | 1.5 |
| 13.4 | 7.3 | 28.0 | 2.1 | 44.0 | 0.6 |
| 14.5 | 42.8 | 28.0 | 2.1 | 44.5 | 1.8 |
| 14.9 | 4.4 | 28.4 | 7.2 | 45.8 | 1.7 |
| 16.2 | 19.2 | 29.2 | 3.2 | 46.9 | 2.4 |
| 16.5 | 9.9 | 30.0 | 2.6 | 47.2 | 1.4 |
| 16.9 | 66.7 | 30.4 | 1.7 | 47.6 | 1.4 |
| 17.2 | 42.2 | 31.4 | 3.3 | 47.9 | 1.3 |
| 17.4 | 30.3 | 31.6 | 1.6 | 48.3 | 2.6 |
| 17.8 | 7.3 | 32.1 | 2.8 | 48.8 | 1.8 |
| 18.1 | 6.3 | 32.5 | 1.5 | 49.4 | 1.1 |
| 18.4 | 13.0 | 33.0 | 5.4 | 50.3 | 1.7 |
| 18.7 | 100.0 | 33.3 | 5.4 | 51.1 | 0.9 |
| 19.3 | 1.4 | 33.6 | 1.8 | 51.4 | 2.0 |
| 19.7 | 4.2 | 34.2 | 1.9 | 52.2 | 0.9 |
| 20.3 | 2.8 | 34.5 | 2.4 | 52.5 | 1.3 |
| 20.8 | 49.7 | 34.9 | 1.3 | 53.5 | 1.2 |
| 21.4 | 9.4 | 35.4 | 4.7 | 53.9 | 1.5 |
| 21.8 | 1.6 | 35.8 | 0.9 | 54.4 | 0.6 |
| 22.7 | 17.3 | 36.1 | 1.8 | 54.8 | 0.9 |
| 22.8 | 14.4 | 36.3 | 3.5 | 55.0 | 0.8 |
| 23.0 | 25.4 | 36.7 | 5.7 | 55.4 | 1.0 |
| 23.2 | 17.0 | 37.1 | 3.5 | 55.9 | 1.2 |
| 23.4 | 12.3 | 37.9 | 0.6 | 56.2 | 0.8 |
| 23.6 | 4.2 | 38.4 | 2.5 | 56.4 | 0.9 |
| 23.8 | 2.5 | 38.7 | 1.1 | 57.2 | 1.3 |
| 24.1 | 11.3 | 38.9 | 1.8 | 59.4 | 0.9 |
| 24.5 | 7.9 | 39.4 | 1.6 | | |

### Study of crystallinity of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification II)

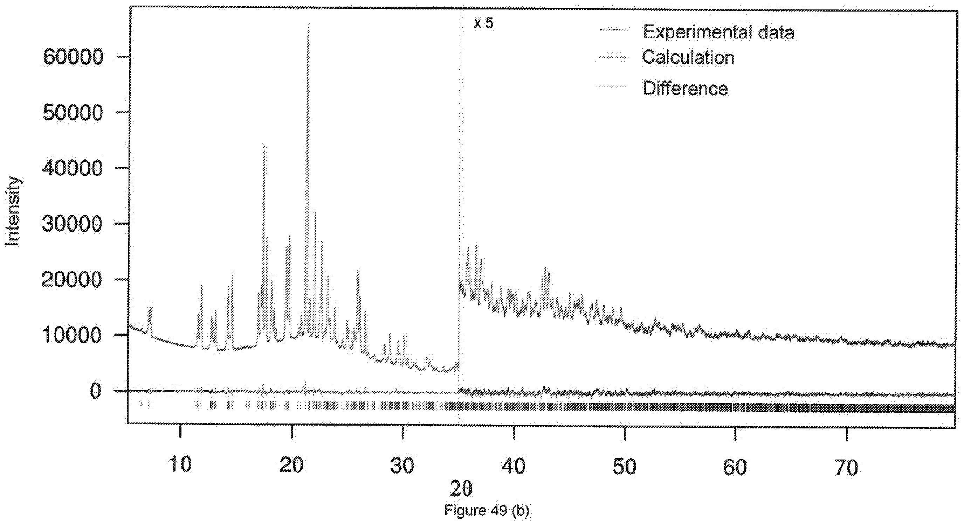

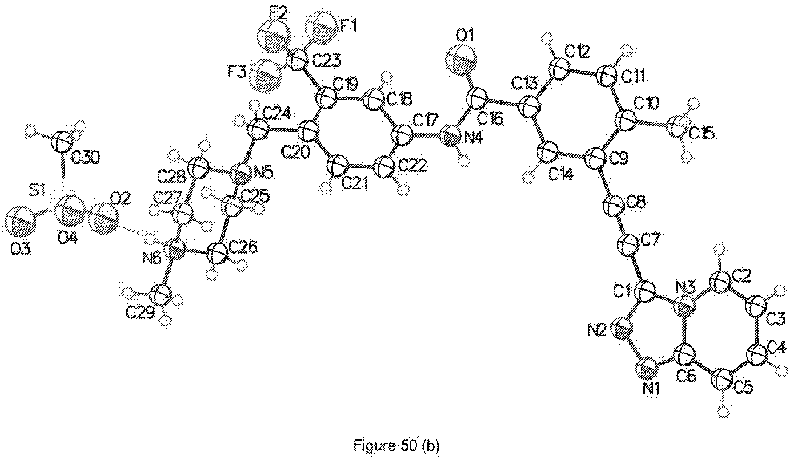

Studies of crystallinity of a sample of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification II) using X-ray powder diffraction method showed that the sample was an individual crystalline phase with the following elementary cell parameters: a = 13.77 ± 0.05 Å; b = 8.09 ± 0.05 Å and c = 30.83 ± 0.05 Å, β = 117.8 ± 0.1, V = 3036.36 ± 0.5 Å³ and P2₁/c space group (see Figure 49b). Positions and intensities of characteristic, visually distinguishable peaks on salt sample Debye powder diagram are given in Table 7. The general appearance of the independent part of an elementary cell of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide in polymorphous modification II are given in Figure 50b.

**Table 7 ― Positions and intensities of characteristic, visually distinguishable peaks on Debye powder diagram of a sample of methanesulfonic acid salt hydrate and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification II). Intensities are peak heights (with a background adjustment). Positions correspond to maximums on the diffraction pattern, not to reflections positions.**

| Peak position (2Θ) | Relative intensity | Peak position (2Θ) | Relative intensity | Peak position (2Θ) | Relative intensity |
|---|---|---|---|---|---|
| 1 | 2 | 3 | 4 | 5 | 6 |
| 6.5 | 1.8 | 29.6 | 8.3 | 43.1 | 2.9 |
| 7.1 | 8.4 | 29.7 | 4.1 | 43.5 | 1.3 |
| 7.3 | 8.3 | 30.1 | 10.3 | 43.8 | 1.2 |
| 11.4 | 4.6 | 30.4 | 2.9 | 44.0 | 1.0 |
| 11.6 | 11.1 | 30.9 | 1.1 | 44.2 | 0.9 |
| 11.8 | 19.1 | 31.1 | 2.2 | 44.6 | 0.9 |
| 12.7 | 10.7 | 31.3 | 1.2 | 45.0 | 1.9 |
| 12.9 | 9.3 | 31.8 | 0.7 | 45.4 | 1.4 |
| 13.1 | 12.7 | 31.9 | 0.6 | 45.6 | 1.3 |
| 14.2 | 20.2 | 32.1 | 4.2 | 45.7 | 1.6 |
| 14.3 | 4.4 | 32.4 | 3.3 | 45.8 | 1.7 |
| 14.6 | 22.2 | 32.5 | 1.8 | 46.1 | 2.0 |
| 15.9 | 0.6 | 32.8 | 1.2 | 46.4 | 1.0 |
| 16.9 | 17.0 | 33.0 | 0.9 | 46.8 | 1.3 |
| 17.2 | 19.7 | 33.6 | 1.9 | 47.0 | 1.5 |
| 17.4 | 63.5 | 33.8 | 0.8 | 47.4 | 1.9 |
| 17.6 | 32.6 | 34.2 | 1.4 | 47.6 | 1.4 |
| 18.1 | 19.7 | 34.4 | 1.5 | 47.8 | 0.7 |
| 18.3 | 10.4 | 34.5 | 2.3 | 48.1 | 1.7 |
| 18.5 | 4.5 | 34.8 | 3.4 | 48.3 | 1.1 |
| 19.4 | 29.9 | 35.0 | 3.3 | 48.6 | 1.0 |
| 19.7 | 32.9 | 35.2 | 1.2 | 48.9 | 1.5 |
| 20.6 | 3.6 | 35.4 | 0.8 | 49.2 | 0.9 |
| 20.8 | 7.8 | 35.7 | 2.6 | 49.4 | 0.7 |
| 21.2 | 100.0 | 35.8 | 3.5 | 49.6 | 1.5 |
| 21.6 | 12.0 | 36.2 | 0.8 | 49.9 | 0.4 |
| 22.0 | 40.8 | 36.5 | 4.0 | 50.1 | 0.5 |
| 22.3 | 1.3 | 37.0 | 3.1 | 50.3 | 0.6 |
| 22.5 | 10.6 | 37.1 | 1.6 | 50.6 | 0.6 |
| 22.6 | 31.6 | 37.2 | 1.3 | 51.0 | 0.4 |
| 22.9 | 4.2 | 37.4 | 1.2 | 51.5 | 0.8 |
| 23.2 | 21.5 | 37.6 | 1.3 | 51.8 | 0.5 |
| 23.4 | 8.3 | 37.9 | 1.8 | 52.1 | 0.6 |
| 23.5 | 2.0 | 38.1 | 0.5 | 52.7 | 1.5 |
| 23.6 | 2.0 | 38.4 | 0.9 | 53.0 | 1.2 |
| 23.8 | 12.1 | 38.7 | 1.8 | 53.4 | 1.0 |
| 24.1 | 2.0 | 38.9 | 1.0 | 53.5 | 1.1 |
| 24.6 | 2.0 | 39.4 | 1.8 | 53.6 | 1.2 |
| 24.9 | 9.3 | 39.7 | 1.8 | 53.9 | 1.0 |
| 25.1 | 7.0 | 39.8 | 1.5 | 54.2 | 1.4 |
| 25.4 | 3.4 | 40.1 | 1.8 | 54.5 | 1.2 |
| 25.6 | 8.0 | 40.3 | 0.4 | 54.7 | 1.3 |
| 25.9 | 26.7 | 40.6 | 0.8 | 54.9 | 1.1 |
| 26.1 | 18.8 | 40.7 | 1.2 | 55.2 | 1.3 |
| 26.4 | 1.8 | 41.0 | 0.9 | 55.8 | 0.6 |
| 26.6 | 14.5 | 41.2 | 1.7 | 56.3 | 0.6 |
| 26.9 | 2.1 | 41.3 | 1.7 | 56.5 | 0.8 |
| 27.3 | 0.7 | 41.6 | 0.6 | 56.7 | 0.7 |
| 27.4 | 1.7 | 41.9 | 1.0 | 57.0 | 0.6 |
| 28.3 | 5.8 | 41.9 | 1.0 | 57.5 | 0.4 |
| 28.7 | 2.1 | 42.1 | 0.4 | 57.8 | 0.4 |
| 28.8 | 9.3 | 42.5 | 2.4 | 58.1 | 0.4 |
| 29.0 | 1.1 | 42.8 | 3.3 | | |

### Study of 3-(1,2,4-triazole[4,3-a]pyridine-3-ylethinyl)-4-methyl-N-(4-((4-methylpiperazine-1-yl)methyl)-3-trifluoromethylphenyl)benzamide free base dissolution kinetics and polymorphous modifications of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide

During further study of polymorphous modifications of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide, kinetics of free base and two salt form synthesized polymorphous modifications dissolution was studied. Dissolution kinetics studies were performed using the Basket, dissolution media volume ― 700 ml, temperature ― 37 ± 1 °C. Paddle stirrers' rotation rate ― 100 rpm. Test sample dissolution rate was calculated as an average of six repeats. Distilled water was used as the dissolution media for polymorphous modifications of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide, and the solution of 12.2 ml of methanesulfonic acid in 500 ml of water for free base. Test portions containing 100 mg of 3-(1,2,4-triazole[4,3-a]pyridine-3-ylethinyl)-4-methyl-N-(4-((4-methylpiperazine-1-yl)methyl)-3-trifluoromethylphenyl)benzamide or 118 mg of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (100 mg calculated as free base) were used for dissolution. In 780 minutes (13 hours) after the beginning of the test, pH of prepared solutions was measured. pH of the solution obtained during dissolution of free base was more acid (4.02) compared to that of solutions prepared by dissolution of polymorphous modifications methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (5.04 ― for polymorphous modification I and 4.95 ― for polymorphous modification II).

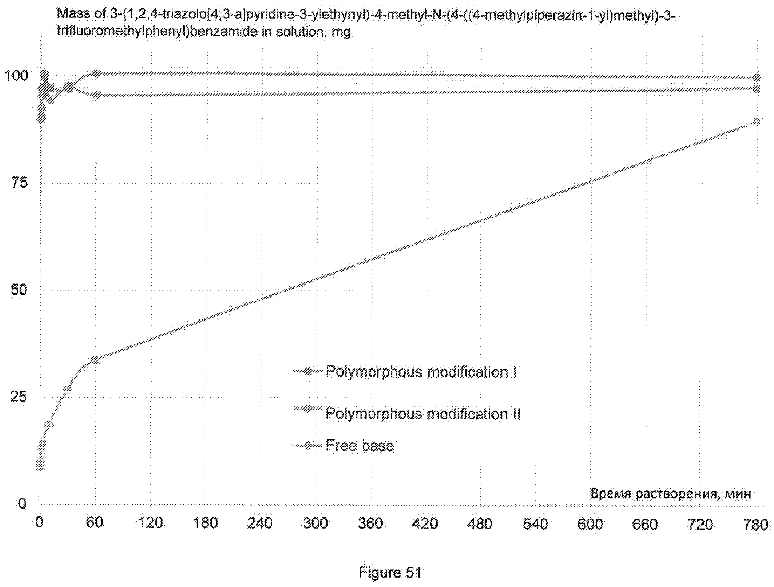

Dissolution kinetics studies results are given in Figure 51. According to the data presented, 95% of 3-(1,2,4-triazole[4,3-a]pyridine-3-ylethinyl)-4-methyl-N-(4-((4-methylpiperazine-1-yl)methyl)-3-trifluoromethylphenyl)benzamide contained in samples of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide transfer to the solution during less than one minute, and full salt dissolution occurs during less than 4 minutes. At the same time, during free base dissolution period of 780 minutes (13) hours, it was impossible to achieve 90% of free base dissolution in the solution containing 12.2 ml of methanesulfonic acid.

Therefore, the conducted studies on synthesis and determination of the crystalline structure of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide showed that this compound may exist at least in two polymorphous modifications, where each may be obtained in low-toxic organic solvents using an easily scalable method and contains a pharmacologically acceptable anion, has crystallinity, high solubility in water. Cation chemical structures in both polymorphous modifications match and correspond to 3-(1,2,4-triazole[4,3-a]pyridine-3-ylethinyl)-4-methyl-N-(4-((4-methylpiperazine-1-yl)methyl)-3-trifluoromethylphenyl)benzamide cation, both compounds are methanesulfonic acid salts and contain protonated form of 3-(1,2,4-triazole[4,3-a]pyridine-3-ylethinyl)-4-methyl-N-(4-((4-methylpiperazine-1-yl)methyl)-3-trifluoromethylphenyl)benzamide and mesylate-anion. 3-(1,2,4-triazole[4,3-a]pyridine-3-ylethinyl)-4-methyl-N-(4-((4-methylpiperazine-1-yl)methyl)-3-trifluoromethylphenyl)benzamide in both polymorphous modifications is protonated by nitrogen atom of the piperazine cycle carrying a methyl group. Both polymorphous modifications do not contain solvent molecules. Main differences in polymorphous modifications structures are mainly associated with the mutual position of the aromatic heterocycle as to the rest of a molecule (see Fig. 50).

### Study of pharmacokinetic characteristics of free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide

To analyze applicability of 3-(1,2,4-triazole[4,3-a]pyridine-3-ylethinyl)-4-methyl-N-(4-((4-methylpiperazine-1-yl)methyl)-3-trifluoromethylphenyl)benzamide salt form as a drug, the study of its pharmacokinetic characteristics has been conducted. As polymorphous modifications of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide have much better dissolution kinetics compared to that of a free base, pharmacokinetic characteristics of a free base and salt are expected to differ significantly. Substance maximum (Cₘₐₓ) and average (AUCt/t) animal plasma concentration for most compounds correlates with solubility of the salt form used for substance administration by lab animals; however, maximum substance animal plasma concentration achievement time (Tₘₐₓ) is usually in inverse relation to the dissolution rate of salt forms used. Therefore, results of the study of physical and chemical properties suggest that maximum (Cₘₐₓ) and average (AUCt/t) 3-(1,2,4-triazole[4,3-a]pyridine-3-ylethinyl)-4-methyl-N-(4-((4-methylpiperazine-1-yl)methyl)-3-trifluoromethylphenyl)benzamide animal plasma concentrations could be expected to be higher, while the maximum concentration achievement time (Tₘₐₓ) ― lower, when animals receive methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide compared to administration of a free base.

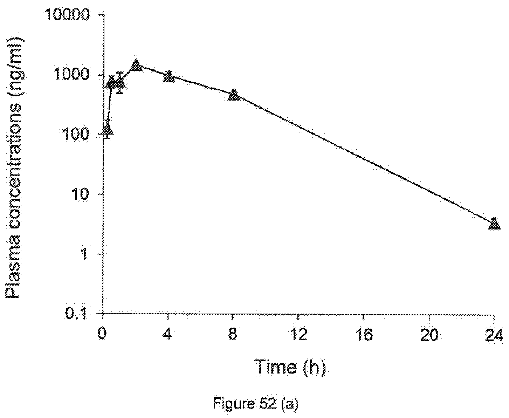

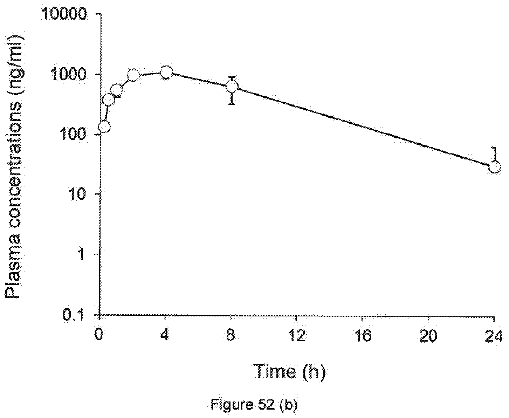

Pharmacokinetics of 3-(1,2,4-triazole[4,3-a]pyridine-3-ylethinyl)-4-methyl-N-(4-((4-methylpiperazine-1-yl)methyl)-3-trifluoromethylphenyl)benzamide was studied after a single administration of a free base at a dose of 50 mg/kg and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification I) at a dose of 59 mg/kg (50 mg/kg calculated as a free base) to C57BL/6 line mice at a dose of 50 mg/kg. Study results are given in Figure 52 and in Tables 8 and 9.

**Table 8 ― Main pharmacokinetic parameters of 23-(1,2,4-triazole[4,3-a]pyridine-3-ylethinyl)-4-methyl-N-(4-((4-methylpiperazine-1-yl)methyl)-3-trifluoromethylphenyl)benzamide when the free base is administered to C57BL/6 line mice at a dose of 50 mg/kg. Average values are determined for each time point based on individual data obtained from three animals.**

| T_{1/2} | Tₘₐₓ | Cmax | AUC₀₋₂₄ | AUC₀₋₂₄/24 | AUC_{0-∞} |
|---|---|---|---|---|---|
| (h) | (h) | (ng/ml) | (ng·h/ml) | (ng/ml) | (ng·h/ml) |
| 2.4 | 2 | 1,490 | 10,856 | 452 | 10,868 |

**Table 9 ― Main pharmacokinetic parameters of 3-(1,2,4-triazole[4,3-a]pyridine-3-ylethinyl)-4-methyl-N-(4-((4-methylpiperazine-1-yl)methyl)-3-trifluoromethylphenyl)benzamide when methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification I)* are administered to C57BL/6 line mice at a dose of 59 mg/kg (50 mg/kg calculated as a free base). Average values are determined for each time point based on individual data obtained from three animals.**

| T_{1/2} | Tₘₐₓ | Cmax | AUC₀₋₂₄ | AUC₀₋₂₄/24 | AUC_{0-∞} |
|---|---|---|---|---|---|
| (h) | (h) | (ng/ml) | (ng·h/ml) | (ng/ml) | (ng·h/ml) |
| 3.8 | 4 | 1,099 | 11,933 | 497 | 12,103 |
| Note: | | | | | |
| No statistically true differences in all pharmacokinetic parameters studied were revealed after administration of the salt form as polymorphous modification II (p < 0.05). | | | | | |

Pharmacokinetic studies lead to unexpected results, where following administration of a free base maximum 3-(1,2,4-triazole[4,3-a]pyridine-3-ylethinyl)-4-methyl-N-(4-((4-methylpiperazine-1-yl)methyl)-3-trifluoromethylphenyl)benzamide animal plasma concentration (Cₘₐₓ = 1,490 ng/ml) exceeded maximum concentration (Cₘₐₓ = 1,099 ng/ml) identified after administration of the salt form by more than one third. Moreover, despite that the salt form had much higher dissolution rate than that of a free base, maximum concentration achievement time (Tₘₐₓ) for the salt form was twice longer than that for the free base. It is importance to note that despite lesser 3-(1,2,4-triazole[4,3-a]pyridine-3-ylethinyl)-4-methyl-N-(4-((4-methylpiperazine-1-yl)methyl)-3-trifluoromethylphenyl)benzamide maximum plasma concentration following oral administration of the salt form, administration of the salt form ensure slightly higher substance concentration average values (AUCt/t).

Therefore, studies of pharmacokinetic parameters of a free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide lead to unexpected results showing significant differences in their pharmacokinetic profiles. Such differences in pharmacokinetic profiles apparently may not be always predicted according to the data of the salt form optimization based on the existing technological capabilities. Here, such differences may potentially lead to changes in the therapeutic efficacy, administration safety and/or other properties of a drug candidate. For further assessment of usability of the developed 3-(1,2,4-triazole[4,3-a]pyridine-3-ylethinyl)-4-methyl-N-(4-((4-methylpiperazine-1-yl)methyl)-3-trifluoromethylphenyl)benzamide salt form acute toxicity and efficacy of a free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide were conducted.

### Studies of safety of a free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide in compounds acute toxicity study experiments.

To study safety of application of a free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide, such substances acute toxicity studies were conducted.

Acute toxicity of a free base of 3-(1,2,4-triazole[4,3-a]pyridine-3-ylethinyl)-4-methyl-N-(4-((4-methylpiperazine-1-yl)methyl)-3-trifluoromethylphenyl)benzamide and its salt with methanesulfonic acid was studied on CD-1 line mice males at the age of 2-3 months. For each drug dose study a group of six animals was used. The study included control groups with the same animal headcount, which received equivalent solvent doses of 0.5% water solution of methylcellulose. The follow-up period was 28 days. Lab mice survivability analysis allowed carrying out Bliss analysis and determining lethal doses of studied drugs. Results of the acute toxicity studies and calculated half-lethal doses for the free base and methanesulfonic acid salt are given in Table 10.

**Table 10 ― Lethal doses of 3-(1,2,4-triazole[4,3-a]pyridine-3-ylethinyl)-4-methyl-N-(4-((4-methylpiperazine-1-yl)methyl)-3-trifluoromethylphenyl)benzamide when administered in the form of a free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (polymorphous modification I) to male mice.**

| | | Dose without observable effects, mg/kg | LD₁₀, mg/kg | LD₅₀, mg/kg | LD₉₀, mg/kg |
|---|---|---|---|---|---|
| 3-(1,2,4-triazole[4,3-a]pyridine-3-ylethinyl)-4-methyl-N-(4-((4-methylpiperazine-1-yl)methyl)-3-trifluoromethylphenyl)benzamide free base | | 160 | 504 | 677 ± 55 | 850 |
| Methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide | | 400 (338*) | 600 (508*) | 1,400 ± 200 (1,186 ± 169*) | 2,200 (1,864*) |
| | * Doses in brackets are calculated as a free base | | | | |

The data given above show that semi-lethal doses (LD₅₀ are doses, which lead to the death of half of animals from the test group) for methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide are around twice higher than lethal doses for the free base. The similar more expressed effect if observed for doses, which lead to the death of 90% of animals from the test group (see Table 10). Administration of 3-(1,2,4-triazole[4,3-a]pyridine-3-ylethinyl)-4-methyl-N-(4-((4-methylpiperazine-1-yl)methyl)-3-trifluoromethylphenyl)benzamide both as free base and salt form caused similar signs of animal intoxication: dyspnea, hypodynamia, tumbled fur, diarrhea, bloating, in some animals ― focal alopecia; however, when the substance was administered in the form of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide, the dose not leading to the development of such effects, was twice higher.

Thus, the study of safety of application of a free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide lead to unexpected results showing that the salt form was characterized by significantly higher application safety, which was confirmed with higher 3-(1,2,4-triazole[4,3-a]pyridine-3-ylethinyl)-4-methyl-N-(4-((4-methylpiperazine-1-yl)methyl)-3-trifluoromethylphenyl)benzamide dose (calculated as a free base), which upon administration by lab animals' organisms caused no observable effects, as well as by almost two-fold increase of the salt form semi-lethal dose compared to a free base. Favorable safety profile of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide make this salt form more perspective drug candidate compared to a free base.

### Study of efficacy of a free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide using BCR/ABL-induced chronic myelogenous leukemia model.

To assess efficacy of application of a free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide to treat chronic myelogenous leukemia (CML) studies of compounds activity on the BCR/ABL-induced CML-like disease in mice were conducted. In the study C57BL/6N line mice were used, which received a sub lethal dose of radiation with intravenous transplantation of donor Sca1+ marrow cells, expressing p185-T315l^{BCR/ABL} due to retroviral transduction. Treatment was started on the 11^{th} day after transplantation of cells expressing p185-T315l^{BCR/ABL}.

Efficacy of the therapy with 3-(1,2,4-triazole[4,3-a]pyridine-3-ylethinyl)-4-methyl-N-(4-((4-methylpiperazine-1-yl)methyl)-3-trifluoromethylphenyl)benzamide administered orally as a free base and methanesulfonic acid salt was studied. Results of the study of the effect of oral administration of 3-(1,2,4-triazole[4,3-a]pyridine-3-ylethinyl)-4-methyl-N-(4-((4-methylpiperazine-1-yl)methyl)-3-trifluoromethylphenyl)benzamide free base (at a dose of 50 mg/kg), methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide (at doses 8.5; 21; 34 and 50 mg/kg calculated as a free base) on average life of lab animals are given in Table 11.

As the data presented show, administration of 3-(1,2,4-triazole[4,3-a]pyridine-3-ylethinyl)-4-methyl-N-(4-((4-methylpiperazine-1-yl)methyl)-3-trifluoromethylphenyl)benzamide both as free base and salt form lead to the increase of average life in animals. Here, upon administration of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide an express dependence of mice average life on the salt form dose is observed. Administration of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide at a dose of 40 mg/kg (34 mg/kg calculated as a free base) has the same therapeutic effect as administration of 50 mg/kg of a free base. Administration of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide at a dose of 59 mg/kg (50 mg/kg calculated as a free base) ensure almost two-fold increase of animals' lives compared to the control group. The salt form efficacy by this indicator is greater than that of a free base.

**Table 11 ― Effect of a free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide on average life of lab animals on BCR/ABL-induced chronic myelogenous leukemia**

| Studied substance | Dose, mg/kg | Average life, days |
|---|---|---|
| Control (solvent) | 0 | 55.0 ± 6.3 |
| 3-(1,2,4-triazole[4,3-a]pyridine-3-ylethinyl)-4-methyl-N-(4-((4-methylpiperazine-1-yl)methyl)-3-trifluoromethylphenyl)benzamide free base | 50 | 93.0 ± 6.1 |
| Methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide | 10 (8.5) | 65.5 ± 6.7 |
| | 25 (21) | 73.9 ± 7.1 |
| | 40 (34) | 90.3 ± 6.9 |
| | 59 (50) | 104.5 ± 5.2 |
| * Values in brackets are calculated as a free base | | |

Therefore, studies of efficacy of application of a free base and methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide lead to unexpected results showing that application of the salt form was more effective, which was manifested in truly longer average lives of animals in the group which received the salt form as compared to the group, which received the equivalent dose of a free base. Therefore, higher efficacy of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide makes this salt form more perspective drug candidate compared to a free base.

### Study of the biologic activity of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide.

Biologic activity of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide, as well as of its crystalline forms, which are the subject of this innovation, has been studied in various experiments.

### Study of the effect of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide on human kinases enzyme activity.

Methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide at the nanomolar range of concentrations inhibit Bcr-Abl tyrosine kinase, activating clinically significant mutant forms of this enzyme. Results of Bcr-Abl kinases inhibition experiments are summed up in Table 12. Half-maximal inhibitory concentration (IC50) Bcr-Abl of wild type for methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide amounted to 0.49-3.1 nM (based on the results of 3 independent experiments). IC50 Bcr-Abl with T315l mutation amounted to 0.78-21 nM (based on the results of 3 independent experiments).

**Table 12 ― Effect of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide on Bcr-Abl tyrosine kinase enzyme activity, including this enzyme clinically significant mutant forms.**

| Kinase | IC50, nM | Kinase | IC50, nM |
|---|---|---|---|
| Bcr-Abl | 0.49-3.1* | Bcr-Abl (H396P) | 1.0 |
| Bcr-Abl (T315I) | 0.78-21* | Bcr-Abl (M351T) | 2.8 |
| Bcr-Abl (E255K) | 9.5 | Bcr-Abl (Q252H) | 12 |
| Bcr-Abl (F317I) | 2.0 | Bcr-Abl (Y253F) | 4.1 |
| Bcr-Abl (G250E) | 7.4 | | |

In concentration of 100 nM methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide significantly (more than 50% inhibition) inhibited activity of the following human tyrosine kinases of 337 tested ones: ABL1, ABL2/ARG, BLK, DDR1, DDR2, EPHA2, EPHA8, EPHB2, FGR, FLT4/VEGFR3, FMS, FRK/PTK5, FYN, HCK, KDR/VEGFR2, LCK, LYN, LYN B, P38a/MAPK14, PDGFRa, PDGFRb, RAF1, RET, RIPK3, ZAK/MLTK, where more than 90% of activity was inhibited in the following kinases: ABL1, ABL2/ARG, DDR1, DDR2, FMS, FRK/PTK5, LCK, LYN, LYN B, PDGFRa, RET.

### Study of cytotoxicity of methanesulfonic acid salt and 3-(1.2.4-triazole[4.3-a]pyridine-3-ylethinyl)-4-methyl-N-(4-((4-methylpiperazine-1-yl)methyl)-3-trifluoromethylphenyl)benzamide with respect to various tumor cell lines

Methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide express cytotoxicity with respect to immature lymphoid cells in Ph+ CML and Ph+ acute lymphoblastic leukemia (ALL) models, including T315l mutation cells. During the experiment, the drug showed toxicity with respect to human tumors of K562 (IC50 8nM), KCL-22 (IC50 9nM), BV-173 (IC50 5nM) lines representing Ph+ CML model, as well as Tom-1 (IC50 5nM), SupB15 (IC50 50nM) lines, representing Ph+ ALL model.

Methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide also showed toxicity with respect to model tumor cell lines obtained by retroviral transduction of murine hematopoietic BaF3 line BCR-ABL gene or its mutant forms [1]: BaF3/BCR-ABL (IC50 5nM), BaF3/BCR-ABL Y253F (IC50 25nM), BaF3/BCR-ABL E255K (IC50 25nM), BaF3/BCR-ABL F317L (IC50 250nM), BaF3/BCR-ABL T315l (IC50 75nM).

Methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide also showed cytotoxicity with respect to human tumor cell lines, including, but not limited to, acute lymphoblastic leukemia line (CCRF-CEM), breast cancer line (MDA-MB-468), ovarian cancer line (SKOV-3), lymphoma lines (SR, EL4).

### Study of efficacy of methanesulfonic acid salt and 3-(1.2.4-triazole[4.3-a]pyridine-3-ylethinyl)-4-methyl-N-(4-((4-methylpiperazine-1-yl)methyl)-3-trifluoromethylphenyl)benzamide using xenograft chronic leukemia model

During the study with the xenograft model with thymus-deprived mice with subcutaneous implantation of K562 line cells, effects of administration of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide at doses of 25 and 40 mg/kg (21 and 34 mg/kg calculated as a free base, correspondingly) per day on the tumor size were assessed. The therapy started after the size of the tumor had reached 500 mm³, therapy duration was 14 days, the follow-up period was 240 days. Administration of the drug at a dose of 25 mg/kg per day led to the decrease of the tumor size to the immeasurable size with subsequent proliferation after the 35^{th} day of follow-up. Administration of the drug at a dose of 40 mg/kg led to elimination of the tumor without relapse during all 240 days of mice follow-up.

### Study of efficacy of methanesulfonic acid salt and 3-(1.2.4-triazole[4.3-a]pyridine-3-ylethinyl)-4-methyl-N-(4-((4-methylpiperazine-1-yl)methyl)-3-trifluoromethylphenyl)benzamide using acute leukemia model

During the study of efficacy of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide using acute leukemia model, to induce the pathology marrow cells obtained from C57BL/6N line mice with induced acute leukemia were used. Cells were injected to the tail vein of an animal, which received a sub lethal dose of radiation. Therapy was initiated on the 5^{th} day after pathology induction and continued for 2 weeks. The study results showed that administration of the drug at a dose of 40 mg/kg (34 mg/kg calculated as a free base) lead to the increase of average lives in animals by more than 25% compared to the control group which received no therapy.

### Study of efficacy of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide using intestinal solid tumor model

During the study of efficacy of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide using intestinal solid tumor model, to induce pathology HCT116 line cells were used. Cells in the amount of 200 µl (2.5 × 10⁷ cells/ml) were subcutaneously injected to the right side of thymus-deprived mice females (SCID). After the tumor had reached the size of 200 mm³ all mice were randomized by tumor size and distributed between the control group and therapeutic group. Administration of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide at a dose of 25 mg/kg (21 mg/kg calculated as a free base) was started on the next day following randomization and continued for 20 days. To determine efficacy of tumor growth inhibition upon completion of treatment (20 days), the average size ratio was calculated for the therapeutic/control groups (% T/C). The results of the study showed that administration of the drug at a dose of 25 mg/kg lead to almost complete tumor non-dissemination (T/C <35%)

### Study of efficacy of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide using non-small cell lung carcinoma

Thymus-deprived mice males were used in the studies. A549 cells in the amount of 1 × 10⁷ were injected with 0.2 ml of matrigel solution (BD Pharmingen) to the left leg of a mouse under the ketamine/xylazine anesthesia. In a week after injections, mice were distributed between the therapeutic and control groups and randomized by tumor size. Administration of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide at a dose of 25 mg/kg (21 mg/kg calculated as a free base) was started on the next day after randomization and continued during 20 days. To determine efficacy of tumor growth inhibition, the average size ratio was calculated for the therapeutic/control groups (% T/C). The results of the study showed that administration of the drug at a dose of 25 mg/kg lead to almost complete tumor non-dissemination (T/C <35%)

### Detailed study of pharmacokinetics of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide

During the study of pharmacokinetics of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide in rats and dogs, it was established that drug bioavailability following oral administration was higher than that following the intravenous administration: bioavailability in dogs F = 45.9% ― 66.1% at a dose range of 2 to 22 mg/kg (2 to 19 mg/kg calculated as a free base), in rats F = 13.8% ― 59.5% at a dose range of 5 to 80 mg/kg (4.2 to 68 mg/kg calculated as a free base).

Methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide following oral administration by mice at a dose range 5 to 50 mg/kg were absorbed for 2-4 hours, reaching corresponding maximum concentrations from 82 ng/ml to 1,099 ng/ml. Here, area under the concentration-time curve (AUC) linearly changed throughout the whole dose range from 5 mg/kg to 50 mg/kg from 372 ng*h/ml to 12,104 ng*h/ml. The drug following oral administration by rats in the form of mesylate at a dose range from 5 to 80 mg/kg absorbed, reaching corresponding maximum concentrations from 72 ng/ml to 1,250 ng/ml for 2.3-5.3 hours. Here, AUC linearly changed throughout the whole dose range from 5 mg/kg to 80 mg/kg from 430 ng*h/ml to 21,124 ng*h/ml. 3-(1,2,4-triazole[4,3-a]pyridine-3-ylethinyl)-4-methyl-N-(4-((4-methylpiperazine-1-yl)methyl)-3-trifluoromethylphenyl)benzamide is relatively slowly absorbed from the GIT after administration of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide to dogs at a dose range from 2 to 45 mg/kg, reaching maximum concentration at a range from 31.8 to 224 ng/ml for 3-8.5 hours. Here, AUC linearly changed throughout the whole dose range from 2 mg/kg to 22 mg/kg from 420 ng*h/ml to 5,480 ng*h/ml, and did not change during subsequent dose escalation to 45 mg/kg (5,173 ng*h/ml).

3-(1,2,4-triazole[4,3-a]pyridine-3-ylethinyl)-4-methyl-N-(4-((4-methylpiperazine-1-yl)methyl)-3-trifluoromethylphenyl)benzamide is quite slowly eliminated from the systemic blood flow; the half-life period in dogs is around 7 hours, rats and mice ― around 3 hours. Clearance following the intravenous administration is quite high: 2.12 l/h/kg for dogs, 1.61 l/h/kg for rats. Large apparent distribution volume (Vd = 14.1 l/kg for dogs, 6.16 l/kg for rats) indicates to intensive drug distribution in tissues.

The study of drug distribution in rat tissues revealed high concentrations of the substance in lungs (approximately 71-fold exceeding the exposure compared to blood plasma), spleen (45-fold exceeding), kidneys (34-fold exceeding), bone marrow (27-fold exceeding), liver (21-fold exceeding). 3-(1,2,4-triazole[4,3-a]pyridine-3-ylethinyl)-4-methyl-N-(4-((4-methylpiperazine-1-yl)methyl)-3-trifluoromethylphenyl)benzamide exposure in brain amounted to approximately 20% from blood plasma exposure.

3-(1,2,4-triazole[4,3-a]pyridine-3-ylethinyl)-4-methyl-N-(4-((4-methylpiperazine-1-yl)methyl)-3-trifluoromethylphenyl)benzamide is metabolized with participation of P450 cytochrome isoform CYP3A4 and without following P450 cytochrome isoforms: CYP1A2, 2B6, 2C8, 2C9, 2C19, 2D6, according to the study using cytochrome enzyme drugs. The study of 3-(1,2,4-triazole[4,3-a]pyridine-3-ylethinyl)-4-methyl-N-(4-((4-methylpiperazine-1-yl)methyl)-3-trifluoromethylphenyl)benzamide metabolism in hepatocytes if rats, dogs and humans revealed formation of similar metabolite profiles, among which two glutathione conjugates, N-desmethyl-derivative, N-oxide, were identified. In blood plasma of rats and dogs 3-(1,2,4-triazole[4,3-a]pyridine-3-ylethinyl)-4-methyl-N-(4-((4-methylpiperazine-1-yl)methyl)-3-trifluoromethylphenyl)benzamide hydrolysis product was identified by amide bond, represented by carboxylic acid. Quantity determination of such metabolites in animal plasma showed that the area under the concentration-time curve never exceeds 10% from main substance exposure.

### Studies of safety of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide

3-(1,2,4-triazole[4,3-a]pyridine-3-ylethinyl)-4-methyl-N-(4-((4-methylpiperazine-1-yl)methyl)-3-trifluoromethylphenyl)benzamide drug (in the form of mesylate) was the subject of expanded pre-clinical safety evaluation trials, including the effect of the drug on the ion hERG channel, drug toxicity after a single and repeated administration, allergenic capacity and immune toxicity studies.

3-(1,2,4-triazole[4,3-a]pyridine-3-ylethinyl)-4-methyl-N-(4-((4-methylpiperazine-1-yl)methyl)-3-trifluoromethylphenyl)benzamide drug (0.1-10 µM) in the form of a mesylate inhibited potassium hERG channel with the IC₅₀ value equal to 7.8 µM.

The results of the acute toxicity studies showed that LD₁₀ of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide for mice was 800 mg/kg (678 mg/kg calculated as a free base), and for rats was 2,000 mg/kg (1,695 mg/kg calculated as a free base). MTD of the drug at a single administration by dogs was 45 mg/kg (38 mg/kg calculated as a free base).

Pursuant to the data of 2 separate studies, MTD of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide for rats at a daily intragastric administration during 28 days amounted to 50-73 mg/kg (42-62 mg/kg calculated as a free base), which corresponded to Cₘₐₓ 661 ± 289 ng/ml and AUC₂₄ 8,596 ± 2,209 ng*h/ml on Day 28 (for dose of 50 mg/kg).

To identify allergenic properties, the following methods were used: evaluation of anaphylactogenic activity using Guinea pigs general systemic anaphylaxis and active cutaneous anaphylaxis models; evaluation of immediate-type hypersensitivity (ITH) reaction and delayed-type hypersensitivity test (DTH) reaction in Guinea pigs following epicutaneous and conjunctival drug application; evaluation of DTH reaction in mice; study of the inflammation reaction to A concanavalin in mice; measuring of eosinophil blood count, evaluation of the effect on phagocyte neutrophils' activity (Nitro Blue-Tetrazolium Test (NBT)), leukocyte specific lysis reaction scenario using Guinea pig model with subcutaneous administration of the drug. The studies showed that methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide demonstrate no allergenic activity in such models.

The studies of the immune toxic effect of methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide showed that single intragastric administration of the drug to mice had no effect on the hemagglutinin and hemolysin level in animals blood compared to control groups. The course of intragastric administration (21 days) of the drug to mice had no effect on the hemagglutinin and hemolysin level in mice blood, had no effect on the delayed-type hypersensitivity test, no effect on the rosette test reaction production and did not change phagocytes activity of neutrophils separated from blood. Therefore, the studies conducted showed that methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide had no immune toxic effect.

The results of gene toxicity using the Ames test, test of somatic mosaicism in the Drosophila melanogaster and metaphase chromosome aberration assay with murine marrow cells showed that methanesulfonic acid salt and 3-(1,2,4-triazole[4,3-a]pyridine-3-ylethinyl)-4-methyl-N-(4-((4-methylpiperazine-1-yl)methyl)-3-trifluoromethylphenyl)benzamide had no gene toxic effect with respect to all models and doses (concentrations) used.

While the invention has been described with reference to the disclosed embodiments, it should be apparent to those skilled in the art that the specific, detailed experiments described are for the purpose of illustrating the present invention only, and should not be construed as in any way limiting the scope of the invention as defined in the claims.

## Claims

1. 3-(1,2,4-triazolo[4,3-a]pyridine-3-ylethynyl)-4-methyl-N-(4-((4-methylpiperazin-1-yl)methyl)-3-trifluoromethylphenyl)benzamide monomesylate salt.

2. The salt of claim 1, wherein the salt is crystalline.

3. The salt according to claim 2, wherein the salt is **characterized by** a DSC curve having an endothermic transition at 220 °C.

4. The salt according to claim 2, wherein the salt is **characterized by** a monoclinic crystal lattice with space group P2₁/n.

5. The salt according to claim 4, wherein the salt is **characterized by** a monoclinic crystal lattice with unit cell parameters a = 51.46±0.05 Å; b = 7.81±0.05 Å; c = 7.63±0.05 Å; and β = 108.9±0.1°, determined by X-ray powder diffraction at 25±5 °C using CuKα1 radiation.

6. The salt according to claim 2, wherein the salt is **characterized by** an X-ray powder diffraction pattern obtained at 25±5 °C using CuKα1 radiation and comprising at least one, two, three or four peaks with a relative intensity of about 20 % or higher at diffraction angles (2θ) selected from 16.9; 17.2; 18.7 and 20.8.

7. The salt according to claim 6, wherein the salt is **characterized by** an X-ray powder diffraction pattern obtained at 25±5 °C using CuKα1 radiation and having peaks at diffraction angles (2θ) 16.9; 17.2; 18.7 and 20.8, each with a relative intensity of about 20 % or greater.

8. The salt according to claim 2, wherein the salt is **characterized by** a monoclinic crystal lattice with space group P2₁/c and with unit cell parameters a = 13.77±0.05 Å; b = 8.09±0.05 Å and c = 30.83±0.05 Å, and β = 117.8±0.1°, determined by X-ray powder diffraction at 25±5 °C using CuKα1 radiation.

9. The salt according to claim 2 or claim 8, wherein the salt is **characterized by** an X-ray powder diffraction pattern obtained at 25±5 °C using CuKα1 radiation and having at least one, two, three, four, five, six or seven peaks with a relative intensity of about 20 % or higher at diffraction angles (2θ) selected from: 17.4; 17.6; 19.4; 19.7; 21.2; 22.0 and 22.6.

10. A method of preparing crystalline 3-(1,2,4-triazolo[4,3-a]pyridine-3-ylethynyl)-4-methyl-N-(4-((4-methylpiperazin-1-yl)methyl)-3-trifluoromethylphenyl)benzamide monomesylate salt which includes:
(1) carrying out a reaction between 3-(1,2,4-triazolo[4,3-a]pyridine-3-ylethynyl)-4-methyl-N-(4-((4-methylpiperazin-1-yl)methyl)-3-trifluoromethylphenyl)benzamide and methanesulfonic acid to form 3-(1,2,4-triazolo[4,3-a]pyridine-3-ylethynyl)-4-methyl-N-(4-((4-methylpiperazin-1-yl)methyl)-3-trifluoromethylphenyl)benzamide monomesylate salt; and step of:
(2a) cooling a solution of 3-(1,2,4-triazolo[4,3-a]pyridine-3-ylethynyl)-4-methyl-N-(4-((4-methylpiperazin-1-yl)methyl)-3-trifluoromethylphenyl)benzamide monomesylate salt dissolved in a mixture of acetone : ethanol in an approximate ratio of 5:1 v/v to a temperature of about 10 °C to obtain the crystalline salt; or
(2b) concentrating a solution of 3-(1,2,4-triazolo[4,3-a]pyridine-3-ylethynyl)-4-methyl-N-(4-((4-methylpiperazin-1-yl)methyl)-3-trifluoromethylphenyl)benzamide monomesylate salt dissolved in a mixture of acetone : ethanol in an approximate ratio of 5:1 v/v and subsequent cooling of the solution to a temperature of about 20-25 °C to form the crystalline salt.

11. Crystalline 3-(1,2,4-triazolo[4,3-a]pyridine-3-ylethynyl)-4-methyl-N-(4-((4-methylpiperazin-1-yl)methyl)-3-trifluoromethylphenyl)benzamide monomesylate salt obtainable by the method according to claim 10.

12. A composition containing a salt according to any one of claims 1-9 or 11 and at least one pharmaceutically acceptable carrier and/or excipient.

13. The salt according to any one of claims 1-9 or 11 for use in medicine.

14. The salt according to any one of claims 1-9 or 11 for use in treating an oncological disease, in particular for treating acute lymphoblastic leukemia, chronic myeloid leukemia, hepatocellular carcinoma, non-small cell lung cancer or gastrointestinal stromal tumor.

## Patentansprüche

1. 3-(1,2,4-Triazolo[4,3-a]pyridin-3-ylethinyl)-4-methyl-N-(4-((4-methylpiperazin-l-yl)methyl)-3-trifluormethylphenyl)benzamid-Monomesylatsalz.

2. Das Salz nach Anspruch 1, wobei das Salz kristallin ist.

3. Das Salz nach Anspruch 2, wobei das Salz durch eine DSC-Kurve mit einem endothermen Übergang bei 220°C gekennzeichnet ist.

4. Das Salz nach Anspruch 2, wobei das Salz durch ein monoklines Kristallgitter mit Raumgruppe *P*2₁/*n* gekennzeichnet ist.

5. Das Salz nach Anspruch 4, wobei das Salz durch ein monoklines Kristallgitter mit Elementarzellenparametern a = 51,46±0,05 Å, b = 7,81±0,05 Å, c = 7,63±0,05 Å und β = 108,9±0,1°, bestimmt durch Röntgenpulverbeugung bei 25±5°C unter Verwendung von CuKα1-Strahlung, gekennzeichnet ist.

6. Das Salz nach Anspruch 2, wobei das Salz durch ein Röntgenpulverbeugungsmuster, erhalten bei 25±5°C unter Verwendung von CuKα1-Strahlung, mit mindestens einem, zwei, drei oder vier Peaks mit einer relativen Intensität von etwa 20% oder mehr bei Beugungswinkeln (2θ) ausgewählt aus 16,9, 17,2, 18,7 und 20,8 gekennzeichnet ist.

7. Das Salz nach Anspruch 6, wobei das Salz durch ein Röntgenpulverbeugungsmuster, erhalten bei 25±5°C unter Verwendung von CuKα1-Strahlung, mit Peaks bei Beugungswinkeln (2θ) ausgewählt aus 16,9, 17,2, 18,7 und 20,8, jeweils mit einer relativen Intensität von 20% oder mehr, gekennzeichnet ist.

8. Das Salz nach Anspruch 2, wobei das Salz durch ein monoklines Kristallgitter mit Raumgruppe P2₁/c und mit Elementarzellenparametern a = 13,77±0,05 Å, b = 8,09±0,05 Å, c = 30,83±0,05 Å und β = 117,8±0,1°, bestimmt durch Röntgenpulverbeugung bei 25±5°C unter Verwendung von CuKα1-Strahlung, gekennzeichnet ist.

9. Das Salz nach Anspruch 2 oder Anspruch 8, wobei das Salz durch ein Röntgenpulverbeugungsmuster, erhalten bei 25±5°C unter Verwendung von CuKα1-Strahlung, mit mindestens einem, zwei, drei, vier, fünf, sechs oder sieben Peaks mit einer relativen Intensität von etwa 20% oder mehr bei Beugungswinkeln (2θ) ausgewählt aus: 17,4, 17,6, 19,4, 19,7, 21,2, 22,0 und 22,6 gekennzeichnet ist.

10. Ein Verfahren zur Herstellung von kristallinem 3-(1,2,4-Triazolo[4,3-a]pyridin-3-ylethinyl)-4-methyl-N-(4-((4-methylpiperazin-1-yl)methyl)-3-trifluormethylphenyl)benzamid-Monomesylatsalz, welches Folgendes umfasst:
(1)das Durchführen einer Umsetzung von 3-(1,2,4-Triazolo[4,3-a]pyridin-3-ylethinyl)-4-methyl-N-(4-((4-methylpiperazin-l-yl)methyl)-3-trifluormethylphenyl)benzamid mit Methansulfonsäure unter Bildung von 3-(1,2,4-Triazolo[4,3-a]pyridin-3-ylethinyl)-4-methyl-N-(4-((4-methylpiperazin-1-yl)methyl)-3-trifluormethylphenyl)benzamid-Monomesylatsalz, und einen der folgenden Schritte:
(2a) das Abkühlen einer Lösung von 3-(1,2,4-Triazolo[4,3-a]pyridin-3-ylethinyl)-4-methyl-N-(4-((4-methylpiperazin-1-yl)methyl)-3-trifluormethylphenyl)benzamid-Monomesylatsalz, gelöst in einer Mischung von Aceton:Ethanol in einem ungefähren Verhältnis von 5:1 v/v, auf eine Temperatur von etwa 10°C unter Erhalt des kristallinen Salzes oder
(2b) das Einengen einer Lösung von 3-(1,2,4-Triazolo[4,3-a]pyridin-3-ylethinyl)-4-methyl-N-(4-((4-methylpiperazin-1-yl)methyl)-3-trifluormethylphenyl)benzamid-Monomesylatsalz, gelöst in einer Mischung von Aceton:Ethanol in einem ungefähren Verhältnis von 5:1 v/v und das anschließende Abkühlen der Lösung auf eine Temperatur von etwa 20-25°C unter Bildung des kristallinen Salzes.

11. Kristallines 3-(1,2,4-Triazolo[4,3-a]pyridin-3-ylethinyl)-4-methyl-N-(4-((4-methylpiperazin-1-yl)methyl)-3-trifluormethylphenyl)benzamid-Monomesylatsalz, erhältlich nach dem Verfahren nach Anspruch 10.

12. Eine Zusammensetzung, enthaltend ein Salz nach einem der Ansprüche 1-9 oder 11 und mindestens einen pharmazeutisch unbedenklichen Träger und/oder Hilfsstoff.

13. Das Salz nach einem der Ansprüche 1-9 oder 11 zur Verwendung in der Medizin.

14. Das Salz nach einem der Ansprüche 1-9 oder 11 zur Verwendung bei der Behandlung einer onkologischen Krankheit, insbesondere zur Behandlung von akuter lymphatischer Leukämie, chronischer myeloischer Leukämie, hepatozellulärem Karzinom, nichtkleinzelligem Lungenkrebs oder gastrointestinalem Stromatumor.

## Revendications

1. Sel monomésylate de 3-(1,2,4-triazolo[4,3-a]pyridin-3-yléthynyl)-4-méthyl-*N*-(4-((4-méthylpipérazin-1-yl)méthyl)-3-trifluorométhylphényl)benzamide.

2. Le sel selon la revendication 1, le sel étant cristallin.

3. Le sel selon la revendication 2, le sel étant **caractérisé par** une courbe de DSC ayant une transition endothermique à 220 °C.

4. Le sel selon la revendication 2, le sel étant **caractérisé par** un réseau cristallin monoclinique assorti du groupe d'espace P2₁/n.

5. Le sel selon la revendication 4, le sel étant **caractérisé par** un réseau cristallin monoclinique assorti des paramètres de maille a= 51,46 ± 0,05 Å ; b= 7,81 ± 0,05 Å ; c = 7,63 ± 0,05 Å ; et β = 108,9 ± 0,1°, déterminés par diffraction des rayons X sur poudre à 25 ± 5 °C à l'aide du rayonnement CuKα1.

6. Le sel selon la revendication 2, le sel étant **caractérisé par** un diagramme de diffraction des rayons X sur poudre obtenu à 25 ± 5 °C à l'aide du rayonnement CuKα1 et comprenant au moins un, deux, trois ou quatre pics assortis d'une intensité relative supérieure ou égale à environ 20 % à des angles de diffraction (2θ) choisis parmi 16,9 ; 17,2 ; 18,7 et 20,8.

7. Le sel selon la revendication 6, le sel étant **caractérisé par** un diagramme de diffraction des rayons X sur poudre obtenu à 25 ± 5 °C à l'aide du rayonnement CuKα1 et ayant des pics aux angles de diffraction (2θ) de 16,9 ; 17,2 ; 18,7 et 20,8, chacun assorti d'une intensité relative supérieure ou égale à environ 20 %.

8. Le sel selon la revendication 2, le sel étant **caractérisé par** un réseau cristallin monoclinique assorti du groupe d'espace P2₁/c et assorti des paramètres de maille a= 13,77 ± 0,05 Å ; b= 8,09 ± 0,05 Å et c = 30,83 ± 0,05 Å et β = 117,8 ± 0,1°, déterminés par diffraction des rayons X sur poudre à 25 ± 5 °C à l'aide du rayonnement CuKα1.

9. Le sel selon la revendication 2 ou la revendication 8, le sel étant **caractérisé par** un diagramme de diffraction des rayons X sur poudre obtenu à 25 ± 5 °C à l'aide du rayonnement CuKal et ayant au moins un, deux, trois, quatre, cinq, six ou sept pics assortis d'une intensité relative supérieure ou égale à environ 20 % à des angles de diffraction (2θ) choisis parmi : 17,4 ; 17,6 ; 19,4 ; 19,7 ; 21,2 ; 22,0 et 22,6.

10. Un procédé de préparation de sel monomésylate de 3-(1,2,4-triazolo[4,3-*a*]pyridin-3-yléthynyl)-4-méthyl-*N*-(4-((4-méthylpipérazin-1-yl)méthyl)-3-trifluorométhylphényl)benzamide cristallin qui comporte :
(1) la mise en oeuvre d'une réaction entre du 3-(1,2,4-triazolo[4,3-*a*]pyridin-3-yléthynyl)-4-méthyl-*N*-(4-((4-méthylpipérazin-1-yl)méthyl)-3-trifluorométhylphényl)benzamide et de l'acide méthanesulfonique pour former du sel monomésylate de 3-(1,2,4-triazolo[4,3-*a*]pyridin-3-yléthynyl)-4-méthyl-*N*-(4-((4-méthylpipérazin-1-yl)méthyl)-3-trifluorométhylphényl)benzamide ; et l'étape de :
(2a) refroidissement d'une solution de sel monomésylate de 3-(1,2,4-triazolo[4,3-*a*]pyridin-3-yléthynyl)-4-méthyl-*N*-(4-((4-méthylpipérazin-1-yl)méthyl)-3-trifluorométhylphényl)benzamide dissous dans un mélange acétone:éthanol en un rapport approximatif de 5:1 v/v à une température d'environ 10 °C pour obtenir le sel cristallin ; ou
(2b) concentration d'une solution de sel monomésylate de 3-(1,2,4-triazolo[4,3-*a*]pyridin-3-yléthynyl)-4-méthyl-*N*-(4-((4-méthylpipérazin-1-yl)méthyl)-3-trifluorométhylphényl)benzamide dissous dans un mélange acétone:éthanol en un rapport approximatif de 5:1 v/v et refroidissement subséquent de la solution à une température d'environ 20-25 °C pour former le sel cristallin.

11. Sel monomésylate de 3-(1,2,4-triazolo[4,3-*a*]pyridin-3-yléthynyl)-4-méthyl-*N*-(4-((4-méthylpipérazin-1-yl)méthyl)-3-trifluorométhylphényl)benzamide cristallin pouvant être obtenu par le procédé selon la revendication 10.

12. Une composition contenant un sel selon l'une quelconque des revendications 1-9 ou 11 et au moins un véhicule et/ou excipient pharmaceutiquement acceptable.

13. Le sel selon l'une quelconque des revendications 1-9 ou 11 destiné à être utilisé en médecine.

14. Le sel selon l'une quelconque des revendications 1-9 ou 11 destiné à être utilisé en traitement d'une maladie oncologique, en particulier pour le traitement de leucémie lymphoblastique aiguë, de leucémie myéloïde chronique, de carcinome hépatocellulaire, de cancer du poumon non à petites cellules ou de tumeur stromale gastro-intestinale.
